(19) Europäisches Patentamt
European Patent Office
Office européen des brevets

(11) Veröffentlichungsnummer: **0 244 595**
**A2**

# (12) EUROPÄISCHE PATENTANMELDUNG

(21) Anmeldenummer: **87103339.5**

(22) Anmeldetag: **09.03.87**

(51) Int. Cl.$^4$: **C07D 211/90**, C07D 401/12, C07D 405/12, C07D 401/04, A61K 31/44

The title of the invention has been amended (Guidelines for Examination in the EPO, A-III, 7.3).

(30) Priorität: **12.03.86 CH 1005/86**

(43) Veröffentlichungstag der Anmeldung: **11.11.87 Patentblatt 87/46**

(84) Benannte Vertragsstaaten: **AT BE CH DE ES FR GB GR IT LI LU NL SE**

(71) Anmelder: **Byk Gulden Lomberg Chemische Fabrik GmbH**
**Byk-Gulden-Strasse 2**
**D-7750 Konstanz(DE)**

(72) Erfinder: **Amschler, Hermann**
**Hohenhewenstrasse 19**
**D-7760 Radolfzell(DE)**
Erfinder: **Eistetter, Klaus**
**Säntisblick 7**
**D-7750 Konstanz 19(DE)**
Erfinder: **Eltze, Manfrid**
**Schützenstrasse 20**
**D-7750 Konstanz(DE)**
Erfinder: **Flockerzi, Dieter**
**Ackerweg 26**
**D-7753 Allensbach(DE)**
Erfinder: **Klemm, Kurt**
**Im Weinberg 2**
**D-7753 Allensbach(DE)**
Erfinder: **Kolassa, Norbert**
**Lerchenweg 12**
**D-7750 Konstanz(DE)**
Erfinder: **Sanders, Karl**
**Felchengang 23**
**D-7750 Konstanz(DE)**
Erfinder: **Schudt, Christian**
**Hoheneggstrasse 102**
**D-7750 Konstanz(DE)**
Erfinder: **Ulrich, Wolf-Rüdiger**
**Hebelstrasse 3**
**D-7750 Konstanz(DE)**

(54) **Dihydropyridinderivate, Verfahren zu ihrer Herstellung und ihre Verwendung als Arzneimittel.**

(57) Monocyclische Ester der Formel I,

EP 0 244 595 A2

Xerox Copy Centre

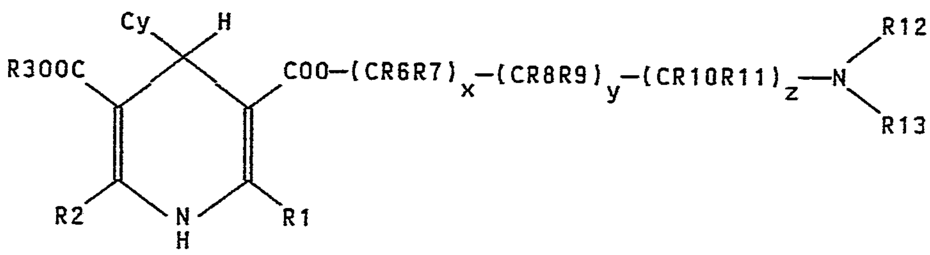

(I)

worin die Substituenten und Symbole die in der Beschreibung genannten Bedeutungen haben, sind neue Verbindungen mit überraschenden pharmakologischen Eigenschaften.

# Neue monocyclische Ester

## Anwendungsgebiet der Erfindung

Die Erfindung betrifft neue monocyclische Ester, Verfahren zu ihrer Herstellung, ihre Verwendung und sie enthaltende Arzneimittel. Die erfindungsgemäßen Verbindungen werden in der pharmazeutischen Industrie zur Herstellung von Arzneimitteln eingesetzt.

## Bekannter technischer Hintergrund

Es ist bekannt, daß bestimmte, auf verschiedene Weise substituierte 1,4-Dihydropyridinderivate pharmakologisch nützliche Eigenschaften aufweisen. Überraschenderweise wurde nun gefunden, daß die unten näher beschriebenen neuen Verbindungen besonders interessante pharmakologische Eigenschaften aufweisen, durch die sie sich von den Verbindungen des Standes der Technik in vorteilhafter Weise unterscheiden.

## Beschreibung der Erfindung

Gegenstand der Erfindung sind neue monocyclische Ester der Formel I

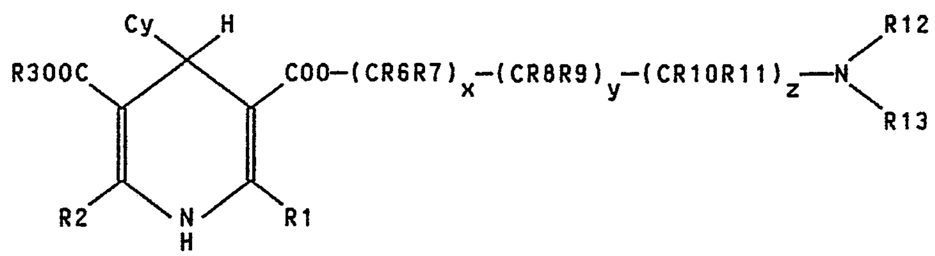

(I)

worin Cy einen Cyclus der Formel

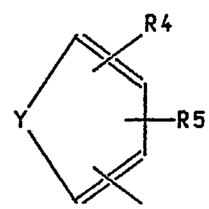

darstellt, in dem Y Sauerstoff (O), Schwefel (S), Vinylen (-CH=CH-)' Azomethin (-CH=N-) oder eine Gruppe der Formel

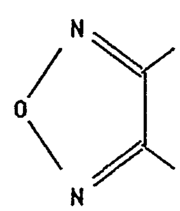

oder

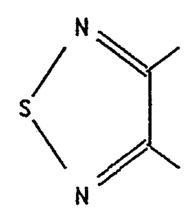

bedeutet,

R1 und R2 gleich oder verschieden sind und Wasserstoff, 1-6C-Alkyl oder 3-7C-Alkoxyalkyl bedeuten,

R3 einen (-)-Menthylrest darstellt,

R4 und R5 gleich oder verschieden sind und Wasserstoff, Hydroxy, Halogen, Nitro, Cyano, Trifluormethyl, 1-4C-Alkyl, 1-4C-Alkoxy, ganz oder teilweise durch Fluor substituiertes 1-4C-Alkoxy, 1-4C-Alkoxycarbonyl, 2-5C-Acyl, Amino oder Mono-oder Di-1-4C-alkylamino bedeuten,

R6 Wasserstoff (H) oder 1-4C-Alkyl bedeutet,

R7 Wasserstoff (H), 1-4C-Alkyl oder gemeinsam mit R12 1-4C-Alkylen bedeutet, das gewünschtenfalls durch eine oder mehrere 1-4C-Alkylgruppen substituiert ist,

R8 Wasserstoff (H) oder 1-4C-Alkyl bedeutet,

R9 Wasserstoff (H), 1-4C-Alkyl, 1-4C-Alkoxy, Aryl, Heteroaryl oder gemeinsam mit R12 1-4C-Alkylen bedeutet, das gewünschtenfalls durch eine oder mehrere 1-4C-Alkylgruppen substituiert ist,

R10 Wasserstoff (H) oder 1-4C-Alkyl bedeutet,

R11 Wasserstoff (H) oder 1-4C-Alkyl bedeutet,

R12 Wasserstoff (H), 1-4C-Alkyl oder gemeinsam mit R7 oder R9 1-4C-Alkylen bedeutet, das gewünschtenfalls durch eine oder mehrere 1-4C-Alkylgruppen substituiert ist, und

R13 1-4C-Alkyl, Aryl, Aryl-1-4C-alkyl, Aryl-2-4C-alkenyl, Aryl-2-4C-alkinyl, 2-5C-Acyl, Arylcarbonyl oder Heteroarylcarbonyl bedeutet, oder

R12 und R13 gemeinsam und unter Einschluß des Stickstoffatoms, an das beide gebunden sind, einen Rest der Formel

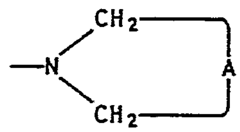

darstellen, worin

A $-CH_2-(CH_2)_m-$,

$-CH_2-C(R14)R15-CH_2-$,

$-CH=CR15-CH_2-$,

$-CH_2-O-CH_2-$,

$-CH_2-S-CH_2-$,

$-CH_2-NR16-CH_2-$oder

$-(CH_2)_2-NR16-CH_2-$bedeutet,

R14 Wasserstoff (H) oder Aryl und

R15 Wasserstoff (H), 1-4C-Alkyl, 1-4C-Alkoxy, Hydroxy, Aryl, Arylcarbonyl oder Heteroaryl bedeutet, oder

R14 und R15 gemeinsam Diarylmethylen bedeuten,

R16 1-4C-Alkyl, Aryl, Aryl-1-4C-alkyl, Aryl-2-4C-alkenyl, Aryl-2-4C-alkinyl, Diaryl-1-4C-alkyl, Heteroaryl, Heteroaryl-1-4C-alkyl, Heteroaryl-aryl-1-4C-alkyl, Di-heteroaryl-1-4C-alkyl, Arylcarbonyl, Heteroarylcarbonyl, Arylsulfonyl, Aryl-1-4C-alkylcarbonyl oder Aryl-2-4C-alkenylcarbonyl bedeutet,

x, y und z gleich oder verschieden sind und die Zahlen 0, 1, 2, 3 oder 4 bedeuten, wobei die Summe von x, y und z eine Zahl von 1 bis 5 ist, und

m die Zahl 1, 2 oder 3 bedeutet, wobei

Aryl für einen Ring der Formel

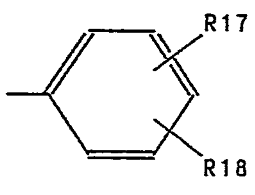

steht, worin R17 und R18 gleich oder verschieden sind und die Bedeutung Wasserstoff (H), 1-4C-Alkyl, 1-4C-Alkoxy, Halogen, Hydroxy, Trifluormethyl oder gemeinsam Methylendioxy haben, und Heteroaryl für

einen 5-oder 6-gliedrigen Heterocyclus mit einem oder zwei gleichen oder verschiedenen Heteroatomen aus der Gruppe Sauerstoff (O), Schwefel (S) oder Stickstoff (N) steht, der ungesättigt oder teilweise oder ganz gesättigt ist und der einen oder zwei Substituenten aus der Gruppe 1-4C-Alkyl, 1-4C-Alkoxy, Halogen, Trifluormethyl oder Cyan tragen kann,
und die Salze dieser Verbindungen.

1-6C-Alkyl ist geradkettig oder verzweigt und bedeutet beispielsweise einen Hexyl-, Neopentyl-, Isopentyl-, Butyl-, i-Butyl-, sec.-Butyl-, t-Butyl-, Propyl-, Isopropyl-oder insbesondere Ethyl-oder Methylrest.

3-7C-Alkoxyalkyl steht beispielsweise für einen Ethoxyethyl-, Propoxyethyl-, Isopropoxyethyl-, Butoxyethyl-, Methoxypropyl-, 2-Methoxy-1-methylethyl-, 2-Ethoxy-1-methylethyl-oder insbesondere Methoxyethylrest.

Der (-)-Menthylrest ist der Rest, der sich vom (1R:3R:4S)-p-Menthanol-(3) herleitet.

Halogen im Sinne der Erfindung bedeutet Brom, Fluor und insbesondere Chlor.

1-4C-Alkyl ist geradkettig oder verzweigt und bedeutet beispielsweise einen Butyl-, i-Butyl-, sec.-Butyl-, t-Butyl-, Propyl-, Isopropyl-, Ethyl-oder insbesondere Methylrest.

1-4C-Alkoxy enthält neben dem Sauerstoffatom einen der vorstehend genannten 1-4C-Alkylreste. Bevorzugt sind der Methoxy-und der Ethoxyrest.

Ganz oder teilweise durch Fluor substituiertes 1-4C-Alkoxy ist beispielsweise 1,1,2,2-Tetrafluorethoxy, Trifluormethoxy, 2,2,2-Trifluorethoxy oder insbesondere Difluormethoxy.

1-4C-Alkoxycarbonyl enthält neben der Carbonylgruppe einen der vorstehend genannten 1-4C-Alkoxyreste. Bevorzugt sind der Methoxycarbonyl-und der Ethoxycarbonylrest.

2-5C-Acyl enthält neben der Carbonylgruppe einen der vorstehend genannten 1-4C-Alkylreste. Bevorzugt ist der Acetylrest.

Mono-oder Di-1-4C-alkylamino enthält neben dem Stickstoffatom einen oder zwei der vorstehend genannten 1-4C-Alkylreste. Bevorzugt ist Di-1-4C-alkylamino, und hier insbesondere Dimethyl-, Diethyl-oder Diisopropylamino.

1-4C-Alkylen, das gemeinsam von R7 oder R9 und R12 gebildet wird, und das gewünschtenfalls durch eine oder mehrere 1-4C-Alkylgruppen substituiert ist, ist beispielsweise Methylen, Tetramethylen, 1-Methylethylen, 2,2-Dimethylethylen und insbesondere Ethylen oder Propylen.

Aryl steht für durch R17 und R18 substituiertes Phenyl. Als beispielhafte bevorzugte Arylreste seien genannt die Reste: Phenyl, 4-Methoxyphenyl, 4-Chlorphenyl, 4-Methylphenyl, 4-Fluorphenyl, 3-Fluorphenyl, 3-Chlorphenyl, 2-Chlorphenyl, 3-Methoxyphenyl, 2-Methoxyphenyl, 2-Ethoxyphenyl, 2-Methylphenyl, 3-Chlor-4-methylphenyl, 3,4-Dichlorphenyl, 3,6-Dichlorphenyl, 2,4-Dimethylphenyl, 2,6-Dimethylphenyl, 3,4-Dimethylphenyl, 3,4-Methylendioxyphenyl, 2-Trifluormethylphenyl und 3-Trifluormethylphenyl.

Aryl-1-4C-alkyl steht für 1-4C-Alkyl, das durch Aryl substituiert ist. Als beispielhafte bevorzugte Aryl-1-4C-alkylreste seien genannt die Reste: 4-Methylbenzyl, 4-Methoxybenzyl, 4-Chlorbenzyl, 1-Phenethyl, 2-Phenylethyl, 3-Chlorbenzyl, 2,5-Dimethylbenzyl, 4-Fluorbenzyl, 3-Methylbenzyl und insbesondere Benzyl.

Aryl-2-4C-alkenyl und Aryl-2-4C-alkinyl steht für Alkenyl-und Alkinylreste mit 2 bis 4 Kohlenstoffatomen, die durch Aryl substituiert sind. Beispielsweise seien der 3-Phenyl-2-propenylrest und der 3-Phenyl-2-propinylrest genannt.

Arylcarbonyl steht für eine Carbonylgruppe, die durch Aryl substituiert ist. Als bevorzugte Arylcarbonylgruppen seien beispielsweise die 4-Chlorphenylcarbonyl, 2-Hydroxyphenylcarbonyl-(Salicyloyl-), die 4-Hydroxyphenylcarbonyl-und insbesondere die 4-Fluorphenylcarbonyl-und die Benzoylgruppe genannt.

Als beispielhafte bevorzugte (substituierte) Heteroarylreste seien genannt die Reste: Furyl, insbesondere 2-Furyl, Thienyl, insbesondere 2-Thienyl, Pyrimidyl, insbesondere 2-Pyrimidyl, 3-Cyan-2-pyridyl, Thiazolyl, insbesondere 2-Thiazolyl sowie Pyridyl, insbesondere 3-Pyridyl und vorzugsweise 2-Pyridyl.

Heteroarylcarbonyl steht für eine Carbonylgruppe, die durch Heteroaryl substituiert ist. Als beispielhafte bevorzugte Heteroarylcarbonylrest seien der Nicotinoyl-und der 2-Furoylrest genannt.

Heteroaryl-1-4C-alkyl ist 1-4C-Alkyl, das durch Heteroaryl substituiert ist. Heteroaryl-1-4C-alkyl ist beispielsweise 2-(2-Pyridyl)-ethyl.

Diaryl-1-4C-alkyl ist 1-4C-Alkyl, das durch zwei Arylreste substituiert ist. Diaryl-1-4C-alkyl ist insbesondere Diphenylmethyl (Benzhydryl), oder substituiertes Benzhydryl, wie z.B. 4,4' -Difluorbenzhydryl, 4,4' -Dimethylbenzhydryl, 4,4' -Dimethoxybenzhydryl oder 4,4' Dichlorbenzhydryl.

Heteroaryl-aryl-1-4C-alkyl ist 1-4C-Alkyl, das durch Heteroaryl und Aryl substituiert ist. Heteroaryl-aryl-1-4C-alkyl ist beispielsweise 2-Pyridylphenylmethyl.

Di-heteroaryl-1-4C-alkyl ist 1-4C-Alkyl, das durch zwei Heteroarylreste substituiert ist. Di-heteroaryl-1-4C-alkyl ist beispielsweise Di-pyrid-2-yl-methyl.

Arylsulfonyl steht für eine Sulfonylgruppe, die durch Aryl substituiert ist. Als beispielhafte bevorzugte Arylsulfonylreste seien der Phenylsulfonyl-, der 4-Chlorphenylsulfonyl-und der 4-Methoxyphenylsulfonylrest genannt.

Als Aryl-1-4C-alkylcarbonylrest sei beispielsweise der 2-Phenylpropionylrest genannt. Als Aryl-2-4C-alkenylcarbonylrest sei beispielsweise der Cinnamoylrest genannt.

Als Salze kommen alle Salze mit Säuren in Betracht. Besonders erwähnt seien die pharmakologisch verträglichen Salze der in der pharmazeutischen Industrie üblicherweise verwendeten anorganischen und organischen Säuren. Pharmakologisch unverträgliche Salze, die beispielsweise bei der Herstellung der erfindungsgemäßen Verbindungen im industriellen Maßstab als Verfahrensprodukte zunächst anfallen können, werden durch dem Fachmann bekannte Verfahren in pharmakologisch verträgliche Salze übergeführt. Als solche eignen sich beispielsweise wasserlösliche und wasserunlösliche Säureadditionssalze, wie das Hydrochlorid, Hydrobromid, Hydroiodid, Phosphat, Nitrat, Sulfat, Acetat, Citrat, Gluconat, Benzoat, Hibenzat, Fendizoat, Butyrat, Sulfosalicylat, Maleat, Laurat, Malat, Fumarat, Succinat, Oxalat, Tartrat, Amsonat, Metembonat, Stearat, Tosilat, 2-Hydroxy-3-naphthoat, 3-Hydroxy-2-naphthoat oder Mesilat, aber auch Salze mit Bumetanid, Furosemid, Azosemid, Galosemid, Besunid, Piretanid, Etacrynsäure, Tienilinsäure oder 4-Chlor-sulfamoyl-benzoesäure.

Bevorzugter Gegenstand der Erfindung sind Verbindungen der Formel I, worin
Cy 3-Nitrophenyl, 2, 3-Dichlorphenyl oder 3-Pyridyl bedeutet,
R1 und R2 gleich oder verschieden sind und 1-4C-Alkyl bedeuten,
R3 einen (-)-Menthylrest darstellt,
die Gruppierung $(CR6R7)_x$-$(CR8R9)_y$-$(CR10R11)_z$ Ethylen oder Propylen bedeutet,
R12 1-4C-Alkyl bedeutet und
R13 Benzyl bedeutet, oder
R12 und R13 gemeinsam und unter Einschluß des Stickstoffatoms, an das beide gebunden sind, einen Rest der Formel

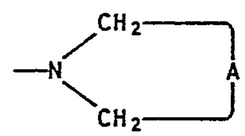

darstellen, worin
A $-CH_2-C(R14)R15-CH_2-$,
$-CH_2-O-CH_2-$oder
$-CH_2-NR16-CH_2-$bedeutet,
R14 Wasserstoff (H) oder Phenyl bedeutet,
R15 Wasserstoff (H), Phenyl oder 4-Fluorbenzoyl bedeutet,
R16 Aryl, 2-Pyridyl oder Benzhydryl bedeutet, wobei
Aryl für einen Ring der Formel

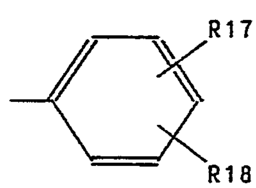

steht, worin R17 und R18 gleich oder verschieden sind und die Bedeutung Wasserstoff (H), 1-4C-Alkyl, 1-4C-Alkoxy, Trifluormethyl oder gemeinsam Methylendioxy haben,
und die Salze dieser Verbindungen.

Eine Ausgestaltung (Ausgestaltung a) der Erfindung sind Verbindungen der Formel I, worin
Cy, R1, R2, R3, R4 und R5 die obengenannten Bedeutungen haben. R6 Wasserstoff (H) bedeutet,
R7 Wasserstoff (H) bedeutet,
R8 Wasserstoff (H) bedeutet,
R9 Wasserstoff (H), 1-4C-Alkyl, 1-4C-Alkoxy, Aryl oder Heteroaryl bedeutet,

R10 Wasserstoff (H) bedeutet,
R11 Wasserstoff (H) bedeutet,
R12 Wasserstoff (H) oder 1-4C-Alkyl bedeutet,
R13 1-4C-Alkyl, Aryl, Aryl-1-4C-alkyl, Aryl-2-4C-alkenyl, Aryl-2-4C-alkinyl, 2-5C-Acyl, Arylcarbonyl oder Heteroarylcarbonyl bedeutet, und
x, y und z gleich oder verschieden sind und die Zahlen 0, 1, 2, 3 oder 4 bedeuten, wobei die Summe von x, y und z eine Zahl von 1 bis 5 ist, wobei
Aryl für einen Ring der Formel

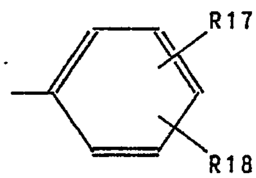

steht, worin R17 und R18 gleich oder verschieden sind und die Bedeutung Wasserstoff (H), 1-4C-Alkyl, 1-4C-Alkoxy, Halogen, Hydroxy oder Trifluormethyl haben, und
Heteroaryl für einen 5-oder 6-gliedrigen Heterocyclus mit einem oder zwei gleichen oder verschiedenen Heteroatomen aus der Gruppe Sauerstoff (O), Schwefel (S) oder Stickstoff (N) steht, der ungesättigt oder teilweise oder ganz gesättigt ist und der einen oder zwei Substituenten aus der Gruppe 1-4C-Alkyl, 1-4C-Alkoxy, Halogen, Trifluormethyl oder Cyan tragen kann,
und die Salze dieser Verbindungen.

Hervorzuhebende Verbindungen der Ausgestaltung a sind solche der Formel I, worin
Cy Phenyl, 2-Nitrophenyl, 3-Nitrophenyl, 2-Cyanophenyl, 3-Cyanophenyl, 2-(1,1,2,2-Tetrafluorethoxy)-phenyl, 3-(1,1,2,2-Tetrafluorethoxy)-phenyl, 2-Difluormethoxyphenyl, 3-Difluormethoxyphenyl, 2-Chlorphenyl, 3-Chlorphenyl, 2,3-Dichlorphenyl, 2-Fluorphenyl, 3-Fluorphenyl, 2-Trifluormethylphenyl, 3-Trifluormethylphenyl, 3-Pyridyl oder Benzoxdiazolyl bedeutet,
R1 Methyl bedeutet,
R2 Methyl bedeutet,
R3 einen (-)-Menthylrest darstellt,
die Gruppierung $(CR6R7)_x$-$(CR8R9)_y$-$(CR10R11)_z$ Ethylen oder Propylen bedeutet,
R12 Wasserstoff (H), Methyl oder Ethyl bedeutet und
R13 Phenyl, 4-Methoxyphenyl, Benzyl, 4-Methylbenzyl, 4-Methoxybenzyl, 4-Chlorbenzyl, 2-(3-Methoxyphenyl)-ethyl, 2-(3,4-Dimethoxyphenyl)-ethyl, 3-Phenyl-2-propenyl, 3-Phenyl-2-propinyl, Benzoyl, Nicotinoyl, Salicyloyl, 4-Fluorbenzoyl oder 4-Hydroxybenzoyl bedeutet,
und ihre Salze.

Bevorzugte Verbindungen der Ausgestaltung a sind solche der Formel I, worin
Cy 3-Nitrophenyl, 2-Chlorphenyl, 2,3-Dichlorphenyl, 2-Trifluormethylphenyl, 2-Difluormethoxyphenyl oder Benzoxdiazolyl bedeutet,
R1 Methyl bedeutet,
R2 Methyl bedeutet,
R3 einen (-)-Menthylrest darstellt, die Gruppierung $(CR6R7)_x$-$(CR8R9)_y$-$(CR10R11)_z$ Ethylen oder Propylen bedeutet,
R12 Methyl bedeutet und
R13 Benzyl, Phenethyl, 2-(3-Methoxyphenyl)-ethyl oder 2-(3,4-Dimethoxyphenyl)-ethyl bedeutet,
und ihre Salze.

Besonders bevorzugte Verbindungen der Ausgestaltung a sind solche der Formel I, worin
Cy 3-Nitrophenyl bedeutet,
R1 Methyl bedeutet,
R2 Methyl bedeutet,
R3 einen (-)-Menthylrest darstellt,
die Gruppierung $(CR6R7)_x$-$(CR8R9)_y$-$(CR10R11)_z$ Ethylen oder Propylen bedeutet,
R12 Methyl bedeutet und
R13 Benzyl bedeutet,
und ihre Salze.

Als hervorzuhebende Verbindungen der Ausgestaltung a seien genannt:
1,4-Dihydro-2,6-dimethyl-4-(3-nitrophenyl)-pyridin-3,5-dicarbonsäure-3-(-)-menthyl-5-[2-(2-phenylethyl-methylamino)-ethyl]-ester,
1,4-Dihydro-2,6-dimethyl-4-(3-nitrophenyl)-pyridin-3,5-dicarbonsäure-3-(-)-menthyl-5-[3-(2-phenylethyl-methylamino)-propyl]-ester,
1,4-Dihydro-2,6-dimethyl-4-(3-nitrophenyl)-pyridin-3,5-dicarbonsäure-3-(-)-menthyl-5-{2-[2-(3-methoxyphenyl)-ethyl-methylamino]-ethyl}-ester,
1,4-Dihydro-2,6-dimethyl-4-(3-nitrophenyl)-pyridin-3,5-dicarbonsäure-3-(-)-menthyl-5-{3-[2-(3-methoxyphenyl)-ethyl-methylamino]-propyl}-ester,
1,4-Dihydro-2,6-dimethyl-4-(3-nitrophenyl)-pyridin-3,5-dicarbonsäure-3-(-)-menthyl-5-{2-[2-(3,4-dimethoxyphenyl)-ethyl-methylamino]-ethyl}-ester,
1,4-Dihydro-2,6-dimethyl-4-(3-nitrophenyl)-pyridin-3,5-dicarbonsäure-3-(-)-menthyl-5-{3-[2-(3,4-dimethoxyphenyl)-ethyl-methylamino]-propyl}-ester
und ihre Salze.

Als bevorzugte Verbindungen der Ausgestaltung a seien genannt:
1,4-Dihydro-2,6-dimethyl-4-(2-chlorphenyl)-pyridin-3,5-dicarbonsäure-3-(-)-menthyl-5-[2-(benzylmethylamino)-ethyl]-ester,
1,4-Dihydro-2,6-dimethyl-4-(2,3-dichlorphenyl)-pyridin-3,5-dicarbonsäure-3-(-)-menthyl-5-[2-(benzylmethylamino)-ethyl]-ester,
1,4-Dihydro-2,6-dimethyl-4-(2-trifluormethylphenyl)-pyridin-3,5-dicarbonsäure-3-(-)-menthyl-5-[2-(benzylmethylamino)-ethyl]-ester,
1,4-Dihydro-2,6-dimethyl-4-(2-difluormethoxyphenyl)-pyridin-3,5-dicarbonsäure-3-(-)-menthyl-5-[2-(benzylmethylamino)-ethyl]-ester,
1,4-Dihydro-2,6-dimethyl-4-(2,1,3-benzoxdiazol-4-yl)-pyridin-3,5-dicarbonsäure-3-(-)-menthyl-5-[2-(benzylmethylamino)-ethyl]-ester,
1,4-Dihydro-2,6-dimethyl-4-(2-chlorphenyl)-pyridin-3,5-dicarbonsäure-3-(-)-menthyl-5-[3-(benzylmethylamino)-propyl]-ester,
1,4-Dihydro-2,6-dimethyl-4-(2,3-dichlorphenyl)-pyridin-3,5-dicarbonsäure-3-(-)-menthyl-5-[3-(benzylmethylamino)-propyl]-ester,
1,4-Dihydro-2,6-dimethyl-4-(2-trifluormethylphenyl)-pyridin-3,5-dicarbonsäure-3-(-)-menthyl-5-[3-(benzylmethylamino)-propyl]-ester,
1,4-Dihydro-2,6-dimethyl-4-(2-difluormethoxyphenyl)-pyridin-3,5-dicarbonsäure-3-(-)-menthyl-5-[3-(benzylmethylamino)-propyl]-ester,
1,4-Dihydro-2,6-dimethyl-4-(2,1,3-benzoxdiazol-4-yl)-pyridin-3,5-dicarbonsäure-3-(-)-menthyl-5-[3-(benzylmethylamino)-propyl]-ester
und ihre Salze.

Als besonders bevorzugte Verbindungen der Ausgestaltung a seien genannt:
1,4-Dihydro-2,6-dimethyl-4-(3-nitrophenyl)-pyridin-3,5-dicarbonsäure-3-(-)-menthyl-5-[2-(benzylmethylamino)-ethyl]-ester,
1,4-Dihydro-2,6-dimethyl-4-(3-nitrophenyl)-pyridin-3,5-dicarbonsäure-3-(-)-menthyl-5-[3-(benzylmethylamino)-propyl]-ester
und ihre Salze.

Eine weitere Ausgestaltung (Ausgestaltung b) der Erfindung sind Verbindungen der Formel I, worin Cy, R1, R2, R3, R4 und R5 die obengenannten Bedeutungen haben,
R6 Wasserstoff (H) oder 1-4C-Alkyl bedeutet,
R7 gemeinsam mit R12 1-4C-Alkylen bedeutet, das gewünschtenfalls durch eine oder mehrere 1-4C-Alkylgruppen substituiert ist,
R8 Wasserstoff (H) oder 1-4C-Alkyl bedeutet,
R9 Wasserstoff (H) oder 1-4C-Alkyl bedeutet,
R10 Wasserstoff (H) oder 1-4C-Alkyl bedeutet,
R11 Wasserstoff (H) oder 1-4C-Alkyl bedeutet,
R12 gemeinsam mit R7 1-4C-Alkylen bedeutet, das gewünschtenfalls durch eine oder mehrere 1-4C-Alkylgruppen substituiert ist,
R13 1-4C-Alkyl, Aryl, Aryl-1-4C-alkyl, Aryl-2-4C-alkenyl, Aryl-2-4C-alkinyl, 2-5C-Acyl, Arylcarbonyl oder Heteroarylcarbonyl bedeutet,

x die Zahl 1 bedeutet und
y und z gleich oder verschieden sind und die Zahlen 0, 1, 2, 3 oder 4 bedeuten, wobei die Summe von x, y und z eine Zahl von 1 bis 5 ist, und wobei
Aryl für einen Ring der Formel

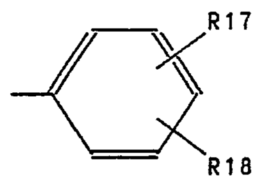

steht, worin R17 und R18 gleich oder verschieden sind und die Bedeutung Wasserstoff (H), 1-4C-Alkyl, 1-4C-Alkoxy, Halogen, Hydroxy oder Trifluormethyl haben, und
Heteroaryl für einen 5-oder 6-gliedrigen Heterocyclus mit einem oder zwei gleichen oder verschiedenen Heteroatomen aus der Gruppe Sauerstoff (O), Schwefel (S) oder Stickstoff (N) steht, der ungesättigt oder teilweise oder ganz gesättigt ist und der einen oder zwei Substituenten aus der Gruppe 1-4C-Alkyl, 1-4C-Alkoxy, Halogen, Trifluormethyl oder Cyan tragen kann,
und die Salze dieser Verbindungen.

Ein Aspekt der Ausgestaltung b kann charakterisiert werden durch die folgende Formel Ib

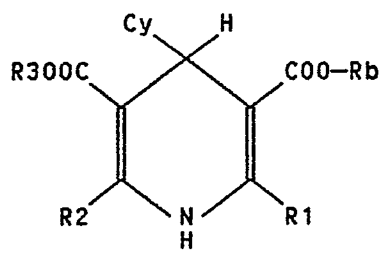

(Ib)

worin Rb einen der folgenden Cyclen darstellt:

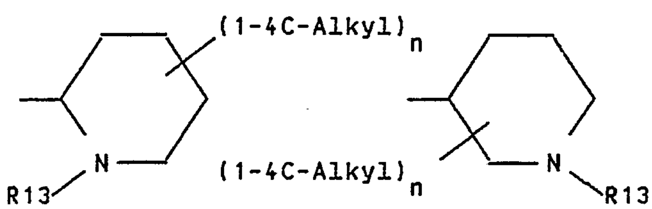

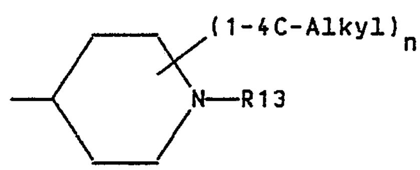

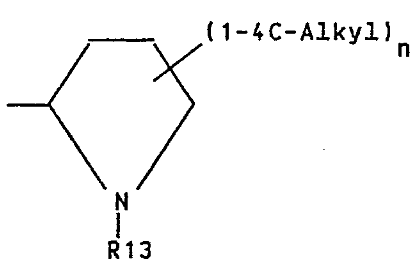

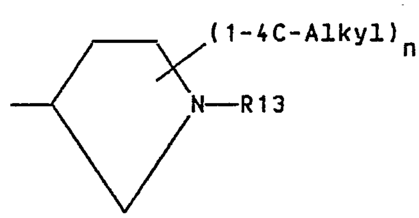

wobei
n die Zahl 0, 1, 2, 3 oder 4 bedeutet, und worin die übrigen Substituenten und Symbole die für die

Ausgestaltung b genannten Bedeutungen haben.

Hervorzuhebende Verbindungen der Ausgestaltung b sind solche der Formel Ib, worin

Cy Phenyl, 2-Nitrophenyl, 3-Nitrophenyl, 2-Cyanophenyl, 3-Cyanophenyl, 2-(1,1,2,2-Tetrafluorethoxy)-phenyl, 3-(1,1,2,2-Tetrafluorethoxy)-phenyl, 2-Difluormethoxyphenyl, 3-Difluormethoxyphenyl, 2-Chlorphenyl, 3-Chlorphenyl, 2,3-Dichlorphenyl, 2-Fluorphenyl, 3-Fluorphenyl, 2-Trifluormethylphenyl, 3-Trifluormethylphenyl, 3-Pyridyl oder Benzoxdiazolyl bedeutet,

R1 Methyl bedeutet,

R2 Methyl bedeutet,

R3 einen (-)-Menthylrest darstellt,

Rb einen der Cyclen

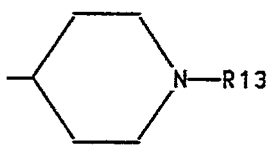 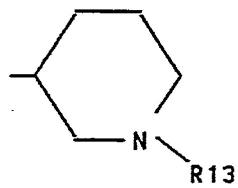 oder 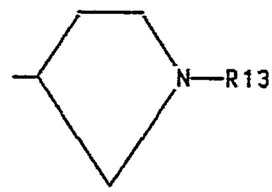

darstellt und

R13 1-4C-Alkyl, Benzyl, Phenethyl, 2-(3-Methoxyphenyl)-ethyl, 2-(3,4-Dimethoxyphenyl)-ethyl, 1-Phenylethyl, 4-Fluorbenzoyl oder Benzoyl bedeutet,

und ihre Salze.

Bevorzugte Verbindungen der Ausgestaltung b sind solche der Formel Ib, worin

Cy 3-Nitrophenyl, 2-Chlorphenyl, 2,3-Dichlorphenyl, 2-Trifluormethylphenyl, 2-Difluormethoxyphenyl oder Benzoxdiazolyl bedeutet

R1 Methyl bedeutet,

R2 Methyl bedeutet,

R3 einen (-)-Menthylrest darstellt,

Rb den Cyclus

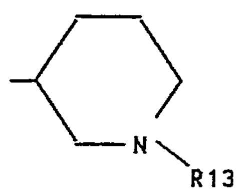 oder 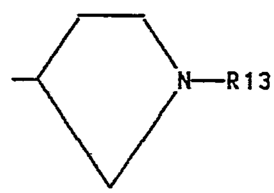

darstellt und

R13 Benzyl oder Phenethyl bedeutet,

und ihre Salze.

Besonders bevorzugte Verbindungen der Ausgestaltung b sind solche der Formel Ib, worin

Cy 3-Nitrophenyl bedeutet,

R1 Methyl bedeutet,

R2 Methyl bedeutet,

R3 einen (-)-Menthylrest darstellt,

Rb den Cyclus

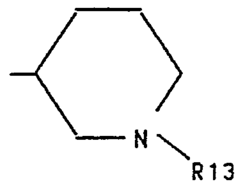

oder

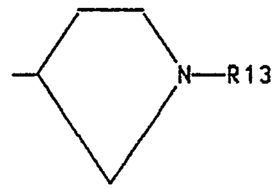

darstellt und

R13 Benzyl bedeutet,

und ihre Salze.

Als hervorzuhebende Verbindungen der Ausgestaltung b seien genannt:

1,4-Dihydro-2,6-dimethyl-4-(3-nitrophenyl)-pyridin-3,5-dicarbonsäure-3-(-)-menthyl-5-[1-(2-phenylethyl)-3-piperidyl]-ester,

1,4-Dihydro-2,6-dimethyl-4-(3-nitrophenyl)-pyridin-3,5-dicarbonsäure-3-(-)-menthyl-5-[1-(2-phenylethyl)-3-pyrrolidinyl]-ester

und ihre Salze.

Als bevorzugte Verbindungen der Ausgestaltung b seien genannt:

1,4-Dihydro-2,6-dimethyl-4-(2-chlorphenyl)-pyridin-3,5-dicarbonsäure-3-(-)-menthyl-5-(1-benzyl-3-piperidyl)-ester,

1,4-Dihydro-2,6-dimethyl-4-(2,3-dichlorphenyl)-pyridin-3,5-dicarbonsäure-3-(-)-menthyl-5-(1-benzyl-3-piperidyl)-ester,

1,4-Dihydro-2,6-dimethyl-4-(2-trifluormethylphenyl)-pyridin-3,5-dicarbonsäure-3-(-)-menthyl-5-(1-benzyl-3-piperidyl)-ester,

1,4-Dihydro-2,6-dimethyl-4-(2-difluormethoxyphenyl)-pyridin-3,5-dicarbon säure-3-(-)-menthyl-5-(1-benzyl-3-piperidyl)-ester,

1,4-Dihydro-2,6-dimethyl-4-(2,1,3-benzoxdiazol-4-yl)-pyridin-3,5-dicarbonsäure-3-(-)-menthyl-5-(1-benzyl-3-piperidyl)-ester,

1,4-Dihydro-2,6-dimethyl-4-(2-chlorphenyl)-pyridin-3,5-dicarbonsäure-3-(-)-menthyl-5-(1-benzyl-3-pyrrolidinyl)-ester,

1,4-Dihydro-2,6-dimethyl-4-(2,3-dichlorphenyl)-pyridin-3,5-dicarbonsäure-3-(-)-menthyl-5-(1-benzyl-3-pyrrolidinyl)-ester,

1,4-Dihydro-2,6-dimethyl-4-(2-trifluormethylphenyl)-pyridin-3,5-dicarbonsäure-3-(-)-menthyl-5-(1-benzyl-3-pyrrolidinyl)-ester,

1,4-Dihydro-2,6-dimethyl-4-(2-difluormethoxyphenyl)-pyridin-3,5-dicarbonsäure-3-(-)-menthyl-5-(1-benzyl-3-pyrrolidinyl)-ester,

1,4-Dihydro-2,6-dimethyl-4-(2,1,3-benzoxdiazol-4-yl)-pyridin-3,5-dicarbonsäure-3-(-)-menthyl-5-(1-benzyl-3-pyrrolidinyl)-ester

und ihre Salze.

Als besonders bevorzugte Verbindungen der Ausgestaltung b seien genannt:

1,4-Dihydro-2,6-dimethyl-4-(3-nitrophenyl)-pyridin-3,5-dicarbonsäure-3-(-)-menthyl-5-(1-benzyl-3-piperidyl)-ester,

1,4-Dihydro-2,6-dimethyl-4-(3-nitrophenyl)-pyridin-3,5-dicarbonsäure-3-(-)-menthyl-5-(1-benzyl-3-pyrrolidinyl)-ester

und ihre Salze.

Eine weitere Ausgestaltung (Ausgestaltung c) der Erfindung sind Verbindungen der Formel I, worin

Cy, R1, R2, R3, R4 und R5 die obengenannten Bedeutungen haben,

R6 Wasserstoff (H) bedeutet,

R7 Wasserstoff (H) bedeutet,

R8 Wasserstoff (H) oder 1-4C-Alkyl bedeutet,

R9 gemeinsam mit R12 1-4C-Alkylen bedeutet, das gewünschtenfalls durch eine oder mehrere 1-4C-Alkylgruppen substituiert ist,

R10 Wasserstoff (H) oder 1-4C-Alkyl bedeutet,

R11 Wasserstoff (H) oder 1-4C-Alkyl bedeutet,

R12 gemeinsam mit R9 1-4C-Alkylen bedeutet, das gewünschtenfalls durch eine oder mehrere 1-4C-Alkylgruppen substituiert ist,

R13 1-4C-Alkyl, Aryl, Aryl-1-4C-alkyl, Aryl-2-4C-alkenyl, Aryl-2-4C-alkinyl, 2-5C-Acyl, Arylcarbonyl oder

Heteroarylcarbonyl bedeutet,
y die Zahl 1 bedeutet und
x und z gleich oder verschieden sind und die Zahlen 0, 1, 2, 3 oder 4 bedeuten, wobei die Summe von x, y und z eine Zahl von 1 bis 5 ist, wobei
Aryl für einen Ring der Formel

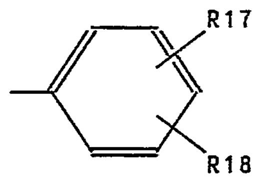

steht, worin R17 und R18 gleich oder verschieden sind und die Bedeutung Wasserstoff (H), 1-4C-Alkyl, 1-4C-Alkoxy, Halogen, Hydroxy oder Trifluormethyl haben, und
Heteroaryl für einen 5-oder 6-gliedrigen Heterocyclus mit einem oder zwei gleichen oder verschiedenen Heteroatomen aus der Gruppe Sauerstoff (O), Schwefel (S) oder Stickstoff (N) steht, der ungesättigt oder teilweise oder ganz gesättigt ist und der einen oder zwei Substituenten aus der Gruppe 1-4C-Alkyl, 1-4C-Alkoxy, Halogen, Trifluormethyl oder Cyan tragen kann,
und die Salze dieser Verbindungen.

Ein Aspekt der Ausgestaltung c kann charakterisiert werden durch die folgende Formel Ic

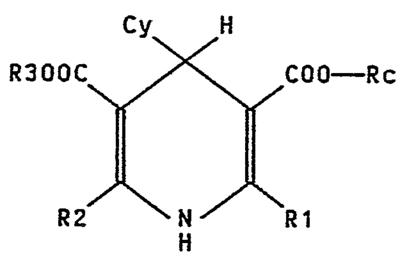

(Ic)

worin Rc einen der folgenden Cyclen darstellt:

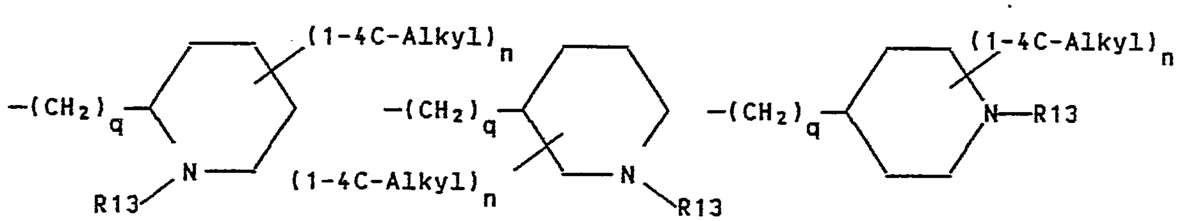

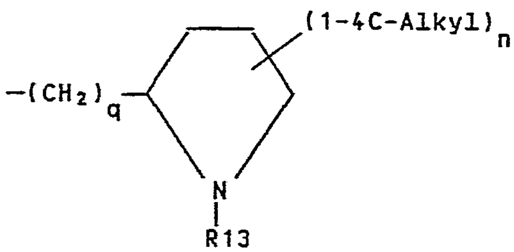

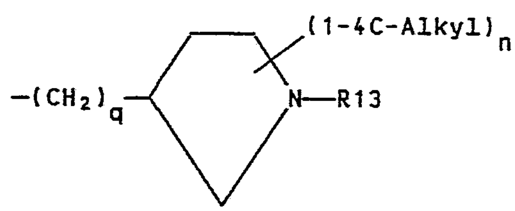

wobei
n die Zahl 0, 1, 2, 3 oder 4 bedeutet, q die Zahl 1 oder 2 bedeutet und worin die übrigen Substitutenten und Symbole die für die Ausgestaltung c genannten Bedeutungen haben.

Hervorzuhebende Verbindungen der Ausgestaltung c sind solche der Formel Ic, worin

Cy Phenyl, 2-Nitrophenyl, 3-Nitrophenyl, 2-Cyanophenyl, 3-Cyanophenyl, 2-(1,1,2,2-Tetrafluorethoxy)-phenyl, 3-(1,1,2,2-Tetrafluorethoxy)-phenyl, 2-Difluormethoxyphenyl, 3-Difluormethoxyphenyl, 2-Chlorphenyl, 3-Chlorphenyl, 2,3-Dichlorphenyl, 2-Fluorphenyl, 3-Fluorphenyl, 2-Trifluormethylphenyl, 3-Trifluormethylphenyl, 3-Pyridyl oder Benzoxdiazolyl bedeutet,

R1 Methyl bedeutet,

R2 Methyl bedeutet,

R3 einen (-)-Menthylrest darstellt,

Rc einen der Cyclen

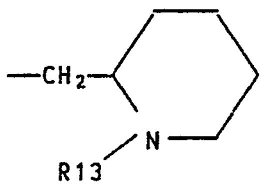 oder 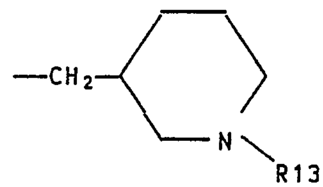

darstellt und

R13 1-4C-Alkyl, Benzyl, Phenethyl, 2-(3-Methoxyphenyl)-ethyl, 2-(3,4-Dimethoxyphenyl)-ethyl, 1-Phenylethyl 4-Fluorbenzoyl oder Benzoyl bedeutet,

und ihre Salze.

Bevorzugte Verbindungen der Ausgestaltung c sind solche der Formel Ic, worin

Cy 3-Nitrophenyl, 2-Chlorphenyl, 2,3-Dichlorphenyl, 2-Trifluormethylphenyl, 2-Difluormethoxyphenyl oder Benzoxdiazolyl bedeutet,

R1 Methyl bedeutet,

R2 Methyl bedeutet,

R3 einen (-)-Menthylrest darstellt,

Rc den Cyclus

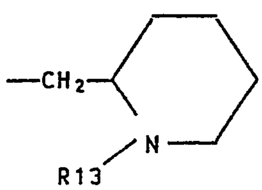 oder 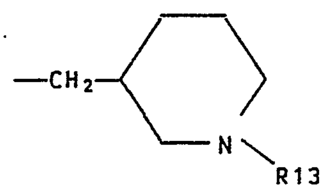

darstellt und

R13 Benzyl oder Phenethyl bedeutet,

und ihre Salze.

Besonders bevorzugte Verbindungen der Ausgestaltung c sind solche der Formel Ic, worin

Cy 3-Nitrophenyl bedeutet,

R1 Methyl bedeutet,

R2 Methyl bedeutet,

R3 einen (-)-Menthylrest darstellt,

Rc den Cyclus

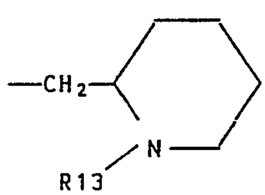 oder 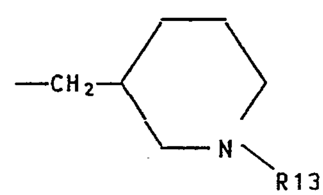

darstellt und

R13 Benzyl bedeutet,

und ihre Salze.

Als hervorzuhebende Verbindungen der Ausgestaltung c seien genannt:

1,4-Dihydro-2,6-dimethyl-4-(3-nitrophenyl)-pyridin-3,5-dicarbonsäure-3-(-)-menthyl-5-{[1-(2-phenylethyl)-2-piperidinyl]-methyl}-ester,

1,4-Dihydro-2,6-dimethyl-4-(3-nitrophenyl)-pyridin-3,5-dicarbonsäure-3-(-)-menthyl-5-{[1-(2-phenylethyl)-3-piperidinyl]-methyl}-ester

und ihre Salze.

Als bevorzugte Verbindungen der Ausgestaltung c seien genannt:

1,4-Dihydro-2,6-dimethyl-4-(2-chlorphenyl)-pyridin-3,5-dicarbonsäure-3-(-)-menthyl-5-[(1-benzyl-2-piperidinyl)-methyl]-ester,

1,4-Dihydro-2,6-dimethyl-4-(2,3-dichlorphenyl)-pyridin-3,5-dicarbonsäure-3-(-)-menthyl-5-[(1-benzyl-2-piperidinyl)-methyl]-ester,

1,4-Dihydro-2,6-dimethyl-4-(2-trifluormethylphenyl)-pyridin-3,5-dicarbonsäure-3-(-)-menthyl-5-[(1-benzyl-2-piperidinyl)-methyl]-ester,

1,4-Dihydro-2,6-dimethyl-4-(2-difluormethoxyphenyl)-pyridin-3,5-dicarbonsäure-3-(-)-menthyl-5-[(1-benzyl-2-piperidinyl)-methyl]-ester,

1,4-Dihydro-2,6-dimethyl-4-(2,1,3-benzoxdiazol-4-yl)-pyridin-3,5-dicarbonsäure-3-(-)-menthyl-5-[(1-benzyl-2-piperidinyl)-methyl]-ester,

1,4-Dihydro-2,6-dimethyl-4-(2-chlorphenyl)-pyridin-3,5-dicarbonsäure-3-(-)-menthyl-5-[(1-benzyl-3-piperidinyl)-methyl]-ester,

1,4-Dihydro-2,6-dimethyl-4-(2,3-dichlorphenyl)-pyridin-3,5-dicarbonsäure-3-(-)-menthyl-5-[(1-benzyl-3-piperidinyl)-methyl]-ester,

1,4-Dihydro-2,6-dimethyl-4-(2-trifluormethylphenyl)-pyridin-3,5-dicarbon säure-3-(-)-menthyl-5-[(1-benzyl-3-piperidinyl)-methyl]-ester,

1,4-Dihydro-2,6-dimethyl-4-(2-difluormethoxyphenyl)-pyridin-3,5-dicarbonsäure-3-(-)-menthyl-5-[(1-benzyl-3-piperidinyl)-methyl]-ester,

1,4-Dihydro-2,6-dimethyl-4-(2,1,3-benzoxdiazol-4-yl)-pyridin-3,5-dicarbonsäure-3-(-)-menthyl-5-[(1-benzyl-3-piperidinyl)-methyl]-ester

und ihre Salze.

Als besonders bevorzugte Verbindungen der Ausgestaltung c seien genannt:

1,4-Dihydro-2,6-dimethyl-4-(3-nitrophenyl)-pyridin-3,5-dicarbonsäure-3-(-)-menthyl-5-[(1-benzyl-2-piperidinyl)-methyl]-ester,

1,4-Dihydro-2,6-dimethyl-4-(3-nitrophenyl)-pyridin-3,5-dicarbonsäure-3-(-)-menthyl-5-[(1-benzyl-3-piperidinyl)-methyl]-ester

und ihre Salze.

Eine weitere Ausgestaltung (Ausgestaltung d) der Erfindung sind Verbindungen der Formel I, worin Cy, R1, R2, R3, R4 und R5 die obengenannten Bedeutungen haben,

R6 Wasserstoff (H) oder 1-4C-Alkyl bedeutet,

R7 Wasserstoff (H) oder 1-4C-Alkyl bedeutet,

R8 Wasserstoff (H) oder 1-4C-Alkyl bedeutet,

R9 Wasserstoff (H), 1-4C-Alkyl, 1-4C-Alkoxy, Aryl oder Heteroaryl bedeutet,

R10 Wasserstoff (H) oder 1-4C-Alkyl bedeutet,

R11 Wasserstoff (H) oder 1-4C-Alkyl bedeutet,

R12 und R13 gemeinsam und unter Einschluß des Stickstoffatoms, an das beide gebunden sind, einen Rest der Formel

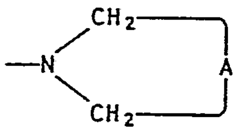

darstellen, worin

A $-CH_2-(CH_2)_m-$,

$-CH_2-O-CH_2-$oder

$-CH_2-S-CH_2-$bedeutet,

x, y und z gleich oder verschieden sind und die Zahlen 0, 1, 2, 3 oder 4 bedeuten, wobei die Summe von x,

y und z eine Zahl von 1 bis 5 ist, und
m die Zahl 1, 2 oder 3 bedeutet, wobei
Aryl für einen Ring der Formel

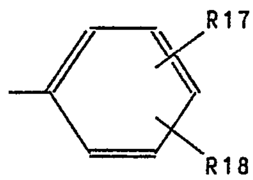

steht, worin R17 und R18 gleich oder verschieden sind und die Bedeutung Wasserstoff (H), 1-4C-Alkyl, 1-4C-Alkoxy, Halogen, Hydroxy oder Trifluormethyl haben, und
Heteroaryl für einen 5-oder 6-gliedrigen Heterocyclus mit einem oder zwei gleichen oder verschiedenen Heteroatomen aus der Gruppe Sauerstoff (O), Schwefel (S) oder Stickstoff (N) steht, der ungesättigt oder teilweise oder ganz gesättigt ist und der einen oder zwei Substituenten aus der Gruppe 1-4C-Alkyl, 1-4C-Alkoxy, Halogen, Trifluormethyl oder Cyan tragen kann,
und die Salze dieser Verbindungen.

Die Ausgestaltung d kann charakterisiert werden durch die folgende Formel Id

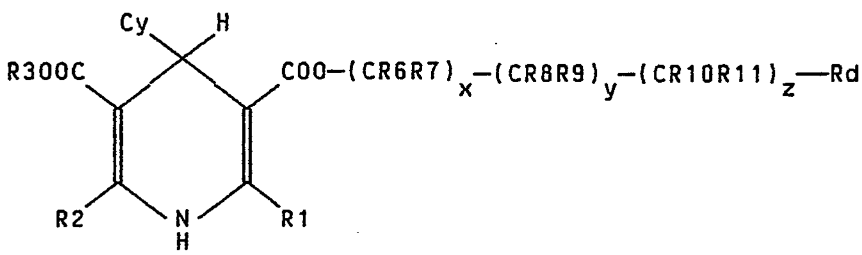

(Id)

worin Rd einen der folgenden Cyclen darstellt:

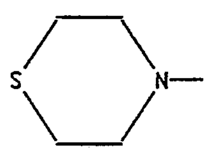

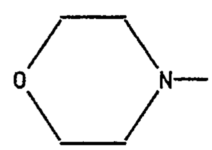

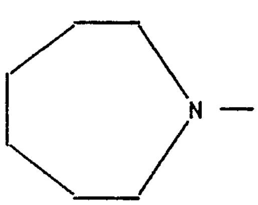

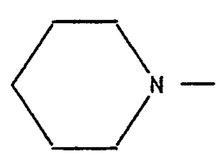

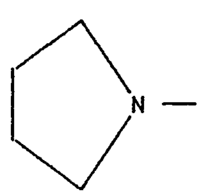

und die übrigen Substituenten und Symbole die für die Ausgestaltung d genannten Bedeutungen haben.

Hervorzuhebende Verbindungen der Ausgestaltung d sind solche der Formel Id, worin
Cy Phenyl, 2-Nitrophenyl, 3-Nitrophenyl, 2-Cyanophenyl, 3-Cyanophenyl, 2-(1,1,2,2-Tetrafluorethoxy)-phenyl, 3-(1,1,2,2-Tetrafluorethoxy)-phenyl, 2-Difluormethoxyphenyl, 3-Difluormethoxyphenyl, 2-Chlorphenyl, 3-Chlorphenyl, 2,3-Dichlorphenyl, 2-Fluorphenyl, 3-Fluorphenyl, 2-Trifluormethylphenyl, 3-Trifluormethylphenyl, 3-Pyridyl oder Benzoxdiazolyl bedeutet,
R1 Methyl bedeutet,
R2 Methyl bedeutet,

R3 einen (-)-Menthylrest darstellt,
R8 Wasserstoff bedeutet,
R9 Wasserstoff, Phenyl, 2-Chlorphenyl, 4-Chlorphenyl, 2-Methylphenyl, 4-Methylphenyl, 4-Fluorphenyl, 4-Methoxyphenyl, 2-Furyl, 2-Thienyl, 2-Pyridyl oder 3-Pyridyl bedeutet,
R10 Wasserstoff bedeutet,
R11 Wasserstoff bedeutet,
und Rd einen 1-Piperidino-, 1-Pyrrolidino-oder 4-Morpholinorest darstellt,
x die Zahl 0 darstellt,
y die Zahl 1 darstellt und
z die Zahl 1 oder 2 darstellt,
und ihre Salze.

Bevorzugte Verbindungen der Ausgestaltung d sind solche der Formel Id, worin
Cy 3-Nitrophenyl, 2-Chlorphenyl, 2,3-Dichlorphenyl, 2-Trifluormethylphenyl, 2-Difluormethoxyphenyl oder Benzoxdiazolyl bedeutet,
R1 Methyl bedeutet,
R2 Methyl bedeutet,
R3 einen (-)-Menthylrest darstellt,
die Gruppierung $(CR6R7)_x$-$(CR8R9)_y$-$(CR10R11)_z$ Ethylen oder Propylen bedeutet und
Rd einen 1-Piperidino-, 1-Pyrrolidino-oder 4-Morpholinorest darstellt,
und ihre Salze.

Besonders bevorzugte Verbindungen der Ausgestaltung d sind solche der Formel Id, worin
Cy 3-Nitrophenyl bedeutet,
R1 Methyl bedeutet,
R2 Methyl bedeutet,
R3 einen (-)-Menthylrest darstellt,
die Gruppierung $(CR6R7)_x$-$(CR8R9)_y$-$(CR10R11)_z$ Ethylen oder Propylen bedeutet und
Rd einen 1-Piperidino-, 1-Pyrrolidino-oder 4-Morpholinorest darstellt,
und ihre Salze.

Als bevorzugte Verbindungen der Ausgestaltung d seien genannt:
1,4-Dihydro-2,6-dimethyl-4-(2-chlorphenyl)-pyridin-3,5-dicarbonsäure-3-(-)-menthyl-5-[2-(4-morpholino)-ethyl]-ester,
1,4-Dihydro-2,6-dimethyl-4-(2,3-dichlorphenyl)-pyridin-3,5-dicarbonsäure-3-(-)-menthyl-5-[2-(4-morpholino)-ethyl]-ester,
1,4-Dihydro-2,6-dimethyl-4-(2-trifluormethylphenyl)-pyridin-3,5-dicarbonsäure-3-(-)-menthyl-5-[2-(4-morpholino)-ethyl]-ester,
1,4-Dihydro-2,6-dimethyl-4-(2-difluormethoxyphenyl)-pyridin-3,5-dicarbonsäure-3-(-)-menthyl-5-[2-(4-morpholino)-ethyl]-ester,
1,4-Dihydro-2,6-dimethyl-4-(2,1,3-benzoxdiazol-4-yl)-pyridin-3,5-dicarbon säure-3-(-)-menthyl-5-[2-(4-morpholino)-ethyl]-ester,

1,4-Dihydro-2,6-dimethyl-4-(2-chlorphenyl)-pyridin-3,5-dicarbonsäure-3-(-)-menthyl-5-[2-(1-piperidino)-ethy]-ester,
1,4-Dihydro-2,6-dimethyl-4-(2,3-dichlorphenyl)-pyridin-3,5-dicarbonsäure-3-(-)-menthyl-5-[2-(1-piperidino)-ethyl]-ester,
1,4-Dihydro-2,6-dimethyl-4-(2-trifluormethylphenyl)-pyridin-3,5-dicarbonsäure-3-(-)-menthyl-5-[2-(1-piperidino)-ethyl]-ester,
1,4-Dihydro-2,6-dimethyl-4-(2-difluormethoxyphenyl)-pyridin-3,5-dicarbonsäure-3-(-)-menthyl-5-[2-(1-piperidino)-ethyl]-ester,
1,4-Dihydro-2,6-dimethyl-4-(2,1,3-benzoxdiazol-4-yl)-pyridin-3,5-dicarbonsäure-3-(-)-menthyl-5-[2-(1-piperidino)-ethyl]-ester,

1,4-Dihydro-2,6-dimethyl-4-(2-chlorphenyl)-pyridin-3,5-dicarbonsäure-3-(-)-menthyl-5-[2-(1-pyrrolidino)-ethyl]-ester,
1,4-Dihydro-2,6-dimethyl-4-(2,3-dichlorphenyl)-pyridin-3,5-dicarbonsäure-3-(-)-menthyl-5-[2-(1-pyrrolidino)-ethyl]-ester,
1,4-Dihydro-2,6-dimethyl-4-(2-trifluormethylphenyl)-pyridin-3,5-dicarbonsäure-3-(-)-menthyl-5-[2-(1-pyrrolidino)-ethyl]-ester,
1,4-Dihydro-2,6-dimethyl-4-(2-difluormethoxyphenyl)-pyridin-3,5-dicarbonsäure-3-(-)-menthyl-5-[2-(1-pyrrolidino)-ethyl]-ester,
1,4-Dihydro-2,6-dimethyl-4-(2,1,3-benzoxdiazol-4-yl)-pyridin-3,5-dicarbonsäure-3-(-)-menthyl-5-[2-(1-pyrrolidino)-ethyl]-ester,

1,4-Dihydro-2,6-dimethyl-4-(2-chlorphenyl)-pyridin-3,5-dicarbonsäure-3-(-)-menthyl-5-[3-(4-morpholino)-propyl]-ester,
1,4-Dihydro-2,6-dimethyl-4-(2,3-dichlorphenyl)-pyridin-3,5-dicarbonsäure-3-(-)-menthyl-5-[3-(4-morpholino)-propyl]-ester,
1,4-Dihydro-2,6-dimethyl-4-(2-trifluormethylphenyl)-pyridin-3,5-dicarbonsäure-3-(-)-menthyl-5-[3-(4-morpholino)-propyl]-ester,
1,4-Dihydro-2,6-dimethyl-4-(2-difluormethoxyphenyl)-pyridin-3,5-dicarbonsäure-3-(-)-menthyl-5-[3-(4-morpholino)-propyl]-ester,
1,4-Dihydro-2,6-dimethyl-4-(2,1,3-benzoxdiazol-4-yl)-pyridin-3,5-dicarbonsäure-3-(-)-menthyl-5-[3-(4-morpholino)-propyl]-ester,

1,4-Dihydro-2,6-dimethyl-4-(2-chlorphenyl)-pyridin-3,5-dicarbonsäure-3-(-)-menthyl-5-[3-(1-piperidino)-propyl]-ester,
1,4-Dihydro-2,6-dimethyl-4-(2,3-dichlorphenyl)-pyridin-3,5-dicarbonsäure-3-(-)-menthyl-5-[3-(1-piperidino)-propyl]-ester,
1,4-Dihydro-2,6-dimethyl-4-(2-trifluormethylphenyl)-pyridin-3,5-dicarbonsäure-3-(-)-menthyl-5-[3-(1-piperidino)-propyl]-ester,
1,4-Dihydro-2,6-dimethyl-4-(2-difluormethoxyphenyl)-pyridin-3,5-dicarbonsäure-3-(-)-menthyl-5-[3-(1-piperidino)-propyl]-ester,
1,4-Dihydro-2,6-dimethyl-4-(2,1,3-benzoxdiazol-4-yl)-pyridin-3,5-dicarbonsäure-3-(-)-menthyl-5-[3-(1-piperidino)-propyl]-ester,

1,4-Dihydro-2,6-dimethyl-4-(2-chlorphenyl)-pyridin-3,5-dicarbonsäure-3-(-)-menthyl-5-[3-(1-pyrrolidino)-propyl]-ester,
1,4-Dihydro-2,6-dimethyl-4-(2,3-dichlorphenyl)-pyridin-3,5-dicarbonsäure-3-(-)-menthyl-5-[3-(1-pyrrolidino)-propyl]-ester,
1,4-Dihydro-2,6-dimethyl-4-(2-trifluormethylphenyl)-pyridin-3,5-dicarbonsäure-3-(-)-menthyl-5-[3-(1-pyrrolidino)-propyl]-ester,
1,4-Dihydro-2,6-dimethyl-4-(2-difluormethoxyphenyl)-pyridin-3,5-dicarbonsäure-3-(-)-menthyl-5-[3-(1-pyrrolidino)-propyl]-ester,
1,4-Dihydro-2,6-dimethyl-4-(2,1,3-benzoxdiazol-4-yl)-pyridin-3,5-dicarbonsäure-3-(-)-menthyl-5-[3-(1-pyrrolidino)-propyl]-ester
und ihre Salze.

Als besonders bevorzugte Verbindungen der Ausgestaltung d seien genannt:
1,4-Dihydro-2,6-dimethyl-4-(3-nitrophenyl)-pyridin-3,5-dicarbonsäure-3-(-)-menthyl-5-[2-(4-morpholino)-ethyl]-ester,
1,4-Dihydro-2,6-dimethyl-4-(3-nitrophenyl)-pyridin-3,5-dicarbonsäure-3-(-)-menthyl-5-[2-(1-piperidino)-ethyl]-ester,
1,4-Dihydro-2,6-dimethyl-4-(3-nitrophenyl)-pyridin-3,5-dicarbonsäure-3-(-)-menthyl-5-[2-(1-pyrrolidino)-ethyl]-ester,
1,4-Dihydro-2,6-dimethyl-4-(3-nitrophenyl)-pyridin-3,5-dicarbonsäure-3-(-)-menthyl-5-[3-(4-morpholino)-propyl]-ester,
1,4-Dihydro-2,6-dimethyl-4-(3-nitrophenyl)-pyridin-3,5-dicarbonsäure-3-(-)-menthyl-5-[3-(1-piperidino)-propyl]-ester,
1,4-Dihydro-2,6-dimethyl-4-(3-nitrophenyl)-pyridin-3,5-dicarbonsäure-3-(-)-menthyl-5-[3-(1-pyrrolidino)-propyl]-ester
und ihre Salze.

Eine weitere Ausgestaltung (Ausgestaltung e) der Erfindung sind Verbindungen der Formel I, worin
Cy, R1, R2, R3, R4 und R5 die obengenannten Bedeutungen haben,
R6 Wasserstoff (H) oder 1-4C-Alkyl bedeutet,
R7 Wasserstoff (H) oder 1-4C-Alkyl bedeutet,
R8 Wasserstoff (H) oder 1-4C-Alkyl bedeutet,
R9 Wasserstoff (H), 1-4C-Alkyl, 1-4C-Alkoxy, Aryl oder Heteroaryl bedeutet,

R10 Wasserstoff (H) oder 1-4C-Alkyl bedeutet,
R11 Wasserstoff (H) oder 1-4C-Alkyl bedeutet,
R12 und R13 gemeinsam und unter Einschluß des Stickstoffatoms, an das beide gebunden sind, einen Rest der Formel

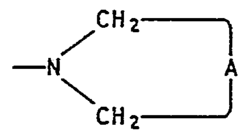

darstellen, worin
A $-CH_2-C(R14)R15-CH_2-$bedeutet,
R14 Wasserstoff (H) bedeutet,
R15 1-4C-Alkyl, 1-4C-Alkoxy, Hydroxy, Aryl, Arylcarbonyl oder Heteroaryl bedeutet, und
x, y und z gleich oder verschieden sind und die Zahlen 0, 1, 2, 3 oder 4 bedeuten, wobei die Summe von x, y und z eine Zahl von 1 bis 5 ist, und wobei Aryl für einen Ring der Formel

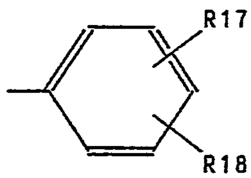

steht, worin R17 und R18 gleich oder verschieden sind und die Bedeutung Wasserstoff (H), 1-4C-Alkyl, 1-4C-Alkoxy, Halogen, Hydroxy oder Trifluormethyl haben, und
Heteroaryl für einen 5-oder 6-gliedrigen Heterocyclus mit einem oder zwei gleichen oder verschiedenen Heteroatomen aus der Gruppe Sauerstoff (O), Schwefel (S) oder Stickstoff (N) steht, der un gesättigt oder teilweise oder ganz gesättigt ist und der einen oder zwei Substituenten aus der Gruppe 1-4C-Alkyl, 1-4C-Alkoxy, Halogen, Trifluormethyl oder Cyan tragen kann,
und die Salze dieser Verbindungen.

Die Ausgestaltung e kann charakterisiert werden durch die folgende Formel I

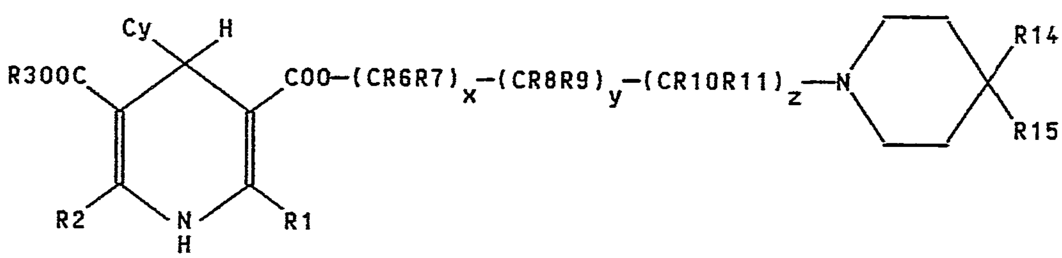

(Ie)

worin die Substituenten und Symbole die für die Ausgestaltung e genannten Bedeutungen haben.

Hervorzuhebende Verbindungen der Ausgestaltung e sind solche der Formel Ie, worin
Cy Phenyl, 2-Nitrophenyl, 3-Nitrophenyl, 2-Cyanophenyl, 3-Cyanophenyl, 2-(1,1,2,2-Tetrafluorethoxy)-phenyl, 3-(1,1,2,2-Tetrafluorethoxy)-phenyl, 2-Difluormethoxyphenyl, 3-Difluormethoxyphenyl, 2-Chlorphenyl, 3-Chlorphenyl, 2,3-Dichlorphenyl, 2-Fluorphenyl, 3-Fluorphenyl, 2-Trifluormethylphenyl, 3-Trifluormethylphenyl, 3-Pyridyl oder Benzoxdiazolyl bedeutet,
R1 Methyl bedeutet,
R2 Methyl bedeutet, -
R3 einen (-)-Menthylrest darstellt,

die Gruppierung $(CR6R7)_x$-$(CR8R9)_y$-$(CR10R11)_z$ Ethylen oder Propylen bedeutet,
R14 Wasserstoff (H) bedeutet und
R15 Aryl oder Arylcarbonyl bedeutet, wobei
Aryl für einen Ring der Formel

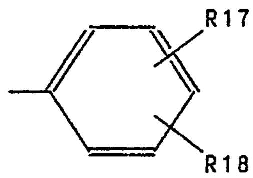

steht worin R17 und R18 gleich oder verschieden sind und die Bedeutung Wasserstoff (H), 1-4C-Alkyl, 1-4C-Alkoxy, Halogen, Hydroxy oder Trifluormethyl haben, und die Salze der Verbindungen.

Bevorzugte Verbindungen der Ausgestaltung e sind solche der Formel Ie, worin
Cy 3-Nitrophenyl, 2-Chlorphenyl, 2,3-Dichlorphenyl, 2-Trifluormethylphenyl, 2-Difluormethoxyphenyl oder Benzoxdiazolyl bedeutet,
R1 Methyl bedeutet,
R2 Methyl bedeutet,
R3 einen (-)-Menthylrest darstellt,
die Gruppierung $(CR6R7)_x$-$(CR8R9)_y$-$(CR10R11)_z$ Ethylen oder Propylen bedeutet,
R14 Wasserstoff bedeutet und
R15 Phenyl, Benzoyl, 4-Chlorbenzoyl oder 4-Fluorbenzoyl bedeutet,
und ihre Salze.

Besonders bevorzugte Verbindungen der Ausgestaltung e sind solche der Formel Ie, worin
Cy 3-Nitrophenyl bedeutet,
R1 Methyl bedeutet,
R2 Methyl bedeutet,
R3 einen (-)-Menthylrest darstellt,
die Gruppierung $(CR6R7)_x$-$(CR8R9)_y$-$(CR10R11)_z$ Ethylen oder Propylen bedeutet,
R14 Wasserstoff bedeutet und
R15 Phenyl oder 4-Fluorbenzoyl bedeutet,
und ihre Salze.

Als hervorzuhebende Verbindungen der Ausgestaltung e seien genannt:
1,4-Dihydro-2,6-dimethyl-4-(3-nitrophenyl)-pyridin-3,5-dicarbonsäure-3-(-)-menthyl-5-[2-(4-benzoylpiperidyl-1)-ethyl]-ester,
1,4-Dihydro-2,6-dimethyl-4-(3-nitrophenyl)-pyridin-3,5-dicarbonsäure-3-(-)-menthyl-5-[3-(4-benzoylpiperidyl-1)-propyl]-ester,
1,4-Dihydro-2,6-dimethyl-4-(3-nitrophenyl)-pyridin-3.5-dicarbonsäure-3-(-)-menthyl-5-{2-[4-(4-chlorbenzoyl)-piperidyl-1)-ethyl]-ester,
1,4-Dihydro-2,6-dimethyl-4-(3-nitrophenyl)-pyridin-3,5-dicarbonsäure-3-(-)-menthyl-5-{3-[4-(4-chlorbenzoyl)-piperidyl-1)-propyl]-ester
und ihre Salze.

Als bevorzugte Verbindungen der Ausgestaltung e seien genannt:
1,4-Dihydro-2,6-dimethyl-4-(2-chlorphenyl)-pyridin-3,5-dicarbonsäure-3-(-)-menthyl-5-[2-(4-phenylpiperidyl-1)-ethyl]-ester,
1,4-Dihydro-2,6-dimethyl-4-(2,3-dichlorphenyl)-pyridin-3,5-dicarbonsäure-3-(-)-menthyl-5[2-(4-phenylpiperidyl-1)-ethyl]-ester,
1,4-Dihydro-2,6-dimethyl-4-(2-trifluormethylphenyl)-pyridin-3,5-dicarbonsäure-3-(-)-menthyl-5-[2-(4-phenylpiperidyl-1)-ethyl]-ester,
1,4-Dihydro-2,6-dimethyl-4-(2-difluormethoxyphenyl)-pyridin-3,5-dicarbonsäure-3-(-)-menthyl-5-[2-(4-phenylpiperidyl-1)-ethyl]-ester,
1,4-Dihydro-2,6-dimethyl-4-(2,1,3-benzoxdiazol-4-yl)-pyridin-3,5-dicarbonsäure-3-(-)-menthyl-5-[2-(4-phenylpiperidyl-1)-ethyl]-ester,

1,4-Dihydro-2,6-dimethyl-4-(2-chlorphenyl)-pyridin-3,5-dicarbonsäure-3-(-)-menthyl-5-[3-(4-phenylpiperidyl-1)-propyl]-ester,

1,4-Dihydro-2,6-dimethyl-4-(2,3-dichlorphenyl)-pyridin-3,5-dicarbonsäure-3-(-)-menthyl-5-[3-(4-phenylpiperidyl-1)-propyl]-ester,
1,4-Dihydro-2,6-dimethyl-4-(2-trifluormethylphenyl)-pyridin-3,5-dicarbonsäure-3-(-)-menthyl-5-[3-(4-phenylpiperidyl-1)-propyl]-ester,
1,4-Dihydro-2,6-dimethyl-4-(2-difluormethoxyphenyl)-pyridin-3,5-dicarbonsäure-3-(-)-menthyl-5-3-(4-phenylpiperidyl-1)-propyl-ester,
1,4-Dihydro-2,6-dimethyl-4-(2,1,3-benzoxdiazol-4-yl)-pyridin-3,5-dicarbonsäure-3-(-)-menthyl-5-[3-(4-phenylpiperidyl-1)-propyl]-ester,

1,4-Dihydro-2,6-dimethyl-4-(2-chlorphenyl)-pyridin-3,5-dicarbonsäure-3-(-)-menthyl-5-{2-[4-(4-fluorbenzoyl)-piperidyl-1]-ethyl}-ester,
1,4-Dihydro-2,6-dimethyl-4-(2,3-dichlorphenyl)-pyridin-3,5-dicarbonsäure-3-(-)-menthyl-5-{2-[4-(4-fluorbenzoyl)-piperidyl-1]-ethyl}-ester,
1,4-Dihydro-2,6-dimethyl-4-(2-trifluormethylphenyl)-pyridin-3,5-dicarbon säure-3-(-)-menthyl-5-{2-[4-(4-fluorbenzoyl)-piperidyl-1]-ethyl}-ester,
1,4-Dihydro-2,6-dimethyl-4-(2,difluormethoxyphenyl)-pyridin-3,5-dicarbon-säure-3-(-)-menthyl-5-{2-[4-(4-fluorbenzoyl)-piperidyl-1]-ethyl}-ester,
1,4-Dihydro-2,,6-dimethyl-4-(2,1,3-benzoxdiazol-4-yl)-pyridin-3,5-dicarbonsäure-3-(-)-menthyl-5-{2-[4-(4-fluorbenzoyl)-piperidyl-1]-ethyl}-ester,

1,4-Dihydro-2,6-dimethyl-4-(2-chlorphenyl)-pyridin-3,5-dicarbonsäure-3-(-)-menthyl-5-{3-[4-(4-fluorbenzoyl)-piperidyl-1]-propyl}-ester,
1,4-Dihydro-2,6-dimethyl-4-(2,3-dichlorphenyl)-pyridin-3,5-dicarbonsäure-3-(-)-menthyl-5-{3-[4-(4-fluorbenzoyl)-piperidyl-1]-propyl}-ester,
1,4-Dihydro-2,6-dimethyl-4-(2-trifluormethylphenyl)-pyridin-3,5-dicarbonsäure-3-(-)-menthyl-5-{3-[4-(4-fluorbenzoyl)-piperidyl-1]-propyl}-ester,
1,4-Dihydro-2,6-dimethyl-4-(2-difluormethoxyphenyl)-pyridin-3,5-dicarbonsäure-3-(-)-menthyl-5-{3-[4-(4-fluorbenzoyl)-piperidyl-1]-propyl}-ester,
1,4-Dihydro-2,6-dimethyl-4-(2,1,3-benzoxdiazol-4-yl)-pyridin-3,5-dicarbonsäure-3-(-)-menthyl-5-{3-[4-(4-fluorbenzoyl)-piperidyl-1]-propyl}-ester
und ihre Salze.

Als besonders bevorzugte Verbindungen der Ausgestaltung e seien genannt:
1,4-Dihydro-2,6-dimethyl-4-(3-nitrophenyl)-pyridin-3,5-dicarbonsäure-3-(-)-menthyl-5-[2-(4-phenylpiperidyl-1)-ethyl]-ester,
1,4-Dihydro-2,6-dimethyl-4-(3-nitrophenyl)-pyridin-3,5-dicarbonsäure-3-(-)-menthyl-5-[3-(4-phenylpiperidyl-1)-propyl]-ester,
1,4-Dihydro-2,6-dimethyl-4-(3-nitrophenyl)-pyridin-3,5-dicarbonsäure-3-(-)-menthyl-5-{2-[4-(4-fluorbenzoyl)-piperidyl-1)-ethyl]-ester.
1,4-Dihydro-2,6-dimethyl-4-(3-nitrophenyl)-pyridin-3,5-dicarbonsäure-3-(-)-menthyl-5-{3-[4-(4-fluorbenzoyl)-piperidyl-1)-propyl]-ester
und ihre Salze.

Eine weitere Ausgestaltung (Ausgestaltung f) der Erfindung sind Verbindungen der Formel I, worin Cy, R1, R2, R3, R4 und R5 die obengenannten Bedeutungen haben,
R6 Wasserstoff (H) oder 1-4C-Alkyl bedeutet,
R7 Wasserstoff (H) oder 1-4C-Alkyl bedeutet,
R8 Wasserstoff (H) oder 1-4C-Alkyl bedeutet,
R9 Wasserstoff (H), 1-4C-Alkyl, 1-4C-Alkoxy, Aryl oder Heteroaryl bedeutet,
R10 Wasserstoff (H) oder 1-4C-Alkyl bedeutet,
R11 Wasserstoff (H) oder 1-4C-Alkyl bedeutet,
R12 und R13 gemeinsam und unter Einschluß des Stickstoffatoms, an das beide gebunden sind, einen Rest der Formel

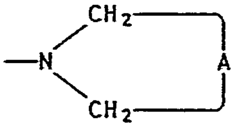

darstellen, worin
A -CH=CR15-$CH_2$-bedeutet,
R15 Wasserstoff (H), 1-4C-Alkyl, 1-4C-Alkoxy, Aryl, Arylcarbonyl oder Heteroaryl bedeutet,
x, y und z gleich oder verschieden sind und die Zahlen 0, 1, 2, 3 oder 4 bedeuten, wobei die Summe von x, y und z eine Zahl von 1 bis 5 ist, und wobei
Aryl für einen Ring der Formel

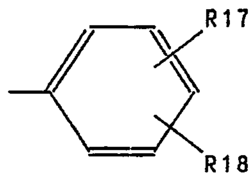

steht, worin R17 und R18 gleich oder verschieden sind und die Bedeutung Wasserstoff (H), 1-4C-Alkyl, 1-4C-Alkoxy, Halogen, Hydroxy oder Trifluormethyl haben, und
Heteroaryl für einen 5-oder 6-gliedrigen Heterocyclus mit einem oder zwei gleichen oder verschiedenen Heteroatomen aus der Gruppe Sauerstoff (O), Schwefel (S) oder Stickstoff (N) steht, der ungesättigt oder teilweise oder ganz gesättigt ist und der einen oder zwei Substituenten aus der Gruppe 1-4C-Alkyl, 1-4C-Alkoxy, Halogen, Trifluormethyl oder Cyan tragen kann,
und die Salze dieser Verbindungen.

Die Ausgestaltung f kann charakterisiert werden durch die folgende Formel If

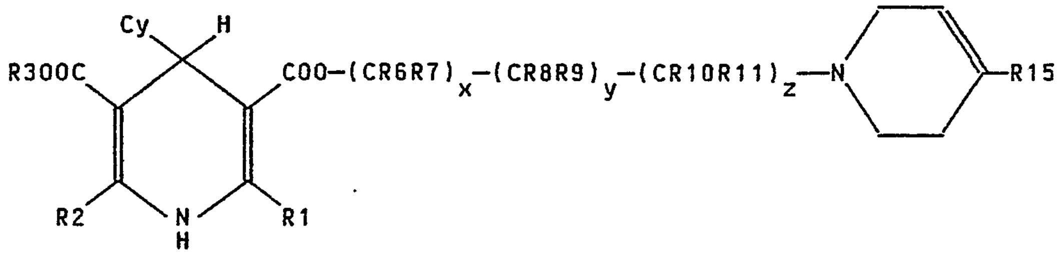

(If)

worin die Substituenten und Symbole die für die Ausgestaltung f genannten Bedeutungen haben.

Hervorzuhebende Verbindungen der Ausgestaltung f sind solche der Formel If, worin
Cy Phenyl, 2-Nitrophenyl, 3-Nitrophenyl, 2-Cyanophenyl, 3-Cyanophenyl, 2-(1,1,2,2-Tetrafluorethoxy)-phenyl, 3-(1,1,2,2-Tetrafluorethoxy)-phenyl, 2-Difluormethoxyphenyl, 3-Difluormethoxyphenyl, 2-Chlorphenyl, 3-Chlorphenyl, 2,3-Dichlorphenyl, 2-Fluorphenyl, 3-Fluorphenyl, 2-Trifluormethylphenyl, 3-Trifluormethylphenyl, 3-Pyridyl oder Benzoxdiazolyl bedeutet,
R1 Methyl bedeutet,
R2 Methyl bedeutet,
R3 einen (-)-Menthylrest darstellt,
die Gruppierung $(CR6R7)_x$-$(CR8R9)_y$-$(CR10R11)_z$ Ethylen oder Propylen bedeutet und
R15 Aryl bedeutet, wobei
Aryl für einen Ring der Formel

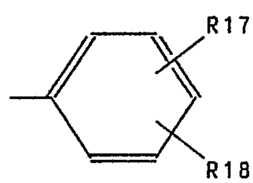

steht worin R17 und R18 gleich oder verschieden sind und die Bedeutung Wasserstoff (H), 1-4C-Alkyl, 1-4C-Alkoxy, Halogen, Hydroxy oder Trifluormethyl haben, und ihre Salze.

Bevorzugte Verbindungen der Ausgestaltung f sind solche der Formel If, worin
Cy 3-Nitrophenyl, 2-Chlorphenyl, 2,3-Dichlorphenyl, 2-Trifluormethylphenyl, 2-Difluormethoxyphenyl oder Benzoxdiazolyl bedeutet,
R1 Methyl bedeutet,
R2 Methyl bedeutet,
R3 einen (-)-Menthylrest darstellt,
die Gruppierung $(CR6R7)_x$-$(CR8R9)_y$-$(CR10R11)_z$ Ethylen oder Propylen bedeutet und
R15 Phenyl bedeutet,
und ihre Salze.

Besonders bevorzugte Verbindungen der Ausgestaltung f sind solche der Formel If, worin
Cy 3-Nitrophenyl bedeutet,
R1 Methyl bedeutet,
R2 Methyl bedeutet,
R3 einen (-)-Menthylrest darstellt,
die Gruppierung $(CR6R7)_x$-$(CR8R9)_y$-$(CR10R11)_z$ Ethylen oder Propylen bedeutet und
R15 Phenyl bedeutet,
und ihre Salze.

Als bevorzugte Verbindungen der Ausgestaltung f seien genannt:
1,4-Dihydro-2,6-dimethyl-4-(2-chlorphenyl)-pyridin-3,5-dicarbonsäure-3-(-)-menthyl-5-[2-(4-phenyl-1,2,3,6-tetrahydropyridyl-1)-ethyl]-ester,
1,4-Dihydro-2,6-dimethyl-4-(2,3-dichlorphenyl)-pyridin-3,5-dicarbonsäure-3-(-)-menthyl-5-[2-(4-phenyl-1,2,3,6-tetrahydropyridyl-1)-ethyl]-ester,
1,4-Dihydro-2,6-dimethyl-4-(2-trifluormethylphenyl)-pyridin-3,5-dicarbonsäure-3-(-)-menthyl-5-[2-(4-phenyl-1,2,3,6-tetrahydropyridyl-1)-ethyl]-ester,
1,4-Dihydro-2,6-dimethyl-4-(2-difluormethoxyphenyl)-pyridin-3,5-dicarbonsäure-3-(-)-menthyl-5-[2-(4-phenyl-1,2,3,6-tetrahydropyridyl-1)-ethyl]-ester,
1,4-Dihydro-2,6-dimethyl-4-(2,1,3-benzoxdiazol-4-yl)-pyridin-3,5-dicarbon säure-3-(-)-menthyl-5-[2-(4-phenyl-1,2,3,6-tetrahydropyridyl-1)-ethyl]-ester,
1,4-Dihydro-2,6-dimethyl-4-(2-chlorphenyl)-pyridin-3,5-dicarbonsäure-3-(-)-menthyl-5-[3-(4-phenyl-1,2,3,6-tetrahydropyridyl-1)-propyl]-ester,
1,4-Dihydro-2,6-dimethyl-4-(2,3-dichlorphenyl)-pyridin-3,5-dicarbonsäure-3-(-)-menthyl-5-[3-(4-phenyl-1,2,3,6-tetrahydropyridyl-1)-propyl]-ester,
1,4-Dihydro-2,6-dimethyl-4-(2-trifluormethylphenyl)-pyridin-3,5-dicarbonsäure-3-(-)-menthyl-5-[3-(4-phenyl-1,2,3,6-tetrahydropyridyl-1)-propyl]-ester,
1,4-Dihydro-2,6-dimethyl-4-(2-difluormethoxyphenyl)-pyridin-3,5-dicarbonsäure-3-(-)-menthyl-5-[3-(4-phenyl-1,2,3,6-tetrahydropyridyl-1)-propyl]-ester,
1,4-Dihydro-2,6-dimethyl-4-(2,1,3-benzoxdiazol-4-yl)-pyridin-3,5-dicarbonsäure-3-(-)-menthyl-5-[3-(4-phenyl-1,2,3,6-tetrahydropyridyl-1)-propyl]-ester
und ihre Salze.

Als besonders bevorzugte Verbindungen der Ausgestaltung f seien genannt:
1,4-Dihydro-2,6-dimethyl-4-(3-nitrophenyl)-pyridin-3,5-dicarbonsäure-3-(-)-menthyl-5-[2-(4-phenyl-1,2,3,6-tetrahydropyridyl-1)-ethyl]-ester und
1,4-Dihydro-2,6-dimethyl-4-(3-nitrophenyl)-pyridin-3,5-dicarbonsäure-3-(-)-menthyl-5-[3-(4-phenyl-1,2,3,6-tetrahydropyridyl-1)-propyl]-ester
und ihre Salze.

Eine weitere Ausgestaltung (Ausgestaltung g) der Erfindung sind Verbindungen der Formel I, worin
Cy, R1, R2, R3, R4 und R5 die obengenannten Bedeutungen haben,
R6 Wasserstoff (H) oder 1-4C-Alkyl bedeutet,
R7 Wasserstoff (H) oder 1-4C-Alkyl bedeutet,
R8 Wasserstoff (H) oder 1-4C-Alkyl bedeutet,
R9 Wasserstoff (H), 1-4C-Alkyl, 1-4C-Alkoxy, Aryl oder Heteroaryl bedeutet,
R10 Wasserstoff (H) oder 1-4C-Alkyl bedeutet,
R11 Wasserstoff (H) oder 1-4C-Alkyl bedeutet,
R12 und R13 gemeinsam und unter Einschluß des Stickstoffatoms, an das beide gebunden sind, einen Rest der Formel

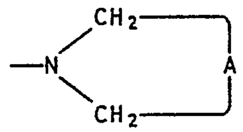

darstellen, worin
A $-CH_2-NR16-CH_2-$oder
$-(CH_2)_2-NR16-CH_2-$bedeutet,
R16 1-4C-Alkyl, Aryl, Aryl-1-4C-alkyl, Aryl-2-4C-alkenyl, Aryl-2-4C-alkinyl, Diaryl-1-4C-alkyl, Heteroaryl, Heteroaryl-1-4C-alkyl, Heteroaryl-aryl-1-4C-alkyl, Di-heteroaryl-1-4C-alkyl, Arylcarbonyl, Heteroarylcarbonyl, Arylsulfonyl, Aryl-1-4C-alkylcarbonyl oder Aryl-2-4C-alkenylcarbonyl bedeutet und
x, y und z gleich oder verschieden sind und die Zahlen 0, 1, 2, 3 oder 4 bedeuten, wobei die Summe von x, y und z eine Zahl von 1 bis 5 ist, und wobei
Aryl für einen Ring der Formel

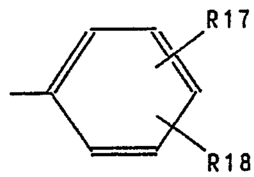

steht, worin R17 und R18 gleich oder verschieden sind und die Bedeutung Wasserstoff (H), 1-4C-Alkyl, 1-4C-Alkoxy, Halogen, Hydroxy, Trifluormethyl oder gemeinsam Methylendioxy haben, und Heteroaryl für einen 5-oder 6-gliedrigen Heterocyclus mit einem
oder zwei gleichen oder verschiedenen Heteroatomen aus der Gruppe Sauerstoff (O), Schwefel (S) oder Stickstoff (N) steht, der ungesättigt oder teilweise oder ganz gesättigt ist und der einen oder zwei Substituenten aus der Gruppe 1-4C-Alkyl, 1-4C-Alkoxy, Halogen, Trifluormethyl oder Cyan tragen kann,
und die Salze dieser Verbindungen.

Die Ausgestaltung g kann charakterisiert werden durch die folgende Formel Ig

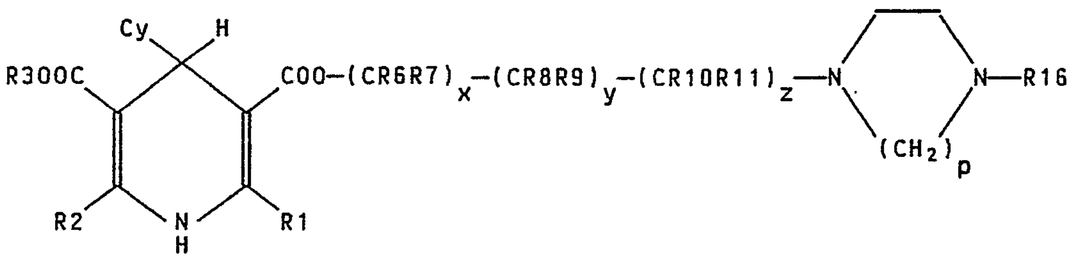 (Ig)

worin p die Zahl 2 oder 3 bedeutet und worin die übrigen Substituenten und Symbole die für die Ausgestaltung g genannten Bedeutungen haben.

Hervorzuhebende Verbindungen der Ausgestaltung g sind in einem ersten Aspekt solche der Formel Ig, worin

Cy Phenyl, 2-Nitrophenyl, 3-Nitrophenyl, 2-Cyanophenyl, 3-Cyanophenyl, 2-(1,1,2,2-Tetrafluorethoxy)-phenyl, 3-(1,1,2,2-Tetrafluorethoxy)-phenyl, 2-Difluormethoxyphenyl, 3-Difluormethoxyphenyl, 2-Chlorphenyl, 3-Chlorphenyl, 2,3-Dichlorphenyl, 2-Fluorphenyl, 3-Fluorphenyl, 2-Trifluormethylphenyl, 3-Trifluormethylphenyl, 3-Pyridyl oder Benzoxdiazolyl bedeutet,
R1 Methyl bedeutet,
R2 Methyl bedeutet,
R3 einen (-)-Menthylrest darstellt,
die Gruppierung $(CR6R7)_x-(CR8R9)_y-(CR10R11)_z$ Ethylen oder Propylen bedeutet,
R16 Aryl, 2-Pyridyl oder 2-Pyrimidyl bedeutet, wobei
Aryl für einen Ring der Formel

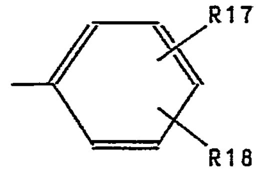

steht worin R17 und R18 gleich oder verschieden sind und die Bedeutung Wasserstoff (H), 1-4C-Alkyl, 1-4C-Alkoxy, Halogen, Hydroxy, Trifluormethyl oder gemeinsam Methylendioxy haben,
p die Zahl 2 darstellt,
und ihre Salze.

Hervorzuhebende Verbindungen der Ausgestaltung g sind in einem weiteren Aspekt solche der Formel Ig, worin
Cy Phenyl, 2-Nitrophenyl, 3-Nitrophenyl, 2-Cyanophenyl, 3-Cyanophenyl, 2-(1,1,2,2-Tetrafluorethoxy)-phenyl, 3-(1,1,2,2-Tetrafluorethoxy)-phenyl, 2-Difluormethoxyphenyl, 3-Difluormethoxyphenyl, 2-Chlorphenyl, 3-Chlorphenyl, 2,3-Dichlorphenyl, 2-Fluorphenyl, 3-Fluorphenyl, 2-Trifluormethylphenyl, 3-Trifluormethylphenyl, 3-Pyridyl oder Benzoxdiazolyl bedeutet,
R1 Methyl bedeutet,
R2 Methyl bedeutet,
R3 einen (-)-Menthylrest darstellt,
die Gruppierung $(CR6R7)_x$-$(CR8R9)_y$-$(CR10R11)_z$ Ethylen oder Propylen bedeutet,
R16 Diaryl-methyl bedeutet, wobei
Aryl für einen Ring der Formel

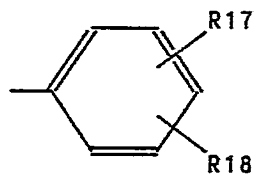

steht worin R17 und R18 gleich oder verschieden sind und die Bedeutung Wasserstoff (H), 1-4C-Alkyl, 1-4C-Alkoxy, Halogen, Hydroxy oder Trifluormethyl haben, und
p die Zahl 2 darstellt,
und ihre Salze.

Bevorzugte Verbindungen der Ausgestaltung g sind in einem ersten Aspekt solche der Formel Ig, worin
Cy 3-Nitrophenyl, 2-Chlorphenyl, 2,3-Dichlorphenyl, 2-Trifluormethylphenyl, 2-Difluormethoxyphenyl oder Benzoxdiazolyl bedeutet,
R1 Methyl bedeutet,
R2 Methyl bedeutet,
R3 einen (-)-Menthylrest darstellt,
die Gruppierung $(CR6R7)_x$-$(CR8R9)_y$-$(CR10R11)_z$ Ethylen oder Propylen bedeutet,
R16 Phenyl, 2-Methoxyphenyl, 2-Ethoxyphenyl, 3-Trifluormethylphenyl, 3-Methoxyphenyl, 3,4-Methylendioxyphenyl, 2-Methylphenyl, 2,4-Dimethylphenyl, 2,6-Dimethylphenyl oder 2-Pyridyl bedeutet, und
p die Zahl 2 darstellt,
und ihre Salze.

Besonders bevorzugte Verbindungen der Ausgestaltung g sind in einem ersten Aspekt solche der Formel Ig, worin
Cy 3-Nitrophenyl oder 2,3-Dichlorphenyl bedeutet,
R1 Methyl bedeutet,
R2 Methyl bedeutet,
R3 einen (-)-Menthylrest darstellt,
die Gruppierung $(CR6R7)_x$-$(CR8R9)_y$-$(CR10R11)_z$ Ethylen oder Propylen bedeutet,

R16 Phenyl, 2-Methoxyphenyl, 2-Ethoxyphenyl, 3-Trifluormethylphenyl, 3-Methoxyphenyl, 3,4-Methylendioxyphenyl, 2-Methylphenyl, 2,4-Dimethylphenyl, 2,6-Dimethylphenyl oder 2-Pyridyl bedeutet, und
p die Zahl 2 darstellt,
und ihre Salze.

Bevorzugte Verbindungen der Ausgestaltung g sind in einem weiteren Aspekt solche der Formel Ig, worin
Cy 3-Nitrophenyl, 2-Chlorphenyl, 2,3-Dichlorphenyl, 2-Trifluormethylphenyl, 2-Difluormethoxyphenyl oder Benzoxdiazolyl bedeutet,
R1 Methyl bedeutet,
R2 Methyl bedeutet,
R3 einen (-)-Menthylrest darstellt,
die Gruppierung $(CR6R7)_x$-$(CR8R9)_y$-$(CR10R11)_z$ Ethylen oder Propylen bedeutet,
R16 Benzhydryl bedeutet und
p die Zahl 2 darstellt,
und ihre Salze.

Besonders bevorzugte Verbindungen der Ausgestaltung g sind in einem weiteren Aspekt solche der Formel Ig, worin
Cy 3-Nitrophenyl bedeutet,
R1 Methyl bedeutet,
R2 Methyl bedeutet,
R3 einen (-)-Menthylrest darstellt,
die Gruppierung $(CR6R7)_x$-$(CR8R9)_y$-$(CR10R11)_z$ Ethylen oder Propylen bedeutet,
R16 Benzhydryl bedeutet und
p die Zahl 2 darstellt,
und ihre Salze.

Als bevorzugte Verbindungen der Ausgestaltung g seien genannt:
1,4-Dihydro-2,6-dimethyl-4-(2-chlorphenyl)-pyridin-3,5-dicarbonsäure-3-(-)-menthyl-5-{2-[4-(2-methoxyphenyl)-1-piperazinyl]-ethyl}-ester,
1,4-Dihydro-2,6-dimethyl-4-(2,3-dichlorphenyl)-pyridin-3,5-dicarbonsäure-3-(-)-menthyl-5-{2-[4-(2-methoxyphenyl)-1-piperazinyl]-ethyl}-ester,
1,4-Dihydro-2,6-dimethyl-4-(2-trifluormethylphenyl)-pyridin-3,5-dicarbonsäure-3-(-)-menthyl-5-{2-[4-(2-methoxyphenyl)-1-piperazinyl]-ethyl}-ester,
1,4-Dihydro-2,6-dimethyl-4-(2-difluormethoxyphenyl)-pyridin-3,5-dicarbonsäure-3-(-)-menthyl-5-{2-[4-(2-methoxyphenyl)-1-piperazinyl]-ethyl}-ester,
1,4-Dihydro-2,6-dimethyl-4-(2,1,3-benzoxdiazol-4-yl)-pyridin-3,5-dicarbonsäure-3-(-)-menthyl-5-{2-[4-(2-methoxyphenyl)-1-piperazinyl]-ethyl}-ester,

1,4-Dihydro-2,6-dimethyl-4-(2-chlorphenyl)-pyridin-3,5-dicarbonsäure-3-(-)-menthyl-5-{3-[4-(2-methoxyphenyl)-1-piperazinyl]-propyl-ester,
1,4-Dihydro-2,6-dimethyl-4-(2,3-dichlorphenyl)-pyridin-3,5-dicarbonsäure-3-(-)-menthyl-5-{3-[4-(2-methoxyphenyl)-1-piperazinyl]-propyl}-ester,
1,4-Dihydro-2,6-dimethyl-4-(2-trifluormethylphenyl)-pyridin-3,5-dicarbonsäure-3-(-)-menthyl-5-{3-[4-(2-methoxyphenyl)-1-piperazinyl]-propyl}-ester,
1,4-Dihydro-2,6-dimethyl-4-(2-difluormethoxyphenyl)-pyridin-3,5-dicarbonsäure-3-(-)-menthyl-5-{3-[4-(2-methoxyphenyl)-1-piperazinyl]-propyl}-ester,
1,4-Dihydro-2,6-dimethyl-4-(2,1,3-benzoxdiazol-4-yl)-pyridin-3,5-dicarbonsäure-3-(-)-menthyl-5-{3-[4-(2-methoxyphenyl)-1-piperazinyl]-propyl}-ester,

1,4-Dihydro-2,6-dimethyl-4-(2-chlorphenyl)-pyridin-3,5-dicarbonsäure-3-(-)-menthyl-5-[2-(4-phenyl-1-piperazinyl]-ethyl]-ester,
1,4-Dihydro-2,6-dimethyl-4-(2,3-dichlorphenyl)-pyridin-3,5-dicarbonsäure-3-(-)-menthyl-5-[2-(4-phenyl-1-piperazinyl]-ethyl]-ester,
1,4-Dihydro-2,6-dimethyl-4-(2-trifluormethylphenyl)-pyridin-3,5-dicarbonsäure-3-(-)-menthyl-5-[2-(4-phenyl-1-piperazinyl]-ethyl]-ester,
1,4-Dihydro-2,6-dimethyl-4-(2-difluormethoxyphenyl)-pyridin-3,5-dicarbonsäure-3-(-)-menthyl-5-[2-(4-phenyl-1-piperazinyl]-ethyl]-ester,
1,4-Dihydro-2,6-dimethyl-4-(2,1,3-benzoxdiazol-4-yl)-pyridin-3,5-dicarbonsäure-3-(-)-menthyl-5-[2-(4-phenyl-1-piperaziny-ethyl]-ester,

1,4-Dihydro-2,6-dimethyl-4-(2-chlorphenyl)-pyridin-3,5-dicarbonsäure-3-(-)-menthyl-5-[3-(4-phenyl-1-piperazinyl]-propyl]-ester,
1,4-Dihydro-2,6-dimethyl-4-(2,3-dichlorphenyl)-pyridin-3,5-dicarbonsäure-3-(-)-menthyl-5-[3-(4-phenyl-1-piperazinyl]-propyl]-ester,
1,4-Dihydro-2,6-dimethyl-4-(2-trifluormethylphenyl)-pyridin-3,5-dicarbonsäure-3-(-)-menthyl-5-[3-(4-phenyl-1-piperazinyl]-propyl]-ester,
1,4-Dihydro-2,6-dimethyl-4-(2-difluormethoxyphenyl)-pyridin-3,5-dicarbonsäure-3-(-)-menthyl-5-[3-(4-phenyl-1-piperazinyl]-propyl]-ester
, 1,4-Dihydro-2,6-dimethyl-4-(2,1,3-benzoxdiazol-4-yl)-pyridin-3,5-dicarbonsäure-3-(-)-menthyl-5-[3-(4-phenyl-1-piperazinyl]-propyl]-ester,

1,4-Dihydro-2,6-dimethyl-4-(2-chlorphenyl)-pyridin-3,5-dicarbonsäure-3-(-)-menthyl-5-{2-[4-(2-pyridyl)-1-piperazinyl]-ethyl}-ester,
1,4-Dihydro-2,6-dimethyl-4-(2,3-dichlorphenyl)-pyridin-3,5-dicarbonsäure-3-(-)-menthyl-5-{2-[4-(2-pyridyl)-1-piperazinyl]-ethyl}-ester,
1,4-Dihydro-2,6-dimethyl-4-(2-trifluormethylphenyl)-pyridin-3,5-dicarbonsäure-3-(-)-menthyl-5-{2-[4-(2-pyridyl)-1-piperazinyl]-ethyl}-ester,
1,4-Dihydro-2,6-dimethyl-4-(2-difluormethoxyphenyl)-pyridin-3,5-dicarbonsäure-3-(-)-menthyl-5-{2-[4-(2-pyridyl)-1-piperazinyl]-ethyl}-ester,
1,4-Dihydro-2,6-dimethyl-4-(2,1,3-benzoxdiazol-4-yl)-pyridin-3,5-dicarbonsäure-3-(-)-menthyl-5-{2-[4-(2-pyridyl)-1-piperazinyl]-ethyl}-ester,

1,4-Dihydro-2,6-dimethyl-4-(2-chlorphenyl)-pyridin-3,5-dicarbonsäure-3-(-)-menthyl-5-{3-[4-(2-pyridyl)-1-piperazinyl]-propyl}-ester,
1,4-Dihydro-2,6-dimethyl-4-(2,3-dichlorphenyl)-pyridin-3,5-dicarbonsäure-3-(-)-menthyl-5-{3-[4-(2-pyridyl)-1-piperazinyl]-propyl}-ester,
1,4-Dihydro-2,6-dimethyl-4-(2-trifluormethylphenyl)-pyridin-3,5-dicarbonsäure-3-(-)-menthyl-5-{3-[4-(2-pyridyl)-1-piperazinyl]-propyl}-ester,
1,4-Dihydro-2,6-dimethyl-4-(2-difluormethoxyphenyl)-pyridin-3,5-dicarbonsäure-3-(-)-menthyl-5-{3-[4-(2-pyridyl)-1-piperazinyl]-propyl}-ester,
1,4-Dihydro-2,6-dimethyl-4-(2,1,3-benzoxdiazol-4-yl)-pyridin-3,5-dicarbonsäure-3-(-)-menthyl-5-{3-[4-(2-pyridyl)-1-piperazinyl]-propyl}-ester,

1,4-Dihydro-2,6-dimethyl-4-(2-chlorphenyl)-pyridin-3,5-dicarbonsäure-3-(-)-menthyl-5-[2-(4-benzhydryl-1-piperazinyl)-ethyl]-ester,
1,4-Dihydro-2,6-dimethyl-4-(2,3-dichlorphenyl)-pyridin-3,5-dicarbonsäure-3-(-)-menthyl-5-[2-(4-benzhydryl-1-piperazinyl)-ethyl]-ester,
1,4-Dihydro-2,6-dimethyl-4-(2-trifluormethylphenyl)-pyridin-3,5-dicarbonsäure-3-(-)-menthyl-5-[2-(4-benzhydryl-1-piperazinyl)-ethyl]-ester,
1,4-Dihydro-2,6-dimethyl-4-(2-difluormethoxyphenyl)-pyridin-3,5-dicarbonsäure-3-(-)-menthyl-5-[2-(4-benzhydryl-1-piperazinyl)-ethyl]-ester,
1,4-Dihydro-2,6-dimethyl-4-(2,1,3-benzoxdiazol-4-yl)-pyridin-3,5-dicarbonsäure-3-(-)-menthyl-5-[2-(4-benzhydryl-1-piperazinyl)-ethyl]-ester,

1,4-Dihydro-2,6-dimethyl-4-(2-chlorphenyl)-pyridin-3,5-dicarbonsäure-3-(-)-menthyl-5-[3-(4-benzhydryl-1-piperazinyl)-propyl]-ester,
1,4-Dihydro-2,6-dimethyl-4-(2,3-dichlorphenyl)-pyridin-3,5-dicarbonsäure-3-(-)-menthyl-5-[3-(4-benzhydryl-1-piperazinyl)-propyl]-ester,
1,4-Dihydro-2,6-dimethyl-4-(2-trifluormethylphenyl)-pyridin-3,5-dicarbonsäure-3-(-)-menthyl-5-[3-(4-benzhydryl-1-piperazinyl)-propyl]-ester,
1,4-Dihydro-2,6-dimethyl-4-(2-difluormethoxyphenyl)-pyridin-3,5-dicarbonsäure-3-(-)-menthyl-5-[3-(4-benzhydryl-1-piperazinyl)-propyl]-ester,
1,4-Dihydro-2,6-dimethyl-4-(2,1,3-benzoxdiazol-4-yl)-pyridin-3,5-dicarbonsäure-3-(-)-menthyl-5-[3-(4-benzhydryl-1-piperazinyl)-propyl]-ester
und ihre Salze.

Als besonders bevorzugte Verbindungen der Ausgestaltung g seien genannt:
1,4-Dihydro-2,6-dimethyl-4-(3-nitrophenyl)-pyridin-3,5-dicarbonsäure-3-(-)-menthyl-5-{2-[4-(2-methoxyphenyl)-1-piperazinyl]-ethyl}-ester,
1,4-Dihydro-2,6-dimethyl-4-(3-nitrophenyl)-pyridin-3,5-dicarbonsäure-3-(-)-menthyl-5-{3-[4-(2-methoxyphenyl)-1-piperazinyl]-propyl}-ester,
1,4-Dihydro-2,6-dimethyl-4-(3-nitrophenyl)-pyridin-3,5-dicarbonsäure-3-(-)-menthyl-5-[2-(4-phenyl-1-piperazinyl)-ethyl]-ester,
1,4-Dihydro-2,6-dimethyl-4-(3-nitrophenyl)-pyridin-3,5-dicarbonsäure-3-(-)-menthyl-5-[3-(4-phenyl-1-piperazinyl)-propyl]-ester,
1,4-Dihydro-2,6-dimethyl-4-(3-nitrophenyl)-pyridin-3,5-dicarbonsäure-3-(-)-menthyl-5-{2-[4-(2-pyridyl)-1-piperazinyl]-ethyl}-ester,
1,4-Dihydro-2,6-dimethyl-4-(3-nitrophenyl)-pyridin-3,5-dicarbonsäure-3-(-)-menthyl-5-{3-[4-(2-pyridyl)-1-piperazinyl]-propyl}-ester,
1,4-Dihydro-2,6-dimethyl-4-(3-nitrophenyl)-pyridin-3,5-dicarbonsäure-3-(-)-menthyl-5-[2-(4-benzhydryl-1-piperazinyl)-ethyl]-ester,
1,4-Dihydro-2,6-dimethyl-4-(3-nitrophenyl)-pyridin-3,5-dicarbonsäure-3-(-)-menthyl-5-[3-(4-benzhydryl-1-piperazinyl)-propyl]-ester
1,4-Dihydro-2,6-dimethyl-4-(3-nitrophenyl)-pyridin-3,5-dicarbonsäure-3--(-)-menthyl-5-{3-[4-(2-ethoxyphenyl)-1-piperazinyl]-propyl}-ester
1,4-Dihydro-2,6-dimethyl-4-(3-nitrophenyl)-pyridin-3,5-dicarbonsäure-3--(-)-menthyl-5-{2-[4-(2-ethoxyphenyl)-1-piperazinyl]-ethyl}-ester
1,4-Dihydro-2,6-dimethyl-4-(3-nitrophenyl)-pyridin-3,5-dicarbonsäure-3-(-)-menthyl-5-{3-[4-(3-trifluormethylphenyl)-1-piperazinyl]-propyl}-ester
1,4-Dihydro-2,6-dimethyl-4-(3-nitrophenyl)-pyridin-3,5-dicarbonsäure-3-(-)-menthyl-5-{2-[4-(3-trifluormethylphenyl)-1-piperazinyl]-ethyl}-ester
1,4-Dihydro-2,6-dimethyl-4-(3-nitrophenyl)-pyridin-3,5-dicarbonsäure-3-(-)-menthyl-5-{3-[4-(3-methoxyphenyl)-1-piperazinyl]-propyl}-ester
1,4-Dihydro-2,6-dimethyl-4-(3-nitrophenyl)-pyridin-3,5-dicarbonsäure-3-(-)-menthyl-5-{2-[4-(3-methoxyphenyl)-1-piperazinyl]-ethyl}-ester
1,4-Dihydro-2,6-dimethyl-4-(3-nitrophenyl)-pyridin-3,5-dicarbonsäure-3-(-)-menthyl-5-{3-[4-(3,4-methylendioxyphenyl)-1-piperazinyl]-propyl}-ester
1,4-Dihydro-2,6-dimethyl-4-(3-nitrophenyl)-pyridin-3,5-dicarbonsäure-3-(-)-menthyl-5-{2-[4-(3,4-methylendioxyphenyl)-1-piperazinyl]-ethyl}-ester
1,4-Dihydro-2,6-dimethyl-4-(3-nitrophenyl)-pyridin-3,5-dicarbonsäure-3-(-)-menthyl-5-{3-[4-(2-tolyl)-1-piperazinyl]-propyl}-ester
1,4-Dihydro-2,6-dimethyl-4-(3-nitrophenyl)-pyridin-3,5-dicarbonsäure-3-(-)-menthyl-5-{2-[4-(2-tolyl)-1-piperazinyl]-ethyl}-ester
1,4-Dihydro-2,6-dimethyl-4-(3-nitrophenyl)-pyridin-3,5-dicarbonsäure-3-(-)-menthyl-5-{3-[4-(2,4-dimethylphenyl)-1-piperazinyl]-propyl}-ester
1,4-Dihydro-2,6-dimethyl-4-(3-nitrophenyl)-pyridin-3,5-dicarbonsäure-3-(-)-menthyl-5-{2-[4-(2,4-dimethylphenyl)-1-piperazinyl]-ethyl}-ester
1,4-Dihydro-2,6-dimethyl-4-(3-nitrophenyl)-pyridin-3,5-dicarbonsäure-3-(-)-menthyl-5-{3-[4-(2,6-dimethylphenyl)-1-piperazinyl]-propyl}-ester
1,4-Dihydro-2,6-dimethyl-4-(3-nitrophenyl)-pyridin-3,5-dicarbonsäure-3-(-)-menthyl-5-{2-[4-(2,6-dimethylphenyl)-1-piperazinyl]-ethyl}-ester
4-(2,3-Dichlorphenyl)-1,4-dihydro-2,6-dimethyl-pyridin-3,5-dicarbonsäure--3-(-)-menthyl-5-[3-(4-phenyl-1-piperazinyl)-propyl]-ester
4-(2,3-Dichlorphenyl)-1,4-dihydro-2,6-dimethyl-pyridin-3,5-dicarbonsäure--3-(-)-menthyl-5-[2-(4-phenyl-1-piperazinyl)-ethyl]-ester
4-(2,3-Dichlorphenyl)-1,4-dihydro-2,6-dimethyl-pyridin-3,5-dicarbonsäure--3-(-)-menthyl-5-{3-[4-(2-methoxyphenyl)-1-piperazinyl]-propyl}-ester
4-(2,3-Dichlorphenyl)-1,4-dihydro-2,6-dimethyl-pyridin-3,5-dicarbonsäure--3-(-)-menthyl-5-{2-[4-(2-methoxyphenyl)-1-piperazinyl]-ethyl}-ester
4-(2,3-Dichlorphenyl)-1,4-dihydro-2,6-dimethyl-pyridin-3,5-dicarbonsäure--3-(-)-menthyl-5-{3-[4-(2-ethoxyphenyl)-1-piperazinyl]-propyl}-ester
4-(2,3-Dichlorphenyl)-1,4-dihydro-2,6-dimethyl-pyridin-3,5-dicarbonsäure--3-(-)-menthyl-5-{2-[4-(2-ethoxyphenyl)-1-piperazinyl]-ethyl}-ester
und ihre Salze.

Eine weitere Ausgestaltung (Ausgestaltung h) der Erfindung sind Verbindungen der Formel I, worin
Cy, R1, R2, R3, R4 und R5 die obengenannten Bedeutungen haben,
R6 Wasserstoff (H) oder 1-4C-Alkyl bedeutet,
R7 Wasserstoff (H) oder 1-4C-Alkyl bedeutet,
R8 Wasserstoff (H) oder 1-4C-Alkyl bedeutet,
R9 Wasserstoff (H), 1-4C-Alkyl, 1-4C-Alkoxy, Aryl oder Heteroaryl bedeutet,
R10 Wasserstoff (H) oder 1-4C-Alkyl bedeutet,
R11 Wasserstoff (H) oder 1-4C-Alkyl bedeutet,
R12 und R13 gemeinsam und unter Einschluß des Stickstoffatoms, an das beide gebunden sind, einen Rest der Formel

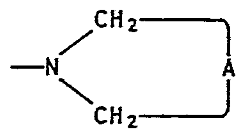

darstellen, worin
A $-CH_2-C(R14)R15-CH_2-$bedeutet,
R14 Aryl bedeutet,
R15 Aryl bedeutet und
x, y und z gleich oder verschieden sind und die Zahlen 0, 1, 2, 3 oder 4 bedeuten, wobei die Summe von x, y und z eine Zahl von 1 bis 5 ist, und wobei
Aryl für einen Ring der Formel

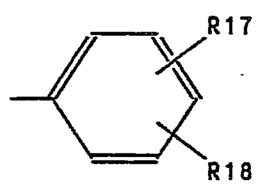

steht, worin R17 und R18 gleich oder verschieden sind und die Bedeutung Wasserstoff (H), 1-4C-Alkyl, 1-4C-Alkoxy, Halogen, Hydroxy oder Trifluormethyl haben, und
Heteroaryl für einen 5-oder 6-gliedrigen Heterocyclus mit einem oder zwei gleichen oder verschiedenen Heteroatomen aus der Gruppe Sauerstoff (O), Schwefel (S) oder Stickstoff (N) steht, der ungesättigt oder teilweise oder ganz gesättigt ist und der einen oder zwei Substituenten aus der Gruppe 1-4C-Alkyl, 1-4C-Alkoxy, Halogen, Trifluormethyl oder Cyan tragen kann,
und die Salze dieser Verbindungen.

Die Ausgestaltung h kann charakterisiert werden durch die folgende Formel Ih

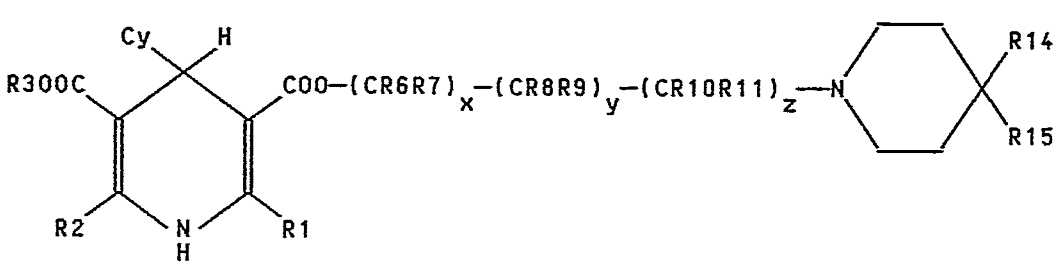

(Ih)

worin die Substituenten und Symbole die für die Ausgestaltung h genannten Bedeutungen haben.

Hervorzuhebende Verbindungen der Ausgestaltung h sind solche der Formel Ih, worin
Cy Phenyl, 2-Nitrophenyl, 3-Nitrophenyl, 2-Cyanophenyl, 3-Cyanophenyl, 2-(1,1,2,2-Tetrafluorethoxy)-phenyl, 3-(1,1,2,2-Tetrafluorethoxy)-phenyl, 2-Difluormethoxyphenyl, 3-Difluormethoxyphenyl, 2-Chlorphenyl, 3-Chlorphenyl, 2,3-Dichlorphenyl, 2-Fluorphenyl, 3-Fluorphenyl, 2-Trifluormethylphenyl, 3-Trifluormethylphenyl, 3-Pyridyl oder Benzoxdiazolyl bedeutet,
R1 Methyl bedeutet,
R2 Methyl bedeutet,
R3 einen (-)-Menthylrest darstellt,
die Gruppierung $(CR6R7)_x$-$(CR8R9)_y$-$(CR10R11)_z$ Ethylen oder Propylen bedeutet,
R14 Aryl bedeutet und
R15 Aryl bedeutet, wobei
Aryl für einen Ring der Formel

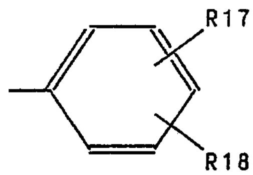

steht, worin R17 und R18 gleich oder verschieden sind und die Bedeutung Wasserstoff (H), 1-4C-Alkyl, 1-4C-Alkoxy, Halogen, Hydroxy oder Trifluormethyl haben, und die Salze der Verbindungen.

Bevorzugte Verbindungen der Ausgestaltung h sind solche der Formel Ih, worin
Cy 3-Nitrophenyl, 2-Chlorphenyl, 2,3-Dichlorphenyl, 2-Trifluormethylphenyl, 2-Difluormethoxyphenyl, 3-Pyridyl oder Benzoxdiazolyl bedeutet,
R1 Methyl bedeutet,
R2 Methyl bedeutet,
R3 einen (-)-Menthylrest darstellt,
die Gruppierung $(CR6R7)_x$-$(CR8R9)_y$-$(CR10R11)_z$ Ethylen oder Propylen be deutet,
R14 Phenyl oder 4-Methoxyphenyl bedeutet und
R15 Phenyl oder 4-Methoxyphenyl bedeutet,
und ihre Salze.

Besonders bevorzugte Verbindungen der Ausgestaltung h sind solche der Formel Ih, worin
Cy 3-Nitrophenyl bedeutet,
R1 Methyl bedeutet,
R2 Methyl bedeutet,
R3 einen (-)-Menthylrest darstellt,
die Gruppierung $(CR6R7)_x$-$(CR8R9)_y$-$(CR10R11)_z$ Ethylen oder Propylen bedeutet,
R14 Phenyl und
R15 Phenyl bedeutet,
und ihre Salze.

Als hervorzuhebende Verbindungen der Ausgestaltung h seien genannt:
1,4-Dihydro-2,6-dimethyl-4-(3-nitrophenyl)-pyridin-3,5-dicarbonsäure-3-(-)-menthyl-5-{2-[4,4-di-(4-methoxyphenyl)-piperidyl-1]-ethyl}-ester,
1,4-Dihydro-2,6-dimethyl-4-(3-nitrophenyl)-pyridin-3,5-dicarbonsäure-3-(-)-menthyl-5-{3-[4,4-di-(4-methoxyphenyl)-piperidyl-1]-propyl}-ester
und ihre Salze.

Als bevorzugte Verbindungen der Ausgestaltung h seien genannt:
1,4-Dihydro-2,6-dimethyl-4-(2-chlorphenyl)-pyridin-3,5-dicarbonsäure-3-(-)-menthyl-5-[2-(4,4-diphenylpiperidyl-1)-ethyl]-ester,
1,4-Dihydro-2,6-dimethyl-4-(2,3-dichlorphenyl)-pyridin-3,5-dicarbonsäure-3-(-)-menthyl-5-[2-(4,4-diphenylpiperidyl-1)-ethyl]-ester,
1,4-Dihydro-2,6-dimethyl-4-(2-trifluormethylphenyl)-pyridin-3,5-dicarbonsäure-3-(-)-menthyl-5-[2-(4,4-diphenylpiperidyl-1)-ethyl]-ester,
1,4-Dihydro-2,6-dimethyl-4-(2-difluormethoxyphenyl)-pyridin-3,5-dicarbonsäure-3-(-)-menthyl-5-[2-(4,4-diphenylpiperidyl-1)-ethyl]-ester,
1,4-Dihydro-2,6-dimethyl-4-(2,1,3-benzoxdiazol-4-yl)-pyridin-3,5-dicarbonsäure-3-(-)-menthyl-5-[2-(4,4-diphenylpiperidyl-1)-ethyl]-ester,
1,4-Dihydro-2,6-dimethyl-4-(2-chlorphenyl)-pyridin-3,5-dicarbonsäure-3-(-)-menthyl-5-[3-(4,4-diphenylpiperidyl-1)-propyl]-ester,
1,4-Dihydro-2,6-dimethyl-4-(2,3-dichlorphenyl)-pyridin-3,5-dicarbonsäure-3-(-)-menthyl-5-[3-(4,4-diphenylpiperidyl-1)-propyl]-ester,
1,4-Dihydro-2,6-dimethyl-4-(2-trifluormethylphenyl)-pyridin-3,5-dicarbonsäure-3-(-)-menthyl-5-[3-(4,4-diphenylpiperidyl-1)-propyl]-ester,
1,4-Dihydro-2,6-dimethyl-4-(2-difluormethoxyphenyl)-pyridin-3,5-dicarbonsäure-3-(-)-menthyl-5-[3-(4,4-diphenylpiperidyl-1)-propyl]-ester,
1,4-Dihydro-2,6-dimethyl-4-(2,1,3-benzoxdiazol-4-yl)-pyridin-3,5-dicarbonsäure-3-(-)-menthyl-5-[3-(4,4-diphenylpiperidyl-1)-propyl]-ester
und ihre Salze.

Als besonders bevorzugte Verbindungen der Ausgestaltung h seien genannt:
1,4-Dihydro-2,6-dimethyl-4-(3-nitrophenyl)-pyridin-3,5-dicarbonsäure-3-(-)-menthyl-5-[2-(4,4-diphenylpiperidyl-1)-ethyl]-ester,
1,4-Dihydro-2,6-dimethyl-4-(3-nitrophenyl)-pyridin-3,5-dicarbonsäure-3-(-)-menthyl-5-[3-(4,4-diphenylpiperidyl-1)-propyl]-ester
1,4-Dihydro-2,6-dimethyl-4-(3-pyridyl)-pyridin-3,5-dicarbonsäure-3-(-)-menthyl-5-[2-(4,4-diphenylpiperidyl-1)-ethyl]-ester,
1,4-Dihydro-2,6-dimethyl-4-(3-pyridyl)-pyridin-3,5-dicarbonsäure-3-(-)-menthyl-5-[3-(4,4-diphenylpiperidyl-1)-propyl]-ester
und ihre Salze.

Eine weitere Ausgestaltung (Ausgestaltung i) der Erfindung sind Verbindungen der Formel I, worin
Cy, R1, R2, R3, R4 und R5 die obengenannten Bedeutungen haben,
R6 Wasserstoff (H) oder 1-4C-Alkyl bedeutet,
R7 Wasserstoff (H) oder 1-4C-Alkyl bedeutet,
R8 Wasserstoff (H) oder 1-4C-Alkyl bedeutet,
R9 Wasserstoff (H), 1-4C-Alkyl, 1-4C-Alkoxy, Aryl oder Heteroaryl bedeutet,
R10 Wasserstoff (H) oder 1-4C-Alkyl bedeutet,
R11 Wasserstoff (H) oder 1-4C-Alkyl bedeutet,
R12 und R13 gemeinsam und unter Einschluß des Stickstoffatoms, an das beide gebunden sind, einen Rest der Formel

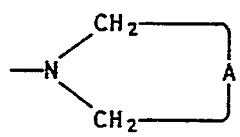

darstellen, worin
A $-CH_2-(R14)R15-CH_2-$bedeutet,
R14 und R15 gemeinsam Diarylmethylen bedeuten und
x, y und z gleich oder verschieden sind und die Zahlen 0, 1, 2, 3 oder 4 bedeuten, wobei die Summe von x, y und z eine Zahl von 1 bis 5 ist, und wobei
Aryl für einen Ring der Formel

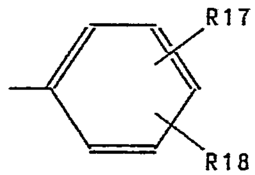

steht, worin R17 und R18 gleich oder verschieden sind und die Bedeutung Wasserstoff (H), 1-4C-Alkyl, 1-4C-Alkoxy, Halogen, Hydroxy oder Trifluormethyl haben, und
Heteroaryl für einen 5-oder 6-gliedrigen Heterocyclus mit einem oder zwei gleichen oder verschiedenen Heteroatomen aus der Gruppe Sauerstoff (O), Schwefel (S) oder Stickstoff (N) steht, der ungesättigt oder teilweise oder ganz gesättigt ist und der einen oder zwei Substituenten aus der Gruppe 1-4C-Alkyl, 1-4C-Alkoxy, Halogen, Trifluormethyl oder Cyan tragen kann,
und die salze dieser Verbindungen.

Die Ausgestaltung i kann charakterisiert werden durch die folgende Formel Ii

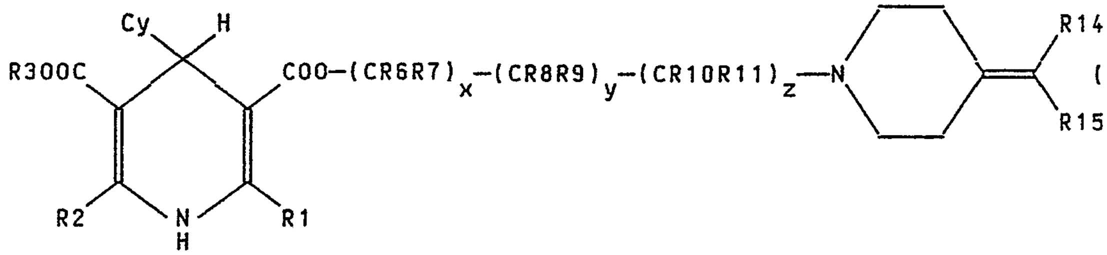

(Ii)

worin die Substituenten und Symbole die für die Ausgestaltung i genannten Bedeutungen haben.

Hervorzuhebende Verbindungen der Ausgestaltung i sind solche der Formel Ii, worin
Cy Phenyl, 2-Nitrophenyl, 3-Nitrophenyl, 2-Cyanophenyl, 3-Cyanophenyl, 2-(1,1,2,2-Tetrafluorethoxy)-phenyl, 3-(1,1,2,2-Tetrafluorethoxy)-phenyl, 2-Difluormethoxyphenyl, 3-Difluormethoxyphenyl, 2-Chlorphenyl, 3-Chlorphenyl, 2,3-Dichlorphenyl, 2-Fluorphenyl, 3-Fluorphenyl, 2-Trifluormethylphenyl, 3-Trifluormethylphenyl, 3-Pyridyl oder Benzoxdiazolyl bedeutet,
R1 Methyl bedeutet,
R2 Methyl bedeutet,
R3 einen (-)-Menthylrest darstellt,
die Gruppierung $(CR6R7)_x$-$(CR8R9)_y$-$(CR10R11)_z$ Ethylen oder Propylen bedeutet,
R14 Aryl bedeutet und
R15 Aryl bedeutet, wobei
Aryl für einen Ring der Formel

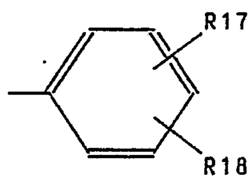

steht, worin R17 und R18 gleich oder verschieden sind und die Bedeutung Wasserstoff (H), 1-4C-Alkyl, 1-4C-Alkoxy, Halogen, Hydroxy oder Trifluormethyl haben, und die Salze der Verbindungen.

Bevorzugte Verbindungen der Ausgestaltung i sind solche der Formel Ii, worin
Cy 3-Nitrophenyl, 2-Chlorphenyl, 2,3-Dichlorphenyl, 2-Trifluromethylphenyl, 2-Difluormethoxyphenyl oder Benzoxidazolyl bedeutet,
R1 Methyl bedeutet,
R2 Methyl bedeutet,
R3 einen (-)-Menthylrest darstellt,
die Gruppierung $(CR6R7)_x$-$(CR8R9)_y$-$(CR10R11)_z$ Ethylen oder Propylen bedeutet,
R14 Phenyl bedeutet und
R15 Phenyl bedeutet,
und ihre Salze.

Besonders bevorzugte Verbindungen der Ausgestaltung i sind solche der Formel Ii, worin
Cy 3-Nitrophenyl bedeutet,
R1 Methyl bedeutet,
R2 Methyl bedeutet,
R3 einen (-)-Menthylrest darstellt,
die Gruppierung $(CR6R7)_x$-$(CR8R9)_y$-$(CR10R11)_z$ Ethylen oder Propylen bedeutet,
R14 Phenyl bedeutet und
R15 Phenyl bedeutet,
und ihre Salze.

Als bevorzugte Verbindungen der Ausgestaltung i seien genannt:
1,4-Dihydro-2,6-dimethyl-4-(2-chlorphenyl)-pyridin-3,5-dicarbonsäure-3-(-)-menthyl-5-[2-(4-diphenylmethylenpiperidyl-1)-ethyl]-ester,
1,4-Dihydro-2,6-dimethyl-4-(2,3-dichlorphenyl)-pyridin-3,5-dicarbonsäure-3-(-)-menthyl-5-[2-(4-diphenylmethylenpiperidyl-1)-ethyl]-ester,
1,4-Dihydro-2,6-dimethyl-4-(2-trifluormethylphenyl)-pyridin-3,5-dicarbonsäure-3-(-)-menthyl-5-[2-(4-diphenylmethylenpiperidyl-1)-ethyl]-ester,
1,4-Dihydro-2,6-dimethyl-4-(2-difluormethoxyphenyl)-pyridin-3,5-dicarbonsäure-3-(-)-menthyl-5-[2-(4-diphenylmethylenpiperidyl-1)-ethyl]-ester,
1,4-Dihydro-2,6-dimethyl-4-(2,1,3-benzoxdiazol-4-yl)-pyridin-3,5-dicarbonsäure-3-(-)-menthyl-5-[2-(4-diphenylmethylenpiperidyl-1)-ethyl]-ester,

1,4-Dihydro-2,6-dimethyl-4-(2-chlorphenyl)-pyridin-3,5-dicarbonsäure-3-(-)-menthyl-5-[3-(4-diphenylmethylenpiperidyl-1)-propyl]-ester,
1,4-Dihydro-2,6-dimethyl-4-(2,3-dichlorphenyl)-pyridin-3,5-dicarbonsäure-3-(-)-menthyl-5-[3-(4-diphenylmethylenpiperidyl-1)-propyl]-ester,
1,4-Dihydro-2,6-dimethyl-4-(2-trifluormethylphenyl)-pyridin-3,5-dicarbonsäure-3-(-)-menthyl-5-[3-(4-diphenylmethylenpiperidyl-1)-propyl]-ester,
1,4-Dihydro-2,6-dimethyl-4-(2-difluormethoxyphenyl)-pyridin-3,5-dicarbonsäure-3-(-)-menthyl-5[3-(4-diphenylmethylenpiperidyl-1)-propyl]-ester,
1,4-Dihydro-2,6-dimethyl-4-(2,1,3-benzoxdiazol-4-yl)-pyridin-3,5-dicarbonsäure-3-(-)-menthyl-5-[3-(4-diphenylmethylenpiperidyl-1)-propyl]-ester
und ihre Salze.

Als besonders bevorzugte Verbindungen der Ausgestaltung i seien genannt:
1,4-Dihydro-2,6-dimethyl-4-(3-nitrophenyl)-pyridin-3,5-dicarbonsäure-3-(-)-menthyl-5-[2-(4-diphenylmethylenpiperidyl-1)-ethyl]-ester,
1,4-Dihydro-2,6-dimethyl-4-(3-nitrophenyl)-pyridin-3,5-dicarbonsäure-3-(-)-menthyl-5-[3-(4-diphenylmethylenpiperidyl-1)-propyl]-ester
und ihre Salze.

Die Verbindungen der Formel I besitzen mindestens view Chiralitätszentren, und zwar drei im (-)-Menthylrest und eins in der 4-Position des 1,4-Dihydropyridinringes. Darüberhinaus können in der Gruppierung $(CR6R7)_x$-$(CR8R9)_y$-$(CR10R11)_z$ und im basischen Rest N(R12)R13 - vor allem wenn dieser einen durch 1-4C-Alkyl substituierten Cyclus darstellt - gegebenenfalls noch weitere Chiralitätszentren vorliegen. Die Erfindung umfaßt sowohl alle Enantiomeren als auch alle Diastereomeren sowie Racemate und Gemische von Verbindungen der Formel I. Die Verbindungen der Formel I mit unterschiedlicher Konfiguration in der 4-Position des Dihydropyridinenringes und im restlichen Molekül übereinstimmender Konfiguration sind zueinander diastereomer. Von besonderem Interesse und daher bevorzugter Gegenstand der Erfindung sind die Diastereomeren, die in der 4-Position des Dihydropyridins die gleiche Konfiguration aufweisen, wie das Diastereomer des 1,4-Dihydro-2,6-dimethyl-4-(3-nitrophenyl)-pyridin-3,5-dicarbonsäure-3-(-)-menthylester, das das linear polarisierte Licht der Wellenlänge 589 nm in (+)-Richtung dreht $\{[\alpha]_D^{22} \cong +29{,}3$ (c=1, Methanol)}.

Ein weiterer Gegenstand der Erfindung ist ein Verfahren zur Herstellung der erfindungensgemäßen Verbindungen und ihrer Salze. Das Verfahren ist dadurch gekennzeichnet, daß man

a) Zimtsäurederivate der Formel II

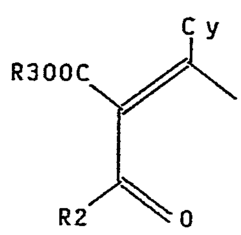

(II),

mit Enaminderivaten der Formel III

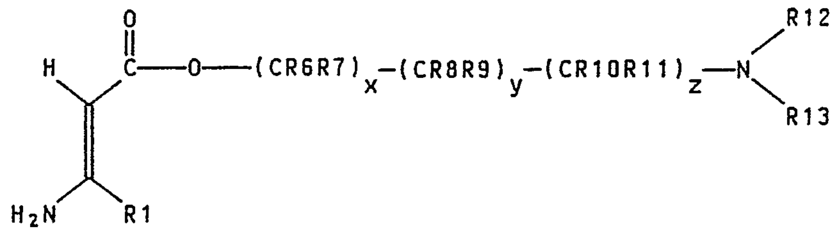

(III),

oder

b) Zimtsäurederivate der Formel II mit Ammoniak und β-Ketocarbonsäurederivaten der Formel IV

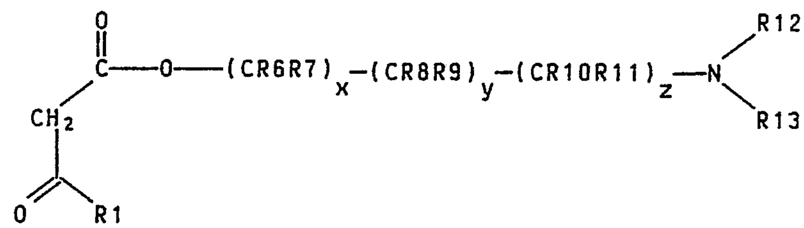

(IV),

oder

c) Enamine der Formel V

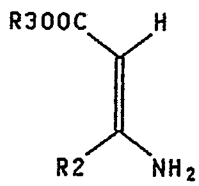

(V).

mit Benzylidencarbonsäurederivaten der Formel VI

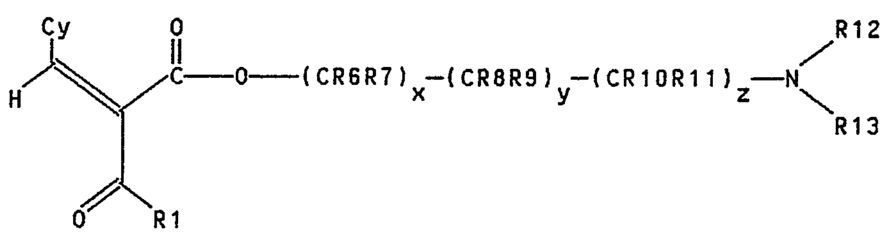

(VI),

oder

d) Ketoverbindungen der Formel VII

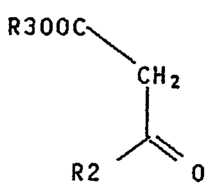

(VII),

mit Ammoniak und Benzylidencarbonsäurederivaten der Formel VI, oder

e) Aldehyde der Formel VIII

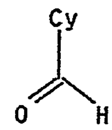

(VIII),

mit Enaminen der Formel V und β-Ketocarbonsäurederivaten der Formel IV, oder

f) Aldehyde der Formel VIII mit Enaminderivaten der Formel III und Ketoverbindungen der Formel VII, oder

g) 1,4-Dihydropyridine der Formel IX

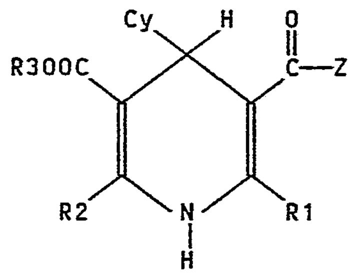

(IX),

mit Aminderivaten der Formel X

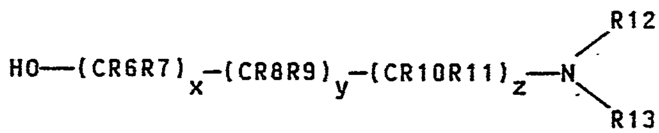

(X),

oder

h) 1,4-Dihydropyridinderivate der Formel XI

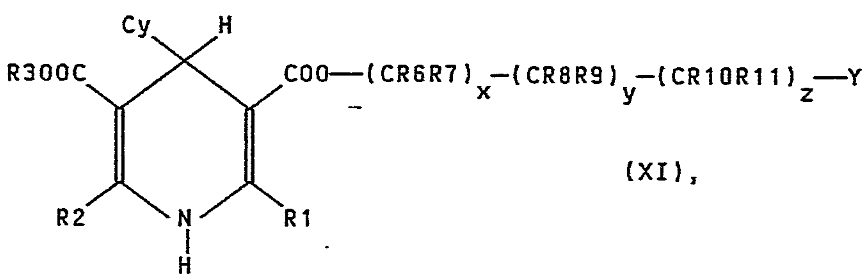

(XI),

mit Aminen der Formel XII

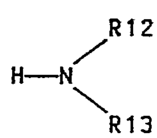

(XII),

als solche(n) oder in Form ihrer Salze umsetzt und gewünschtenfalls anschließend erhaltene Salze in die freien Basen oder erhaltene Basen in die Salze überführt, wobei Cy, R1, R2, R3, R4, R5, R6, R7, R8, R9,

R10, R11, R12, R13, x, y und z die oben angegebenen Bedeutungen haben, Z gemeinsam mit der Carbonylgruppe, woran es gebunden ist, eine Carboxylgruppe oder ein reaktives Carbonsäurederivat (z. B. ein Carbonsäurehalogenid) und Y eine Fluchtgruppe darstellt.

Ausgestaltungen des Verfahrens sind solche, bei denen in den Formeln II bis XII die Substituenten bzw. Symbole Cy, R1, R2, R3, R4, R5, R6, R7, R8, R9, R10, R11, R12, x, y und z die in den Unter-und Nebenansprüchen angegebenen Bedeutungen haben, Z gemeinsam mit der Carbonylgruppe, woran es gebunden ist, eine Carboxylgruppe oder ein reaktives Carbonsäurederivat und Y eine Fluchtgruppe darstellt.

Das Verfahren gemäß den Varianten a bis f wird in geeigneten, vorzugsweise inerten organischen Lösungsmitteln durchgeführt. Beispielsweise seien genannt Alkohole, wie Ethanol, Methanol, Isopropanol oder insbesondere t-Butanol, Kohlenwasserstoffe, wie Toluol oder Xylol, Ether, wie Dioxan, Diethylether, Tetrahydrofuran, Glykolmonoethylether, Glykoldimethylether oder sonstige, beispielsweise polare Lösungsmittel wie Dimethylformamid, Dimethylsulfoxid, Acetonitril oder Hexamethylphosphorsäuretriamid, oder chlorierte Kohlenwasserstoffe wie Methylenchlorid, Chloroform oder Tetrachlorethylen.

Die Reaktionstemperaturen können - je nach Reaktivatät der Edukte - in einem weiten Bereich variieren. Im allgemeinen wird die Umsetzung bei Temperaturen zwischen 20°C und 150°C, vorzugsweise zwischen 20°C und 100°C, insbesondere bei der Siedetemperatur des verwendeten Lösungsmittels durchgeführt.

Das Verfahren kann bei Normaldruck oder bei erhöhtem Druck durchgeführt werden, wobei das Arbeiten bei Normaldruck die Regel ist und erhöhter Druck insbesondere bei Umsetzungen mit Ammoniak zur Anwendung kommen kann.

Bei der Durchführung des erfindungsgemäßen Verfahrens gemäß Varianten a bis f werden die an der Reaktion beteiligten Stoffe in der Regel jeweils in molaren Mengen eingesetzt, wobei jedoch - je nach Reaktionsbedingung - gewünschtenfalls auch ein Überschuß (beispielsweise an Ammoniak bei den Varianten b und d) eingesetzt werden kann.

Bei der Durchführung des Verfahrens gemäß Variante g kommen ähnliche Reaktionsbedingungen wie bei den Varianten a bis f zur Anwendung, jedoch sind - je nach Art des Substituenten Z - gegebenenfalls zusätzliche Maßnahmen erforderlich. Stellt Z beispielsweise eine Hydroxylgruppe dar, so ist die Reaktion bevorzugt in Gegenwart eines wasserabspaltenden oder wasserbindenden Kondensationsmittels (wie z.B. Diocyclohexylcarbodiimid) durchzuführen. Stellt Z ein Halogenatom (z.B. ein Chloratom) dar, so ist die Reaktion gewünschtenfalls in Gegenwart einer Base (z. B. eines tertiären organischen Amins, wie Triethylamin, oder eines anorganischen Carbonates, wie Natriumcarbonat) durchzuführen.

Bei der Durchführung des Verfahrens gemäß Variante h kommen ähnliche Reaktionsbedingungen wie bei den Varianten a bis f zur Anwendung. Die Umsetzung erfolgt in einer Weise, wie sie für die Herstellung von sekundären bzw. tertiären Aminen bekannt ist. Je nach Art der Fluchtgruppe Y, die vorzugsweise ein Halogenatom, insbesondere ein Chlor-oder Bromatom ist, kann die Reaktion gewünschtenfalls in Gegenwart einer Base (z.B. eines anorganischen Carbonates, wie Kaliumcarbonat) oder unter Einsetzung eines Überschusses von Amin XII durchgeführt werden.

Die Isolierung und Reinigung der erfindungsgemäßen Substanzen erfolgt in an sich bekannter Weise z. B. derart, daß man das Lösungsmittel im Vakuum abdestilliert und den erhaltenen Rückstand aus einem geeigneten Lösungsmittel umkristallisiert oder einer der Üblichen Reinigungsmethoden, wie beispielsweise der Säulenchromatographie an geeignetem Trägermaterial, unterwirft. Die Trennung von Diasteromeren erfolgt in für den Fachmann bekannter Weise, beispielsweise durch Umkristallisation oder durch Chromatografie. Diastereomer reine Verbindungen können auch erhalten werden, indem von diastereomer reinen Ausgangsverbindungen ausgegangen wird. Diese wiederum lassen sich durch übliche Trennverfahren erhalten.

Säuradditionssalze erhält man durch Auflösen der freien Base in einem geeigneten Lösungsmittel, z.B. in einem chlorierten Kohlenwasserstoff, wie Methylenchlorid oder Chloroform, oder einem niedermolekularen aliphatischen Alkohol (Ethanol, Isopropanol), das die gewünschte Säure enthält, oder dem die gewünschte Säure anschließend zugegeben wird.

Die Salze werden durch Filtrieren, Umfällen, Ausfällen mit einem Nichtlösungsmittel für das Anlagerungssalz oder durch Verdampfen des Lösungsmittels gewonnen.

Erhaltene Salze können durch Alkalisierung, z.B. mit wäßriger Ammoniaklösung, in die freien Basen umgewandelt werden, welche wiederum in Säureadditionssalze übergeführt werden können. Auf diese Weise lassen sich pharmakologisch nicht verträgliche Säureadditionssalze in pharmakologisch verträgliche Säureadditionssalze umwandeln.

Die Ausgangsverbindungen sind literaturbekannt oder können in Analogie zu literaturbekannten Methoden hergestellt werden. Die Zimtsäurederivate II und die Benzylidencarbonsäurederivate VI können beispielsweise in Analogie zu G. Jones ["The Knoevenagel Condensation" in Org. Reactions, Vol. XV, 204 f (1967)] hergestellt werden. Die Enaminderivate III bzw. die Enamine V sind beispielsweise analog A.C. Cope

[J. Amer Chem. Soc. 67, 1017 (1945)] erhältlich. β-Ketocarbonsäurederivate IV und Ketoverbindungen VII können gemäß D. Borrmann ["Umsetzung von Diketen mit Alkoholen, Phenolen und Mercaptanen" in Houben-Weyl, Methoden der Organischen Chemie, Vol. VII/4, 230ff (1968)] oder Y. Oikawa et al. [J. Org. Chem. 43, 2087 (1978)] hergestellt werden. Die Verbindungen IX sind aus entsprechenden Ausgangsverbindungen analog Verfahrensvariante a bis f zugänglich. Verbindungen X sind durch Umsetzung entsprechender Amine mit omega-Halogenalkanolen erhältlich. Die Dihydropyridinderivate XI erhält man durch Umsetzung von Enaminen der Formel V mit z.B. entsprechend substituierten omega-Halogen-2-acyl-acrylsäureestern, die ihrerseits wiederum aus Aldehyden der Formel VIII und geeigneten beta-Keto-omegahalogencarbonsäureestern zugänglich sind.

Die vorstehenden Herstellungsverfahren sind lediglich zur Verdeutlichung angegeben, und die Herstellung der erfindungsgemäßen Verbindungen der Formel I ist nicht auf diese Verfahren beschränkt. Vielmehr ist auch jede Modifikation dieser Verfahren in gleicher Weise für die Herstellung der erfindungsgemäßen Verbindungen anwendbar.

Die folgenden Herstellungsbeispiele sollen die Erfindung näher erläutern, ohne sie einzuschränken. Schmp. bedeutet Schmelzpunkt, h steht für Stunden, Kp. steht für Siedepunkt, Zers. bedeutet Zersetzung.

## Beispiele

### Endprodukte

1. 1,4-Dihydro-2,6-dimethyl-4-(3-nitrophenyl)-pyridin-3,5-dicarbonsäure-3-(-)-menthyl-5-[3-(4,4-diphenylpiperidyl-1)-propyl]-ester-hydrochlorid

(a) 7,45 g 2-(3-Nitrobenzyliden)-acetessigsäure-(-)-menthylester und 7,56 g 3-Aminocrotonsäure-[3-(4,4-diphenylpiperidyl-1)-propyl]-ester werden in 100 ml 2-Propanol und 0,5 ml Essigsäure unter einer Stickstoffatmosphäre 6 h unter Rückfluß zum Sieden erhitzt. Nach dem Abkühlen wird die Mischung mit 10 ml gesättigter Salzsäurelösung in Ether versetzt. Unter Zusatz von Toluol engt man den Ansatz zur Trockene ein und kristallisiert den festen Rückstand in Essigester um. Man erhält die Titelverbindung als feine Kristallnadeln (Diastereomerengemisch). Schmp.: ab 220°C, langsames Zerfließen; Ausbeute: 13,2 g.

(b) 5,77 g (-)-1,4-Dihydro-2,6-dimethyl-4-(3-nitrophenyl)-pyridin-3,5-dicarbonsäure-3-(-)-menthyl-5-(3-brompropyl-1)-ester {$[\alpha]_D^{22}$ = -16,8°, (c=1, $CH_3OH$)} und 2,74 g 4,4-Diphenylpiperidin-hydorchlorid werden unter kräftigem Rühren in einem Gemisch aus 80 ml Toluol und 6 g Kaliumcarbonat in 40 ml Wasser 48 h unter Rückfluß zum Sieden erhitzt. Nach dem Erkalten trennt man die organische Phase ab, extrahiert die Wasserphase noch zweimal mit je 50 ml Toluol und wäscht die vereinigten Toluolphasen mit Wasser. Nach dem Trocknen der Toluollösung über Natriumsulfat engt man ein und versetzt den Rückstand mit 15 ml Methanol und etherischer Salzsäure. Nach dem vollständigen Einengen zur Trockene wird der Rückstand in 10 ml Methanol/Dichlormethan (1+1) gelöst und bis zur ersten bleibenden feinen Trübung mit Diethylether versetzt. Nach Anreiben und Stehenlassen der Lösung im Kühlschrank erhält man ein reines Diastereomer der Titelverbindung in Form feiner Kristallnadeln vom Schmp. 267-68°C mit $[\alpha]_D^{22}$ = -10,4° (c=1, $CH_3OH$); Ausbeute: 6,2 g.

(c) Das gemäß (b) hergestellte Diastereomer wird ebenfalls erhalten, wenn das nach (a) hergestellte Diastereomerengemisch in Methanol/Dichlormethan (1+1) gelöst und die Lösung mit Dieethylether und einem Impfkristall des reinen Diastereomeren nach (b) versetzt wird. Aus der bei Raumtemperatur stehengelassenen Lösung kristallisert langsam das reine Diastereomer aus.

2. 1,4-Dihydro-2,6-dimethyl-4-(3-nitrophenyl)-pyridin-3,5-dicarbonsäure-3-(-)-menthyl-5-[3-(4-phenylpiperidyl-1)-propyl]-ester-hydrochlorid

(a) 7,4 g 1,4-Dihydro-2,6-dimethyl-4-(3-nitrophenyl)-pyridin-3,5-dicarbonsäure-3-(-)-menthyl-5-(3-brompropyl-1)-ester und 2,07 g 4-Phenylpiperidin werden under kräftigem Rühren in einer Stickstoffatomosphäre in einem Gemisch aus 80 ml Toluol und 5,3 g Kaliumcarbonat in 40 ml Wasser 14 h unter Rückfluß zum Sieden erhitzt. Nach dem Erkalten trennt man die organische Phase ab, extrahiert die Wasserphase noch zweimal mit 50 ml Toluol und wäscht die vereinigten Toluolphasen mit Wasser. Nach dem Trocknen der Toluollösung mit Natriumsulfat engt man ein und versetzt den öligen Rückstand mit 10 ml Methanol und 1,1 ml konzentrierter Salzsäurelösung. Nach dem vollständigen Einengen bis zur

Trockene kristallisiert man den erhaltenen fest aufgeschäumten Rücktstand in Essigsäureethylester/Ether um. Man erhält die Titelverbindung als Diastereomerengemisch vom Schmp. 186-87°C (Nadeln). Ausbeute: 6,5 g.

(b) 2,02 g (-)-1,4-Dihydro-2,6-dimethyl-4(3-nitrophenyl)-pyridin-3,5-di-carbonsäure-3-(-)-menthyl-5-(3-brompropyl-1)-ester {$[\alpha]_D^{22}$ = -16,8°, (c=1, $CH_3OH$}, 0,56 g 4-Phenylpiperidin und 1,45 g Kaliumcarbonat in 30 ml Toluol und 15 ml Wasser werden analog Beispiel 1(b) umgesetzt. Man erhält das eine Diastereomer der Titelverbindung vom Schmp. 158-60°C (Nadeln, Ethylacetat/Diethylether) mit $[\alpha]_D^{22}$ = -33,9° (c=1, $CH_3OH$); Ausbeute: 2,1 g.

(c) 2,28g (+)-1,4-Dihydro-2,6-dimethyl-4-(3-nitrophenyl)-pyridin-3,5-dicarbonsäure-3-(-)-menthyl-5-(3-brompropyl-1)-ester {$[\alpha]_D^{22}$ = +2,0 (c=1, $CH_3OH$), 0,61 g 4-Phenylpiperidin und 1,6 g Kaliumcarbonat in 40 ml Toluol und 20 ml Wasser werden analolg Beispiel I(b) umgesetzt. Man erhält das andere Diastereomer der Titelverbindung als feine Nadeln vom Schmp. 156-58°C (Ethylacetat/Diethylether) mit $[\alpha]_D^{22}$ = +24,4° (c=1, $CH_3OH$); Ausbeute: 620 mg.

3. 1,4-Dihydro-2,6-dimethyl-4-(3-nitrophenyl)-pyridin-3,5-dicarbonsäure-3-(-)-menthyl-5-{3-[4-(2-methoxyphenyl)-1-piperazinyl]-propyl}-ester-fumarat

(a) 3 g 2-(3-Nitrobenzyliden)-acetessigsäure-(-)-menthylester und 2,67 g 3-Aminocrotonsäure-{3-[4-(2-methoxyphenyl)-1-piperazinyl]-propyl}-ester werden in 40 ml tert. Butanol mit 0,25 ml Eisessig versetzt und das Gemisch unter Stickstoff 5 h unter Rückfluß zum Sieden erhitzt. Nach Abkühlen der Lösung wird das Lösungsmittel im Vakuum abdestilliert und der Rückstand in Essigester gelöst. Die Lösung wird mit 2N Ammoniaklösung ausgeschüttelt, über Natriumsulfat getrocknet, im Vakuum eingeengt und der Rückstand über eine Kieselgelsäule mit Essigester als Laufmittel chromatographiert. Die chromatographisch einheitlichen Fraktionen werden im Vakuum zur Trockene eingedampft und das zurückbleibende Öl (5,4 g) in Methanol gelöst, die Lösung mit 0,9 g Fumarsäure versetzt, kurz erwärmt und dann wieder eingeengt. Der Rückstand wird in Acetonitril gelöst und gekühlt. Das auskristallisierte Produkt wird abgesaugt, mit kaltem Acetonitril gewaschen und dann bei 80°C im Vakuum getrocknet. Man erhält 2,8 g der Titelverbindung als Diastereomerengemisch vom Schmp. 132-35°C.

(b) Analog Beispiel 1(b) erhält man das eine Diastereomer der Titelverbindung aus 3,0 g (-)-1,4-Dihydro-2,6-dimethyl-4-(3-nitrophenyl)-pyridin-3,5-dicarbonsäure-3-(-)-menthyl-5-(3-brompropyl-1)-ester {$[\alpha]_D^{22}$ =-16,8° (c=1, $CH_3OH$)}, 1,15 g 2-Methoxiphenylpiperazin-hydrochlorid und 3,0 g Kaliumcarbonat in 55 ml Toluol und 35 ml Wasser als feine Nadeln vom Schmp. 169-71°C (Acetonitril/Diisopropylether) mit $[\alpha]_D^{22}$ = -27,5° (c=1, $CH_3OH$); Ausbeute: 2,4 g.

(c) Analog Beispiel 1(b) erhält man das andere Diastereomer der Titelverbindung aus 3,0 g (+)-1,4-Dihydro-2,6-dimethyl-4-(3-nitrophenyl)-pyridin-3,5-dicarbonsäure-3-(-)-menthyl-5-(3-brompropyl-1)-ester {$[\alpha]_D^{22}$ = +2,0°, (cc=1, $CH_3OH$)}, 1,15 g 2-Methoxiphenylpiperazin-hydrochlorid und 3,0 g Kaliumcarbonat in 55 ml Toluol und 35 ml Wasser als feine Nadeln vom Schmp. 119-20°C (Acetonitril/Diisopropylether) mit $[\alpha]_D^{22}$ = +14,3° (c=1), $CH_3OH$); Ausbeute: 2,1 g.

4. 1,4-Dihydro-2,6-dimethyl-4-(3-nitrophenyl)-pyridin-3,5-dicarbonsäure-3-(-)-menthyl-5-[2-(4-phenyl-1-piperazinyl)-ethyl]ester-fumarat

(a) 3 g 1,4-Dihydro-2,6-dimethyl-4-(3-nitrophenyl)-pyridin-3,5-dicarbonsäure-3-(-)-menthylester werden in 45 ml absolutem Toluol mit 3 ml Oxalylchlorid versetzt und das Gemisch bei Raumtemperatur bis zur Beendigung der Gasentwicklung gerührt. Dann wird im Vakuum zur Trockene eingedampft, der Rückstand in 30 ml Dichlormethan gelöst und 1,35 g 2-(4-Phenyl-1-piperazinyl)-ethanol zugegeben. Es wird über Nacht bei Raumtemperatur stehengelassen, dann mit 2N Ammoniaklösung ausgeschüttelt, die organische Phase über Natriumsulfat getrocknet und im Vakuum eingedampft. Der Rückstand wird über eine Kieselgelsäule mit Chloroform/Ethanol 95/5 chromatographiert. Die chromatographisch einheitlichen Fraktionen werden zur Trockene im Vakuum eingeengt und der Rückstand (3,26 g) in Methanol gelöst. Nach Zugabe von 0,58 g Fumarsäure wird kurz erwärmt, dann wieder eingeengt und der Rückstand in Acetonitril gelöst. Beim Abkühlen kristallisiert die Titelverbindung aus. Man erhält 3,2 g als Diastereomerengemisch vom Schmp. 68-71°C.

(b) 3 g 1,4-Dihydro-2,6-dimethyl-4-(3-nitrophenyl)-pyridin-3,5-dicarbonsäure-3-(-)-menthylester mit $[\alpha]_D^{22}$ = +29,3° (c=1, $CH_3OH$) werden in 50 ml abs. Toluol suspendiert und unter Kühlung 3 ml Oxalylchlorid zugegeben. Die Mischung wird 2 h bei Raumtemperatur bis zur Beendigung der Gasentwicklung gerührt und dann im Vakuum zur Trockene eingeengt. Der Rückstand wird in 50 ml abs. Dichlormethan aufgenommen und dann mit 1,35 g 2-(4-Phenyl-1-piperazinyl)-ethanol versetzt. Die Mischung wird 3 h bei Raumtemperatur gerührt, dann werden 20 ml 10 %ige Ammoniak-Lösung zugegeben und kräftig gerührt. Die organische Phase wird abgetrennt, mit Natriumsulfat getrocknet und dann in Vakuum das Lösungsmittel vollkommen abdestilliert. Der Rückstand wird im Acetonitril gelöst und dann mit einer Lösung von 0,76 g Fumarsäure in Methanol versetzt. Beim Abdestillieren des Methanols am Rotationsverdampfer beginnt die Titelverbindung auszukristallisieren. Es wird gekühlt, abgesaugt und nochmals aus Acetonitril umkristallisiert. Man erhält 3,4 g des einen Diasteromers der Titelverbindung vom Schmp. 181-84°C mit $[\alpha]_D^{22}$ = -48,7° (c=1, $CH_3OH$).

5. 1,4-Dihydro-2,6-dimethyl-4-(3-nitropheny)-pyridin-3,5-dicarbonsäure-3-(-)-menthyl-5-{2-[4-(2-pyridyl)-1-piperazinyl]-ethyl}-ester-fumarat

(a) Analog Beispiel 4(a) erhält man aus 3 g 1,4-Dihydro-2,6-dimethyl-4-(3-nitrophenyl)-pyridin-3,5-dicarbonsäure-3-(-)-menthylester und 1,37 g 2[4-(2-Pyridyl)-1-piperazinyl]-ethanol nach Chromatographie 3,35 g der freien Base, die mit 0,6 g Fumarsäure in Acetonitril in die kristalline Titelverbindung überführt wird. Man erhält 2,9 g als Diastereomerengemisch vom Schmp. 59°C.

(b) Analog Beispiel 4(b) erhält man aus 4 g 1,4-Dihydro-2,6-dimethyl-4-(3-nitrophenyl)-pyridin-3,5-dicarbonsäure-3-(-)-menthylester {$[\alpha]_D^{22}$ = +29,3° (c=1, $CH_3OH$)} und 4 ml Oxalylchlorid in 60 ml abs. Toluol das Säurechlorid, das mit 1,8 g 2-[4-(2-Pyridyl)-1-piperazinyl]-ethanol und 1 g Fumarsäure zum einen Diastereomer der Titelverbindung umgesetzt wird. Man erhält nach zweimaligem Umkristallisieren aus Acetonitril 3,34 g vom Schmp. 178-84°C mit $[\alpha]_D^{22}$ = -44,1° (c=1, $CH_3OH$).

6. 1,4-Dihydro-2,6-dimethyl-4-(3-nitrophenyl)-pyridin-3,5-dicarbonsäure-3-(-)-menthyl-5-[3-benzylmethylamino)-propyl-1]-ester-hydrochlorid

Analog Beispiel 1(b) erhält man ein Diastereomer der Titelverbindung aus 4,04 g (-)-1,4-Dihydro-2,6-dimethyl-4-(3-nitrophenyl)-pyridin-3,5-dicarbonsäure-3-(-)-menthyl-5-(3-brompropyl-1)-ester {$[\alpha]_D^{22}$ = -16,8° (c=1, $CH_3OH$)}, 0,85 g N-Benzylmethylamin und 4,83 g Kaliumcarbonat in 100 ml Toluol und 50 ml Wasser als amorphes Pulver vom Schmp. 108-20°C (langsames Zerfließen; ausgefällt aus Petrolether) mit $[\alpha]_D^{22}$ = -34,2° (c=1, $CH_3OH$); Ausbeute: 3,3 g.

7. 1,4-Dihydro-2,6-dimethyl-4-(3-nitrophenyl)-pyridin-3,5-dicarbonsäure-3-(-)-menthyl-5-{3-[4-(4-fluorbenzoyl)-piperidyl-1]-propyl}-ester-hydrochlorid

Analog Beispiel 1(b) erhält man ein Diastereomer der Titelverbindung aus 3,47 g (-)-1,4-Dihydro-2,6-dimethyl-4-(3-nitrophenyl)-pyridin-3,5-dicarbonsäure-3-(-)-menthyl-5-3-brompropyl-1)-ester {$[\alpha]_D^{22}$ = -16,8° (c=1, $CH_3OH$)}, 1,46 g 4-(4-Fluorbenzoyl)-piperidin-hydrochlorid und 2,1 g Kaliumcarbonat in 50 ml n-Butanol und 50 ml Dioxan als Kristalle vom Schmp. 146-60°C (langsames Zerfließen, Methanol/Diethylether) mit $[\alpha]_D^{22}$ = -30,4°, (c=1, $CH_3OH$); Ausbeute: 2,2 g.

8. 1,4-Dihydro-2,6-dimethyl-4-(3-nitrophenyl)-pyridin-3,5-dicarbonsäure-3-(-)-menthyl-5-[(3-morpholino)-propyl-1]-ester

Analog Beispiel 1(b) erhält man ein Diastereomer der Titelverbindung aus 3,0 g (-)-1,4-Dihydro-2,6-dimethyl-4-(3-nitrophenyl)-pyridin-3,5-dicarbonsäure-3-(-)-menthyl-5-(3-brompropyl-1)-ester {$[\alpha]_D^{22}$ = -16,8° (c=1, $CH_3OH$)}, 0,9 g Morpholin und 2,9 g Kaliumcarbonat in 50 ml Toluol und 30 ml Wasser als kantige Kristalle vom Schmp. 123-24°C (Methanol) mit $[\alpha]_D^{22}$ = -22,3° (c=1, $CH_3OH$); Ausbeute: 1,9 g.

9. 1,4-Dihydro-2,6-dimethyl-4-(3-nitrophenyl)-pyridin-3,5-dicarbonsäure-3-(-)-menthyl-5-{2-[4-(2-methoxyphenyl)1-piperazinyl]-ethyl}-ester

3 g 1,4-Dihydro-2-6-dimethyl-4-(3-nitrophenyl)-pyridin-3,5-dicarbonsäure-3-(2-bromethyl)-5-(-)-menthylester {$[\alpha]_D^{22}$ = -30,9° (c-1, $CH_3OH$)}, 1,5 g 1-(2-Methoxyphenyl)-piperazin-semicarbonat und 1,8 ml Diisopropylethylamin werden in 50 ml Toluol gelöst und die Mischung 24 h unter Rückfluß zum Sieden erhitzt. Nach Abkühlen wird mit 50 ml Wasser kräftig ausgerührt, die organische Phase abtrennt, nochmals mit Wasser gewaschen, über Natriumsulfat getrocknet und dann das Lösungsmittel in Vakuum völlig abgedampft. Der Rückstand wird über eine Kieselgelsäule mit Ethylacetat als Laufmittel chromatografiert. Die reinen Produktfraktionen werden eingedampft und der Rückstand mit Diethylether angerieben. Man erhält 1,8 g des Diastereomers der Titelverbindung vom Schmp. 139-41°C mit $[\alpha]_D^{22}$ = -36,2 (c=1, $CH_3OH$).

10. 1,4-Dihydro-2,6-dimethyl-4-(3-nitrophenyl)-pyridin-3,5-dicarbonsäure-3-(-)-menthyl-5-[3-(4-phenyl-1-piperazinyl)-propyl]-ester-fumarat

3 g 1,4-Dihydro-2,6-dimethyl-4-(3-nitrophenyl)-pyridin-3,5-dicarbonsäure-3-(3-brompropyl)-5-(-)-menthylester mit $[\alpha]_D^{22}$ = -16,4 (c=1, $CH_3OH$) und 0,84 g N-Phenylpiperazin werden in einem Gemisch aus 20 ml Toluol und 2,1 g Kaliumcarbonat in 10 ml Wasser 36 h zum Sieden erhitzt. Nach Abkühlen wird die Toluolphase abgetrennt, mit Wasser gewaschen, über Natrium sulfat getrocknet und dann in Vakuum das Lösungsmittel abdestilliert. Der Rückstand wird über eine Kieselgelsäule mit Essigsäureethylester als Eluens chromatografiert; die reinen Produktfraktionen werden gesammelt, eingeengt und im Vakuum getrocknet. Das zurückbleibende Öl (2,4 g) wird in Acetonitril gelöst und mit einer Lösung von 0,42 g Fumarsäure in Methanol versetzt. Nach Abdampfen des Methanols am Rotationsverdampfer kristallisiert das Produkt aus, es wird abgesaugt, mit wenig kaltem Acetonitril gewaschen und im Hochvakuum getrocknet. Man erhält 2,3 g des Diastereomers der Titelverbindung vom Schmp. 145-48°C $[\alpha]_D^{22}$ = -28,8°C (c=1, $CH_3OH$).

11. 1,4-Dihydro-2,6-dimethyl-4-(3-nitrophenyl)-pyridin-3,5-dicarbonsäure-3-(-)-menthyl-5-{3-[4-(2-ethoxyphenyl)-1-piperazinyl]-propyl}ester-fumarat

Analog Beispiel 10 werden aus 3 g 1,4-Dihydro-2,6-dimethyl-4-(3-nitrophenyl)-pyridin-3,5-dicarbonsäure-3-(3-brompropyl)-5-(-)-menthylester {$[\alpha]_D^{22}$ = -16,4° (c=1, $CH_3OH$)} und 1,1 g 1-(2-Ethoxyphenyl)piperazin 3,8 g des Diastereomers der Titelverbindung vom Schmp. 138,5-42°C mit $[\alpha]_D^{22}$ = -24,6° (c=1, $CH_3OH$) erhalten.

12. 1,4-Dihydro-2,6-dimethyl-4-(3-nitrophenyl)-pyridin-3,5-dicarbonsäure-3-(-)-menthyl-5-{3-[4-(2-pyridyl)-1-piperazinyl]-propyl}-ester-fumarat

Analog Beispiel 10 werden aus 4 g l,4-dihydro-2,6-dimethyl-4-(3-nitrophenyl)-pyridin-3,5-dicarbonsäure-3-(3-brompropyl)-5-5(-)-menthylester {$[\alpha]_D^{22}$ = -16,4° (c=1, $CH_3OH$)} und 1,13 g 1-(2-Pyridyl)-piperazin 2,6 g des Diastereomers der Titelverbindung vom Schmp. 122-25°C (aus $CH_3OH$) mit $[\alpha]_D^{22}$ = -27,5° (c=1, $CH_3OH$) erhalten.

13. 1,4-Dihydro-2,6-dimethyl-4-(3-nitrophenyl)-pyridin-3,5-dicarbonsäure-3-(-)-menthyl-5-[3-(4-benzhydryl-1-piperazinyl)-propyl]-ester-semifumarat

Analog Beispiel 10 werden aus 4 g 1,4-Dihydro-2,6-dimethyl-4-(3-nitrophenyl)-pyridin-3,5-dicarbonsäure-3-(3-brompropyl)-5-(-)-menthylester {$[\alpha]_D^{22}$ = -16,4° (c=1, $CH_3OH$)} und 1,73 g 1-Benzhydrylpiperazin 1,9 g des Diastereomers der Titelverbindung vom Schmp. 125-28°C mit $[\alpha]_D^{22}$ = -22,2° (c=1, $CH_3OH$) erhalten.

14. 1,4-Dihydro-2,6-dimethyl-4-(3-nitrophenyl)-pyridin-3,5-dicarbonsäure-3-(-)-menthyl-5-{3-[4-(3-trifluormethylphenyl)-1-piperazinyl]-propyl}-ester-fumarat

Analog Beispiel 10 werden aus 4 g 1,4-Dihydro-2,6-dimethyl-4-(3-nitrophenyl)-pyridin-3,5-dicarbonsäure-3-(3-brompropyl)-5(-)-menthylester {$[\alpha]^{22}_{D}$ = -16,4° (c=1, $CH_3OH$)} und 1,7 g 1-(3-Trifluormethylphenyl)-piperazin 2,4 g des Diastereomers der Titelverbindung vom Schmp. 135-38°C mit $[\alpha]^{22}_{D}$ = -24,1 (c-1, $CH_3OH$) erhalten.

15. 1,4-Dihydro-2,6-dimethyl-4-(3-nitrophenyl)-pyridin-3,5-dicarbonsäure-3-(-)-menthyl-5-{3-[4-(3-methoxyphenyl)-1-piperazinyl]-propyl}-ester-fumarat

Analog Beispiel 10 werden aus 4 g 1,4-Dihydro-2,6-dimethyl-4-(3-nitrophenyl)-pyridin-3,5-dicarbonsäure-3-(3-brompropyl)-5-(-)-menthylester {$[\alpha]^{22}_{D}$ = -16,4° (c=1, $CH_3OH$)} und 1,83 g 1-(3-Methoxyphenyl)-piperazin-dihydrochlorid 3,5 g des Diastereomers der Titelverbindung vom Schmp. 114-17°C mit $[\alpha]^{22}_{D}$ = -25,0° (c=1, $CH_3OH$) erhalten.

16. 1,4-Dihydro-2,6-dimethyl-4-(3-nitrophenyl)-pyridin-3,5-dicarbonsäure-3-(-)-menthyl-5-{3-[4-(3,4-methylendioxyphenyl)-1-piperazinyl]-propyl}ester-fumarat

Analog Beispiel 10 werden aus 4 g 1,4-Dihydro-2,6-dimethyl-4-(3-nitrophenyl)-pyridin-3,5-dicarbonsäure-3-(3-brompropyl)-5-(-)-menthylester {$[\alpha]^{22}_{D}$ = -16,4° (c=1, $CH_3OH$)} und 1,7 g 1-(3,4-Methylendioxyphenyl)-piperazin-hydrochlorid 1,8 g des Diastereomers der Titelverbindung vom Schmp. 133-36.5°C mit $[\alpha]^{22}_{D}$ = -26,4° (c=1, $CH_3OH$) erhalten.

17. 1,4-Dihydro-2,6-dimethyl-4-(3-nitrophenyl)-pyridin-3,5-dicarbonsäure-3-(-)-menthyl-5-{3-[4-(2-tolyl)-1-piperazinyl]-propyl}-ester-fumarat

Analog Beispiel 10 werden aus 4 g 1,4-Dihydro-2,6-dimethyl-4-(3-nitrophenyl)-pyridin-3,5-dicarbonsäure-3-(3-brompropyl)-5-(-)-menthylester {$[\alpha^{22}_{D}$ = -16,4° (c=1, $CH_3OH$)} und 1,63 g 1-(2-Tolyl)-piperazin-dihydrochlorid 2,7 g des Diastereomers der Titelverbindung vom Schmp. 188-93°C mit $[\alpha]^{22}_{D}$ = -26,6° (c=1, $CH_3OH$) erhalten.

18. 1,4-Dihydro-2,6-dimethyl-4-(3-nitrophenyl)-pyridin-3,5-dicarbonsäure-3-(-)-menthyl-5-{3-[4-(2,4-dimethylphenyl)-1-piperazinyl]-propyl}-ester-fumarat

Analog Beispiel 10 werden aus 4 g 1,4-Dihydro-2,6-dimethyl-4-(3-nitrophenyl)-pyridin-3,5-dicarbonsäure-3-(3-brompropyl)-5-(-)-menthylester {$[\alpha]^{22}_{D}$ = -16,4° (c=1, $CH_3OH$)} undd 1,25 g 1-(2,4-Dimethylphenyl)-piperazin 3,2 g des Diastereomers der Titelverbindung vom Schmp. 148-50°C mit $[\alpha]^{22}_{D}$ = -27,6° (c=1, $CH_3OH$) erhalten.

19. 1,4-Dihydro-2,6-dimethyl-4-(3-nitrophenyl)-pyridin-3,5-dicarbonsäure-3-(-)-menthyl-5-{3-[4-2,6-dimethylphenyl)-1-piperazinyl]-propyl}-ester-fumarat

Analog Beispiel 10 werden aus 4 g 1,4-Dihydro-2,6-dimethyl-4-(3-nitrophenyl)-pyridin-3,5-dicarbonsäure-3-(3-brompropyl)-5-(-)-menthylester {$[\alpha]^{22}_{D}$ = -16,4° (c=1, $CH_3OH$)} und 1,3 g 1-(2,6-Dimethylphenyl)-piperazin 1,9 g des Diastereomers der Titelverbindung vom Schmp. 165-68°C (aus $CH_3OH$) mit $[\alpha]^{22}_{D}$ = -25,0° (c=1, $CH_3OH$) erhalten.

20. 4-(2,3-Dichlorphenyl)-1,4-dihydro-2,6-dimethyl-pyridin-3,5-dicarbonsäure-3-(-)-menthyl-5-[3-(4-phenyl-1-piperazinyl)-propyl]-ester-fumarat

Analog Beispiel 10 werden aus 3 g 4-(2,3-Dichlorphenyl)-1,4-dihydro-2,6-dimethyl-pyridin-3,5-dicarbonsäure-3-(3-brompropyl)-5-(-)-menthylester {$[\alpha]_D^{22}$ = -33,9° (c = 1, $CH_3OH$)} und 0,8 g 1-Phenylpiperazin nach Säulenchromatografie an Kieselgel mit Ethylacetat als Laufmittel 2,5 g der freien Base erhalten, die mit 0,4 g Fumarsäure in Methanol in das Fumarat überführt werden. Nach Eindampfen und Anreiben mit Diisopropylether erhält man 2,4 g des Diastereomers der Titelverbindung vom Schmp. ab 90°C (Zers.) mit $[\alpha]_D^{22}$ = -42,7° (c = 1, $CH_3OH$).

21. 4-(2,3-Dichlorphenyl)-1,4-dihydro-2,6-dimethyl-pyridin-3,5-dicarbonsäure-3-(-)-menthyl-5-{3-[4-(2-methoxyphenyl)-1-piperazinyl]-propyl}ester-fumarat

Analog Beipiel 20 werden aus 3 g 4-(2,3-Dichlorphenyl)-1,4-dihydro-2,6-dimethyl-pyridin-3,5-dicarbonsäure-3-(3-brompropyl)-5-(-)-menthylester {$[\alpha]_D^{22}$ = -33,9° (c = 1, $CH_3OH$)} und 1,1 g 1-(2-Methoxyphenyl)-piperazin-semicarbonat 1,7 g des Diastereomers der Titelverbindung vom Schmp. ab 95°C (Zers.) mit $[\alpha]_D^{22}$ = -40,6° (c = 1, $CH_3OH$) erhalten.

22. 4-(2,3-Dichlorphenyl)-1,4-dihydro-2,6-dimethyl-pyridin-3,5-dicarbonsäure-3-(-)-menthyl-5-{3-[4-(2-ethoxyphenyl)-1-piperazinyl]-propyl}ester-fumarat

Analog Beispiel 20 werden aus 3 g 4-(2,3-Dichlorphenyl)-1,4-dihydro-2,6-dimethyl-pyridin-3,5-dicarbonsäure-3-(3-brompropyl)-5-(-)-menthylester {$[\alpha]_D^{22}$ = -33,9° (c = 1, $CH_3OH$)} und 1,03 g 1-(2-Ethoxyphenyl)-piperazin 2,9 g des Diastereomers der Titelverbindung vom Schmp. ab 110°C (Zers.) mit $[\alpha]_D^{22}$ = -37,9° (c = 1, $CH_3OH$) erhalten.

23. 1,4-Dihydro-2,6-dimethyl-4-(3-nitrophenyl)-pyridin-3,5-dicarbonsäure-3-(-)-menthyl-5-[2-(1-piperidinyl)-ethyl]-ester-fumarat

Analog Beispiel 4(b) erhält man aus 3,7 g 1,4-Dihydro-2,6-dimethyl-4-(3-nitrophenyl)-pyridin-3,5-dicarbonsäure-3-(-)-menthylester {$[\alpha]_D^{22}$ = +29,3° (c = 1, $CH_3OH$)} und 3,7 ml Oxalylchlorid in 60 ml abs. Toluol das Säurechlorid, das mit 1,03 g 1-(2-Hydroxyethyl)-peperidin nach Säulenchromatografie an Kieselgel (Laufmittel: Chloroform/Methanol) 9:1) 3,27 g der freien Base liefert, die mit 0,66 g Fumarsäure in Methanol in das Fumarat überführt wird. Nach Eindampfen wird der zurüchkbleibende Schaum mit Diisopropylether angerieben. Man erhält 3,3 g des Diastereomers der Titelverbindung vom Schmp. 98-104°C mit $[\alpha]_D^{22}$ = -59,0° (c = 1, $CH_3OH$).

24. 1,4-Dihydro-2,6-dimethyl-4-(3-pyridyl)-pyridin-3,5-dicarbonsäure-3-(-)-menthyl-5-[3-(4,4-diphenylpiperidyl-1)-propyl]-ester-fumarat

Analog Beispiel 1(a) erhält man die Titelverbindung vom Schmp. 155-157°C.

Ausgangsverbindungen

A. 1,4-Dihydro-2,6-dimethyl-4-(3-nitrophenyl)-pyridin-3,5-dicarbonsäure-3-(-)-menthyl-5-(3-brompropyl-1)-ester

(a) 21,4 g 2-(3-Nitrobenzyliden)-acetessigsäure-3-brompropyl-1-ester und 14,4 g 3-Aminocrotonsäure-(-)-menthylester werden in einer Stickstoffatmosphäre 6 h unter Rückfluß in 100 ml Tetrahydrofuran und 1 ml Essigsäure zum Sieden erhitzt. Nach dem Erkalten engt man die Lösung unter zusatz von je 50 ml Toluol zweimal ein bis zur Gewichtskonstanz. Die Titelverbindung (Diastereomerengemisch) wird als zähflüssiges Öl erhalten und ohne weitere Reinigung direkt weiter umgesetzt.

(b) 36 g des nach (a) erhaltenen vollständig zur Trockene eingeengten Diastereomerengemischs werden in 200 ml siedenem Methanol gelöst. Die Lösung wird unter Rühren langsam abgekühlt. Das erhaltenen Kristallisat (14 g) wird in Methanol/Chloroform (2+1) umkristallisiert. Man erhält das eine Diastereomer der Titelverbindung mit $[\alpha]_D^{22}$ = -16,8° (c=1, $CH_3OH$) als würfelige Kristalle vom Schmp. 173-74°C; Ausbeute: 11,4g.

(c) Die bei (b) zurückbleibende Mutterlauge wird durch Chromatographie über eine Kieselgelsäule (Dichlormethan/Petrolether 9+1) gereinigt. Der Rückstand der Produktfraktionen wird in einem Gemisch aus Cyclohexan/Methanol 9+1 gelöst und mit dem gleichen Volumen Petrolether versetzt. Nach Stehen im Kühlschrank kristallisiert das andere Diastereomer der Titelverbindung mit $[\alpha]_D^{22}$ = +2,0° (c=1, $CH_3OH$) langsam in Form feiner Nadeln vom Schmp. 75-78°C aus; Ausbeute: 4,5 g.

B. 2-(3-Nitrobenzyliden)-acetessigsäure-(-)-menthylester

15,6 g Acetessigsäure-(-)-menthylester und 9,8 g 3-Nitrobenzaldehyd werden in 160 ml Benzol mit 8 ml Eisessig und 0,7 ml Piperidin versetzt und dann das Gemisch am Wasserabscheider unter Rückfluß solange zum Sieden erhitzt, bis sich ca. 1,2 ml Wasser abgeschieden haben. Nach Abkühlen wird die Lösung mit Wasser und verdünnter Natriumhydrogencarbonat-Lösung gewaschen, über Natriumsulfat getrocknet und im Vakuum zur Trockene eingeengt. Das zurückbleibende zähe, gelborange Öl wird ohne weitere Reinigung weiter umgesetzt.

C. 3-Aminocrotonsäure-{3-[4-(2-methoxyphenyl)-1-piperazinyl]-propyl}-ester

25 g 3-[4-(2-Methoxyphenyl)-1-piperazinyl]-propanol werden in 100 ml Methylenchlorid gelöst und mit einer Spatelspitze 4-Dimethylaminopyridin versetzt. Dann werden unter kräftigem Rühren 20 ml einer 50%igen Diketenlösung in Aceton zugetropft. Das Demisch wird über Nacht bei Raumtemperatur stehengelassen. Nach Einengen im Vakuum wird der verbliebene Rückstand unter Kühlung mit ca. 200 ml einer gesättigten Lösung von Ammoniak in Isopropanol versetzt und das Gemisch drei Tage im gut verschlossenen Gefäß bei Raumtemperatur stehengelassen. Nach Animpfen mit der Titelverbindung beginnt das Produkt zu kristallisieren. Es wird gut gekühlt, abgesaugt und über Calciumchlorid getrocknet. Man erhält 28,5 g vom Schmp. 114-115°C.

D. 1,4-Dihydro-2,6-dimethyl-4-(3-nitrophenyl)-pyridin-3,5-dicarbonsäure-3-(-)-menthylester

(a) 23,9 g 3-Aminocrotonsäure-(-)-menthylester und 28,8 g 2-(3-Nitrobenzyliden)-acetessigsäure-(2-cyanethyl)-ester werden in 300 ml Isopropanol 8,5 h unter Stickstoff zum Sieden erhitzt. Nach Abkühlen werden 52 ml 2N Natriumhydroxyd-Lösung zugetropft und das Gemisch 2 h bei Raumtemperatur gerührt. Dann werden 55 ml 2N Salzsäure-Lösung zugetropft und danach das Lösungsmittel im Vakuum abdestilliert. Es wird mit Wasser verdünnt und mit Dichlormethan extrahiert. Nach Trockenen der organischen Phase mit Natriumsulfat wird im Vakuum eingedampft und das zurückbleibende Öl direkt weiterverarbeitet oder aus Methanol in der Kälte kristallisiert. Dann erhält man 24,5 g der Titelverbindung als Diastereomerengemisch vom Schmp. 199°C.

(b) 77,5 g 1,4-Dihydro-2,6-dimethyl-4-(3-nitrophenyl)-pyridin-3,5-dicarbonsäure-3-(2-bromethyl)-5-(-)-menthylester {$[\alpha]_D^{22}$ = -30,9° )c=1, $CH_3OH$)} werden in 430 ml Dimethylformamid gelöst und mit 14,8 g Natriumcy anid und 0,5 g Tetrabutylammoniumcyanid versetzt. Das Gemisch wird 24 h bei Raumtemperatur gerührt, dann werden 70 ml 2N Natronlauge zugetropft. Es wird noch eine Stunde gerührt, dann wird das Gemisch auf 1,3 l Eiswasser gegossen und unter Rühren durch Zutropfen von 2N Salzsäure ein pH von 2-3 eingestellt. Es wird 2 h im Eisbad gerührt, abgesaugt und mit Wasser gewaschen. Das isolierte feuchte Produkt wird in Dichlormethan gelöst, die Lösung gut mit Wasser gewaschen, über Natriumsulfat getrocknet und im Vakuum zur Trockene eingeengt. Der Rückstand wird in Diisopropylether gelöst, die Lösung mit einigen Impfkristallen des Diastereomers der Titelverbindung angeimpft und dann 24 h im Kühlschrank stehengelassen. Es wird abgesaugt, mit wenig kalten Diisopropylether gewaschen und getrocknet. Man erhält 38,5 g des Diastereomers der Titelverbindung mit $[\alpha]_D^{22}$ = +29,3° vom Schmp. 180,5-84°C.

E. 3-Aminocrotonsäure-[3-(4,4-diphenylpiperidyl-1)-propyl]-ester

260,5 g Acetessigsäure-[3-(4,4-diphenylpiperidyl-1)-propyl]-ester in 1,6 l 2-Propanol werden über Nacht mit 260 ml konzentrierter Ammoniaklösung gerührt. Der ausgefallene feine, leicht ockerfarbige Niederschlag wird abgesaugt und mit kaltem 2-Propanol, Diethylether und schließlich Petrolether gewaschen. Schmp.: 144-150°C; Ausbeute: 225 g. Nach Zusatz von weiteren 100 ml konzentrierter Ammoniaklösung zum Filtrat erhält man nach mehrtägigem Stehen im Kühlschrank weitere 12 g Produkt mit identischem Schmelzpunkt.

Alternativ wird die Ausgangsverbindung erhalten, wenn zur Lösung von Acetessigsäure-[3-(4,4-diphenylpiperidyl-1)-propyl]-ester in 2-Propanol unter Rühren solange gasförmiges Ammoniak eingeleitet wird, bis kein weiterer Niederschlag mehr ausfällt. Ausbeute ca. 90 % der Theorie.

F. 3-Amino-crotonsäure-[3-(4-phenylpiperidyl-1)-propyl]-ester

In eine Lösung von 10 g Acetessigsäure-[3-(4-phenylpiperidyl-1)-propyl]-ester in 100 ml 2-Propanol wird unter Eiskühlung bis zur Sättigung Ammoniak eingeleitet. Die klare Lösung bleibt über Nacht im verschlossenen Kolben bei Raumtemperatur stehen und wird dann bis zur Gewichtskonstanz am Rotationsverdampfer eingeengt. Die Titelverbindung bleibt als zähes gelbliches Öl im Rückstand und wird ohne weitere Reinigung weiter umgesetzt. Ausbeute: 10,1 g.

G. 1,4-Dihydro-2,6-dimethyl-4-(3-nitrophenyl)-pyridin-3,5-dicarbonsäure-3-(2-bromethyl)-5-(-)-menthylester

146 g 2-(3-Nitrobenzyliden)-acetessigsäure-2-bromethylester und 102 g 3-Amino-crotonsäure-(-)-menthylester werden in 1,4 l Tetrahydrofuran mit 7,2 ml Essigsäure versetzt und das Gemisch 24 h unter Rückfluß zum Sieden erhitzt. Nach Abkühlen wird filtriert, im Vakuum zur Trockene eingedampft und der Rückstand in 1,3 l Methanol in der Siedehitze gelöst. Nach langsamem Abkühlen und Stehen über Nacht bei Raumtemperatur wird abgesaugt, mit kaltem Methanol gewaschen und getrockent. Man erhält 79,2 g des reinen Diastereomers mit $[\alpha]_D^{22}$ = -30,9° (c = 1, $CH_3OH$) vom Schmp. 161-164°C. Für die Isolierung des anderen Diastereomers wird die Mutterlauge etwa zur Hälfte eingeengt und bei Raumtemperatur stehengelassen. Nach 2 Tagen wird abgesaugt und die Kristalle nochmals aus siedendem Methanol umkristallisiert. Man erhült 51,3 g NMR-spektroskopisch reines (+)-Diastereomer mit $[\alpha]_D^{22}$ = +21,1° (c = 1, $CH_3OH$) vom Schmp. 88-90°C.

H. 4-(2,3-Dichlorphenyl)-1,4-dihydro-2,6-dimethyl-pyridin-3,5-dicarbonsäure-3-(3-brompropyl)-5-(-)-menthylester

33,5 Acetessigsäure-3-brompropyl-ester und 26,3 g 2,3-Dichlorbenzaldehyd werden in 150 ml Benzol gelöst und mit 7,4 ml Essigsäure und 0,7 ml Piperidin versetzt. Das Gemisch wird am Wasserabscheider zum Sieden erhitzt bis sich 3 ml Wasser abgeschieden haben. Nach Abkühlen wird das Lösungsmittel im Vakuum abdestilliert, der Rückstand in 400 ml Tetrahydrofuran gelöst und mit 35,9 g 3-Amino-crotonsäure-(-)-menthylester versetzt. Das Gemisch wird 48 h zum Sieden erhitzt, nach dem Abkühlen im Vakuum zur Trockene eingedampft und der Rückstand in 600 ml Methanol gelöst. Nach Animpfen mit einigen Kristallen des Diastereomers der Titelverbindung läßt man 2 Tage bei Raumtemperatur stehen, saugt dann die abgeschiedenen Kristalle ab und erhält 17 g des Diastereomers der Titelverbindung mit $[\alpha]_D^{22}$ = -33,9° (c = 1, $CH_3OH$) vom Schmp. 197-201°C. Weitere 7 g des Diastereomers der Titelverbindung werden erhalten, wenn man die Mutterlauge zur Hälfte einengt, animpft und 2 Tage bei 0°C stehenläßt.

I. 3-Aminocrotonsäure-(-)-menthylester

217 g Acetessigsäure-(-)-menthylester werden in 2 l Methanol gelöst. Die Lösung wird unter Eiskühlen mit gasförmigem Ammoniak gesättigt. Man läßt 5-7 Tage gut verschlossen bei Raumtemperatur stehen, dann wird die Lösung im Vakuum auf ca. 300 ml eingeengt, wobei die Titelverbindung zu kristallisieren beginnt. Es wird 12 h bei 0-4°C stehengelassen, abgesaugt, mit kaltem Methanol gewaschen und

getrocknet. Man erhält 139 g der Titelverbindung mit $[\alpha]_D^{22}$ =-87,8° (c=1, $CH_3OH$) vom Schmp. 93-94,5°C.
Weitere 42 g der Titelverbindung können nach Einengen der Mutterlauge und Kristallisation aus wenig Methanol erhalten werden.

Gewerbliche Anwendbarkeit

Die erfindungsgemäßen Verbindungen der Formel I und ihre Salze besitzen wertvolle Eigenschaften, die sie gewerblich verwertbar machen. Sie stellen insbesondere wirksame Vasodilatoren mit coronartherapeutischen Eigenschaften dar. Die pharmakologische Wirksamkeit der erfindungsgemäßen Verbindungen, die gepaart ist mit einer geringen Toxizität, zeigt sich insbesondere in einer langsam eintretenden, starke und langanhaltenden Blutdrucksenkung. Darüberhinaus besitzen die erfindungsgemäßen Verbindungen hemmende Wirkung auf den Calciumeinstrom sowie fördernde Wirkung auf den Kaliumausstrom von Zellen, glattmuskulär relaxierende und peripher, coronar, cerebral und renal gefäßerweiternde sowie salidiuretische, antithrombotische, antiarteriosklerotische und günstige hämorheologische Eigenschaften.

In ihrer ausgezeichneten Wirksamkeit, die gepaart ist mit einer geringen Toxizität und dem Fehlen wesentlicher Nebenwirkungen, unterscheiden sich die erfindungsgemäßen Verbindungen in überraschender und vorteilhafter Weise von den Verbindungen des Standes der Technik.

Als besonders überaschend ist die Tatsache zu werten, daß die erfindungsgemäßen (-)-Menthylester eine wesentlich stärkere und insbesondere länger anhaltende Wirkung als die bekannten Methyl-oder Ethylester, aber auch als die enantiomeren (+)-Menthylester zeigen. Vor allem letztere Tatsache war für den Fachmann nicht vorhersehbar, da aufgrund der in der Europäischen Patentanmeldungg 26 317 offenbarten Befunde zu erwarten gewesen wäre, daß die pharmakologische Wirkung nicht durch die unterschiedliche Konfiguration im chiralen Esterrest, sondern einzig durch die Konfiguration des Kohlenstoffs in 4-Position des Dihydropyridinringes beeinflußt wird.

Als vorteilhafte Eigenschaften der Verbindungen I sind beispielsweise zu nennen: das Ausmaß der Blutdrucksenkung, das lange Anhalten der Blutdrucksenkung, die gute Steuerbarkeit der Blutdrucksenkung, die überraschend geringe und bei wiederholter Gabe verschwindende Herzfrequenzsteigerung, die ausgezeichnete Bioverfügbarkeit, die große theapeutische Breite, das Fehlen zentraler Nebenwirkungen, das Fehlen kinetischer Interaktionen mit anderen Substanzen, das Ausbleiben einer Toleranzentwicklung, die ausgewogenen physikalischen Eigenschaften und die große Stabilität.

Die ausgezeichnete Wirksamkeit der erfindungsgemäßen Verbindungen der Formel I und ihrer Salze gestattet ihren Einsatz in der Humanmedizin, wobei als Indikation insbesondere primäre (essentielle) und sekundäre, arterielle und pulmonale Hypertonien aller Schweregrade, koronare Herzkrankheiten (Koronarinsuffizienz, Angia Pectoris, Myocardinfarkt etc.), periphere und cerebrale Zirkulationsstörungen (Gehirnschlag, temporäre cerebrale Durchblutungsstörungen, Migräne, Schwindel, renale Arterienverengung etc.), hypertrophe Kardiomyopathie, Herzinsuffizienz, Krankheiten, die auf einer erhöhten Wasser-und Natriumretention beruhen und Krankheiten, die auf einem erhöhten Calciumeinstrom beruhen, wie z.B. Spasmen glattmuskulärer Organe (Atemwege, Gastrointestinaltrakt, Urogenitaltrakt etc.) sowie Arrhythmie, Arteriosklerose und Zellschädigungen verschiedener Genese (z.B. Hypoxie) in Betracht kommen.

Ein weiterer Gegenstand der Erfindung ist daher ein Verfahren zur Behandlung von Säugetieren, insbesondere Menschen, die an einer der obengenannten Krankheiten erkrankt sind. Das Verfahren ist dadurch gekennzeichnet, daß man dem erkrankten Individuum eine therapeutisch wirksame und pharmakologisch verträgliche Menge einer oder mehrerer Verbindungen der Formel I verabreicht.

Gegestand der Erfindung sind außerdem die Verbindungen der Formel I zur Anwendung bei der Behandlung der genannten Krankheiten.

Ebenso umfaßt die Erfindung die Verwendung von Verbindungen der Formel I bei der Herstellung von Arzneimitteln, die zur Bekämpfung der genannten Krankheiten eingesetzt werden.

Ein weiterer Gegenstand der Erfindung sind Arzneimittel, die eine oder mehrere Verbindungen der allgemeinen Formel I enthalten.

Die Arzneimittel werden nach an sich bekannten, dem Fachmann geläufigen Verfahren hergestellt. Als Arzneimittel werden die erfindungsgemäßen pharmakologisch wirksamen Verbindungen (=Wirkstoffe) entweder als solche, oder vorzugsweise in Kombination mit geeigneten pharmazeutischen Hilfsstoffen in Form von Tabletten, Dragees, Kapseln, Suppositorien, Pflastern (z.B. als TTS), Emulsionen, Suspensionen, Aerosolen, Sprays, Salben, Cremes, Gelen oder Lösungen eingesetzt, wobei der Wirkstoffgehalt vorteilhafterweise zwischen 0,1 und 95% beträgt.

Welche Hilfsstoffe für die gewünschten Arzneimittelformulierungen geeignet sind, ist dem Fachmann aufgrund seines Fachwissens geläufig. Neben Lösemitteln, Gelbildnern, Suppositoriengrundlagen, Tabletten, Hilfsstoffen und anderen Wirkstoffträgern können beispielsweise Antioxidantien, Dispergiermittel, Emulgatoren, Entschäumer, Geschmackskorrigentien, Konservierungsmittel, Lösungsvermittler, Farbstoffe oder insbesondere Permeationspromotoren und Komplexbildner (z.B. Cyclodextrine) verwendet werden.

Die Wirkstoffe können oral, rektal, per inhalationem oder parenteral (insbesondere perlingual, intravenös oder percutan) appliziert werden.

Im allgemeinen hat es sich in der Humanmedizin als vorteilhaft erwiesen, den oder die Wirkstoffe bei oraler Gabe in einer Tagesdosis von etwa 0,01 bis etwa 10, vorzugsweise 0,05 bis 5 mg/kg Körpergewicht, gewünschtenfalls in Form mehrerer, vorzugsweise 1 bis 4 Einzelgaben zur Erzielung des gewünschten Ergebnisses zu verabreichen. Bei einer parenteralen Behandlung können ähnliche bzw. (insbesondere bei der intravenösn Verabreichung der Wirkstoffe) in der Regel niedrigere Dosierungen zur Anwendung kommen. Bei einschleichender Dosierung wird zu Beginn der Behandlung eine geringere Dosis verabreicht, dann langsam auf eine höhere Dosis übergegangen. Nach Erreichen des gewünschten Therapieerfolges wird wieder auf eine niedrigere Dosis zurückgegangen.

Die Festlegung der jeweils erforderlichen optimalen Dosierung und Applikationsart der Wirkstoffe kann durch jeden Fachmann aufgrund seines Fachwissens leicht erfolgen.

Sollen die erfindungsgemäßen Verbindungen und/oder ihre Salze zur Behandlung der genannten Krankheiten eingesetzt werden, so können die pharmazeutischen Zubereitungen auch einen oder mehrere andere pharmakologisch aktive Bestandteile anderer Arzneimittelgruppen, wie andere Vasodilatoren, Antihypertensiva, alpha-1-Rezeptorenblocker, alpha-2-Rezeptorstimulatoren, beta-1-Rezeptorenblocker, beta-2-Rezeptorstimulatoren, ACE-Hemmstoffe, Nitroverbindungen, Cardiotonika, Diuretika, Saluretika, Alkaloide, Analgetika, Lipidsenker, Antikoagulantien, Anticholiergika, Methylxanthine, Antiarrhythmika, Antihistaminika, Dopaminstimulatoren, Serotonin-Rezeptorenblocker etc., wie Nifedipin, Dihydralazin, Prazosin, Clonidin, Atenolol, Labetalol, Fenoterol, Captopril, Isosorbiddinitrat, Digoxin, Milrinon, Mefrusid, Clopamid, Spironolacton, Chlorthalidon, Furosemid, Polythiazid, Hydrochlorothiazid, Reserpin, Dihydroergocristin, Rescinnamin, Rauwolfia-Gesamtalkaloide, Acetylsalicylsäure, Bezafibrat, Warfarin, Atropin, Theophyllin, Lidocain, Astemizol, Bromocryptin, Ketanserin etc. enthalten.

Pharmakologie

Die antihypertensive Wirksamkeit der erfindungsgemäßen Verbindungen kann am Modell der spontan hypertonen Ratte nachgewiesen werden.

Zur Bestimmung der antihypertensiven Wirkung werden die unten aufgeführten Verbindungen in den angegebenen Dosen an vier aufeinander folgenden Tagen an je 6 männlichen Ratten (Stamm SHR/N/Ibm/Bm, 250-350 g) mit genetisch bedingtem Hochdruck (systolischer Blutdruck > 180 mmHg) täglich einmal mittels Schlundsonde verabfolgt. Die Messung des Blutdrucks erfolgt jeweils 6 und gegebenenfalls 2 oder 24 Studnen nach Substanzgabe.

Die Blutdruckmessung wird in einer Wärmekammer bei 36°C vorgenommen, um eine bessere Durchblutung der Schwanzarterie zu erreichen. Hierzu werden die Tiere in perforierte Lochblechkäfige verbracht und 20 - 40 Min. nach Beginn der Aufwärmung gemessen. Zur Messung des systolischen arteriellen Drucks wird eine ringförmige Manschette mit aufblasbarer Gummimembran zur Unterbindung der Durchblutung und ein ringförmiger Piezokristallaufnehmer zur Erfassung der Pulswellen auf den Schwanz aufgeschoben. Nach erfolgter Unterbindung des Blutstroms in der Schwanzarterie wird der Manschettendruck kontinuierlich reduziert. Die Wiederkehr der Pulswellen bei Druckablassen wird automatisch als systolischer Blutdruck erkannt und ausgedruckt (Bühler, R. er al." Microprocessor-based automation of blood pressure measurement in the conscious rat. Proceedings of the 4th international symposium on rats with spontaneous hypertension and related studies, Rascher, R. et al. (Eds.), Schattauer Verlag, Stuttgart, New York, 1982, S. 410-413). Pulssignale und Druckverlauf werden zur Auswertung graphisch aufgezeichnet.

Zur Gewöhnung an den Meßvorgang werden die Tiere vor Substanzprüfung 14 Tage trainiert. In der zweiten Trainingswoche werden Blutdruck-Vorwerte erhoben. Tiergruppen, die Substanz erhalten, werden gegen eine Kontrollgruppe geprüft.

In der anschließenden Tabelle werden die untersuchten Verbindungen durch laufende Nummern gekennzeichnet, die mit den jewiligen Beispielnummern übereinstimmen.

Tabelle I gibt für die Vertreter der erfindungsgemäßen Verbindungen die prozentuale Senkung des Blutdrucks (BP) nach oraler Verabreichung bei der Ratte wieder.

Tabelle I

%-Änderungen (BP) an genetisch hypertonen Ratten nach täglich einmaliger p.o.-Applikation an view aufeinanderfolgenden Tagen (N = 6/Dosis).

| Beisp. Nr. | Dosis µmol/kg | BP (% Änderung vs. Kontrolle), Mittelwert für Meßzeitpunkte: Stunden nach Gabe (Tage) | | |
|---|---|---|---|---|
| | | 2h (1.+4.Tag) | 6h (1.-4.Tag) | 24h (1.+3.Tag) |
| 1a | 25 | -24 | -20 | -15 |
| 2a | 25 | -25 | -24 | -16 |
| 2b | 25 | -36 | -33 | -16 |
| 3a | 25 | -46 | -42 | -15 |
| 3b | 25 | -23 | -36 | -17 |
| 4a | 5 | -34 | -23 | -14 |
| 4b | 5 | -18 | -16 | +4 |
| 5a | 10 | -29 | -11 | -3 |
| 5b | 10 | -20 | -12 | -10 |
| 6 | 25 | -27 | -33 | -10 |
| 7 | 10 | -12 | -11 | +5 |
| 9 | 10 | -22 | -32 | -4 |
| 10 | 25 | -34 | -34 | -6 |
| 11 | 10 | -21 | -26 | -10 |
| 12 | 10 | -17 | -20 | -1 |
| 13 | 25 | -30 | -40 | -35 |
| 14 | 25 | -42 | -43 | -21 |
| 15 | 10 | -14 | -7 | -4 |
| 16 | 10 | -21 | -21 | -7 |
| 17 | 10 | -17 | -14 | -3 |
| 18 | 10 | -15 | -18 | -3 |
| 21 | 25 | -50 | -51 | -46 |
| 22 | 25 | -46 | -55 | -43 |
| 23 | 25 | -17 | -26 | +4 |

## Ansprüche

·1. Verbindungen der Formel I

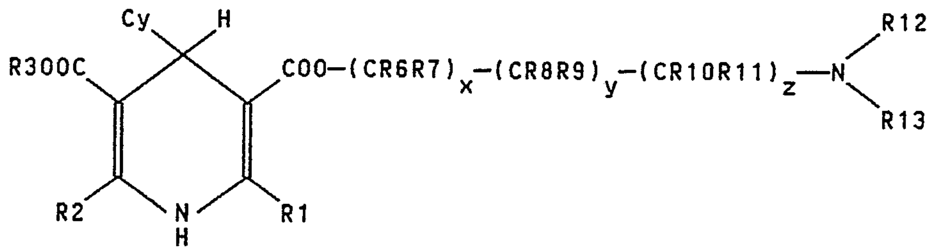

(I)

worin Cy einen Cyclus der Formel

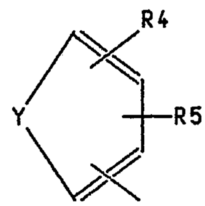

darstellt, in dem Y Sauerstoff (O), Schwefel (S), Vinylen (-CH = CH-), Azomethin (-CH = N-) oder eine Gruppe der Formel

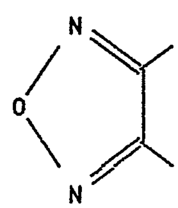

oder

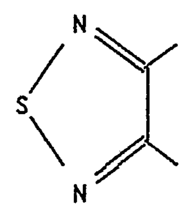

bedeutet,
R1 und R2 gleich oder verschieden sind und Wasserstoff, 1-6C-Alkyl oder 3-7C-Alkoxyalkyl bedeuten,
R3 einen (-)-Menthylrest darstellt,
R4 und R5 gleich oder verschieden sind und Wasserstoff, Hydroxy, Halogen, Nitro, Cyano, Trifluormethyl, 1-4C-Alkyl, 1-4C-Alkoxy, ganz oder teilweise durch Fluor substituiertes 1-4C-Alkoxy, 1-4C-Alkoxy-carbonyl, 2-5C-Acyl, Amino oder Mono-oder Di-1-4C-alkylamino bedeuten,
R6 LWasserstoff (H) oder 1-4C-Alkyl bedeutet,
R7 Wasserstoff (H), 1-4C-Alkyl oder gemeinsam mit R12 1-4C-Alkylen bedeutet, das gewünschtenfalls durch eine oder mehrere 1-4C-Alkylgruppen substituiert ist,
R8 Wasserstoff (H) oder 1-4C-Alkyl bedeutet,
R9 Wasserstoff (H), 1-4C-Alkyl, 1-4C-Alkoxy, Aryl, Heteroaryl oder gemeinsam mit R12 1-4C-Alkylen bedeutet, das gewünschtenfalls durch eine oder mehrere 1-4C-Alkylgruppen substituiert ist,
R10 Wasserstoff (H) oder 1-4C-Alkyl bedeutet,
R11 Wasserstoff (H) oder 1-4C-Alkyl bedeutet,
R12 Wasserstoff (H), 1-4C-Alkyl oder gemeinsam mit R7 oder R9 1-4C-Alkylen bedeutet, das gewünschtenfalls durch eine oder mehrere 1-4C-Alkylgruppen substituiert ist, und
R13 1-4C-Alkyl, Aryl, Aryl-1-4C-alkyl, Aryl-2-4C-alkenyl, Aryl-2-rC-alkinyl, 2-5C-Acyl, Arylcarbonyl oder Heteroarylcarbonyl bedeutet, oder
R12 und R13 gemeinsam und unter Einschluß des Stickstoffatoms, an das beide gebunden sind, einen Rest der Formel

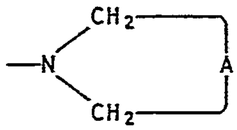

darstellen, worin

A $-CH_2-(CH_2)_m-$,
$-CH_2-C(R14)R15-CH_2-$,
$-CH=CR15-CH_2-$,
$CH_2-O-CH_2-$,
$-CH_2-S-CH_2-$,
$-CH_2-NR16-CH_2-$oder
$-(CH_2)_2-NR16-CH_2-$bedeutet,

R14 Wasserstoff (H) oder Aryl und

R15 Wasserstoff (H), 1-4C-Alkyl, 1-4C-Alkoxy, Hydroxy, Aryl, Arylcarbonyl oder Heteroaryl bedeutet, oder

R14 und R15 gemeinsam Diarylmethylen bedeuten,

R16 1-4C-Alkyl, Aryl, Aryl-1-4C-alkyl, Aryl-2-4C-alkenyl, Aryl-2-4C-alkinyl, Diaryl-1-4C-alkyl, Heteroaryl, Heteroaryl-1-4C-alkyl, Heteroaryl-aryl-1-4C-alkyl, Di-heteroaryl-1-4C-alkyl, Arylcarbonyl, Heteroarylcarbonyl, Arylsulfonyl, Aryl-1-4C-alkylcarbonyl oder Aryl-2-4C-alkenylcarbonyl bedeutet,

x, y und z gleich oder verschieden sind und die Zahlen 0, 1, 2, 3 oder 4 bedeuten, wobei die Summe von x, y und z eine Zahl von 1 bis 5 ist, und

m die Zahl 1, 2 oder 3 bedeutet, wobei

Aryl für einen Ring der Formel

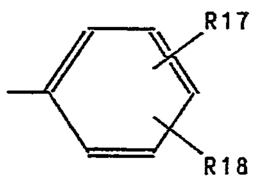

steht, worin R17 und R18 gleich oder verschieden sind und die Bedeutung Wasserstoff (H), 1-4C-Alkyl, 1-4C-Alkoxy, Halogen, Hydroxy, Trifluormethyl oder gemeinsam Methylendioxy haben, und

Heteroaryl für einen 5-oder 6-gliedrigen Heterocyclus mit einem oder zwei gleichen oder verschiedenen Heteroatomen aus der Gruppe Sauerstoff (O), Schwefel (S) oder Stickstoff (N) steht, der ungesättigt oder teilweise oder ganz gesättigt ist und der einen oder zwei Substituenten aus der Gruppe 1-4C-Alkyl, 1-4C-Alkoxy, Halogen, Trifluormethyl oder Cyan tragen kann,

und die Salze dieser Verbindungen.

2. Verbindungen der Formel I nach Anspruch 1, worin Cy 3-Nitrophenyl, 2,3-Dichlorphenyl oder 3-Pyridyl bedeutet,

R1 und R2 gleich oder verschieden sind und 1-4C-Alkyl bedeuten,

R3 einen (-)-Menthylrest darstellt,

die Gruppierung $(CR6R7)_x-(CR8R9)_y-(CR10R11)_z$ Ethylen oder Propylen bedeutet,

R12 1-4C-Alkyl bedeutet und

R13 Benzyl bedeutet, oder

R12 und R13 gemeinsam und unter Einschluß des Stickstoffatoms, an das beide gebunden sind, einen Rest der Formel

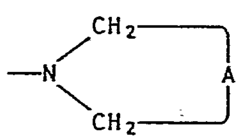

darstellen, worin

A $-CH_2-C(R14)R15-CH_2-$,
$-CH_2-O-CH_2-$oder
$-CH_2-NR16-CH_2-$bedeutet,

R14 Wasserstoff (H) oder Phenyl bedeutet,

R15 Wasserstoff (H), Phenyl oder 4-Fluorbenzoyl bedeutet,

R16 Aryl, 2-Pyridyl oder Benzhydryl bedeutet, wobei

Aryl für einen Ring der Formel

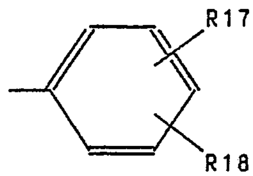

steht, worin R17 und R18 gleich oder verschieden sind und die Bedeutung Wasserstoff (H), 1-4C-Alkyl, 1-4C-Alkoxy, Trifluormethyl oder gemeinsam Methylendioxy haben,
und die Salze dieser Verbindungen.

3. Verbindungen der Formel I nach Anspruch 1, charakterisiert durch die folgende Formel Ig

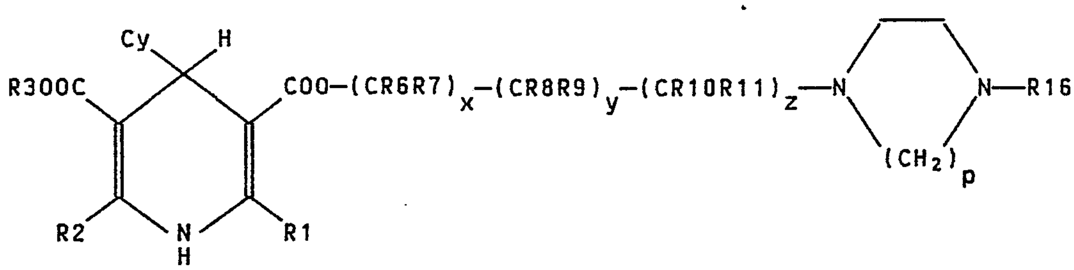

(Ig)

worin

Cy Phenyl, 2-Nitrophenyl, 3-Nitrophenyl, 2-Cyanophenyl, 3-Cyanophenyl, 2-(1,1,2,2-Tetrafluorethoxy)-phenyl, 3-(1,1,2,2-Tetrafluorethoxy)-phenyl, 2-Difluromethoxyphenyl, 3-Difluormethoxyphenyl, 2-Chlorphenyl, 3-Chlorphenyl, 2,3-Dichlorphenyl, 2-Fluorphenyl, 3-Fluorphenyl, 2-Trifluormethylphenyl, 3-Trifluormethylphenyl, 3-Pyridyl oder Benzoxdiazolyl bedeutet,

R1 Methyl bedeutet,

R2 Methyl bedeutet,

R3 einen (-)-Menthylrest darstellt,

die Gruppierung $(CR6R7)_x-(CR8R9)_y-(CR10R11)_z$ Ethylen oder Propylen bedeutet,

R16 Aryl, Diaryl-methyl, 2-Pyridyl oder 2-Pyrimidyl bedeutet, wobei

Aryl für einen Ring der Formel

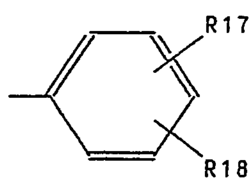

steht worin R17 und R18 gleich oder verschieden sind und die Bedeutung Wasserstoff (H), 1-4C-Alkyl, 1-4C-Alkoxy, Halogen, Hydorxy, Trifluormethyl oder gemeinsam Methylendioxy haben,

p die Zahl 2 darstellt,

und ihre Salze.

4. Verbindungen der Formel Ig nach Anspruch 3, worin Cy 3-Nitrophenyl, 2-Chlorphenyl, 2,3-Dichlorphenyl, 2-Trifluormethylphenyl, 2-Difluormethoxyphenyl oder Benzoxdiazolyl bedeutet,
R1 Methyl bedeutet,
R2 Methyl bedeutet,
R3 einen (-)-Menthylrest darstellt,
die Gruppierung $(CR6R7)_x$-$(CR8R9)_y$-$(CR10R11)_z$ Ethylen oder Propylen bedeutet,
R16 Phenyl, 2-Methoxyphenyl, 2-Ethoxyphenyl, 3-Trifluormethylphenyl, 3-Methoxyphenyl, 3,4-Methylendioxyphenyl, 2-Methylphenyl, 2,4-Dimethylphenyl, 2,6-Dimethylphenyl, Benzhydryl oder 2-Pyridyl bedeutet,
p die Zahl 2 darstellt,
und ihre Salze.

5. Verbindungen der Formel Ig nach Anspruch 3, worin Cy 3-Nitrophenyl oder 2,3-Dichlorphenyl bedeutet,
R1 Methyl bedeutet,
R2 Methyl bedeutet,
R3 einen (-)-Menthylrest darstellt,
die Gruppierung $(CR6R7)_x$-$(CR8R9)_y(CR10R11)_z$ Ethylen oder Propylen bedeutet,
R16 Phenyl, 2-Methoxyphenyl, 2-Ethoxyphenyl, 3-Trifluormethylphenyl, 3-Methoxyphenyl, 3,4-Methylendioxyphenyl, 2-Methylphenyl, 2,4-Dimethylphenyl, 2,6-Dimethylphenyl, Benzhydryl oder 2-Pyridyl bedeutet, und
p die Zahl 2 darstellt,
und ihre Salze.

6. Verbindungen der Formel I nach Anspruch 1, worin
Cy 3-Nitrophenyl, 2-Chlorphenyl, 2,3-Dichlorphenyl, 2-Trifluormethylphenyl, 2-Difluormethoxyphenyl, 3-Pyridyl oder Benzoxdiazolyl bedeutet,
R1 Methyl bedeutet,
R2 Methyl bedeutet,
R3 einen (-)-Menthylrest darstellt,
die Gruppierung $(CR6R7)_x$-$(CR8R9)_y$-$(CR10R11)_z$ Ethylen oder Propylen be deutet,
R12 und R13 gemeinsam und unter Einschluß des Stickstoffatoms, an das beide gebunden sind, einen Rest der Formel

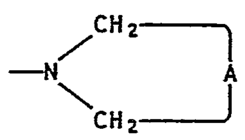

darstellen, worin
A $-CH_2-C(R14)R15-CH_2-$bedeutet,
R14 Phenyl oder 4-Methoxyphenyl bedeutet und
R15 Phenyl oder 4-Methoxyphenyl bedeutet,
und ihre Salze.

7. Verbindungen der Formel I nach Anspruch l, ausgewählt aus der Gruppe bestehend aus
1,4-Dihydro-2,6-dimethyl-4-(3-nitrophenyl)-pyridin-3,5-dicarbonsäure-3-(-)-menthyl-5-{2-[4-(2-methoxyphenyl)-1-piperazinyl]-ethyl}-ester,
1,4-Dihydro-2,6-dimethyl-4-(3-nitrophenyl)-pyridin-3,5-dicarbonsäure-3-(-)-menthyl-5{3-[4-(2-methoxyphenyl)-1-piperazinyl]-propyl}-ester,
1,4-Dihydro-2,6-dimethyl-4-(3-nitrophenyl)-pyridin-3,5-dicarbonsäure-3-(-)-menthyl-5-[2-(4-phenyl-1-piperazinyl)-ethyl]-ester,
1,4-Dihydro-2,6-dimethyl-4-(3-nitrophenyl)-pyridin-3,5-dicarbonsäure-3-(-)-menthyl-5-[3-(4-phenyl-1-piperazinyl)-propyl]-ester,
1,4-Dihydro-2,6-dimethyl-4-(3-nitrophenyl)-pyridin-3,5-dicarbonsäure-3-(-)-menthyl-5-{2-[4-(2-pyridyl)-1-piperazinyl]-ethyl}-ester,
1,4-Dihydro-2,6-dimethyl-4-(3-nitrophenyl)-pyridin-3,5-dicarbonsäure-3-(-)-menthyl-5-{3-[4-(2-pyridyl)-1-piperazinyl]-propyl}-ester,
1,4-Dihydro-2,6-dimethyl-4-(3-nitrophenyl)-pyridin-3,5-dicarbonsäure-3-(-)-menthyl-5-[2-(4-benzhydryl-1-piperazinyl)-ethyl]-ester,

1,4-Dihydro-2,6-dimethyl-4-(3-nitrophenyl)-pyridin-3,5-dicarbonsäure-3-(-)-menthyl-5-[3-(4-benzhydryl-1-piperazinyl)-propyl]-ester

1,4-Dihydro-2,6-dimethyl-4-(3-nitrophenyl)-pyridin-3,5-dicarbonsäure-3-(-)-menthyl-5-{3-[4-(2-ethoxyphenyl)-1-piperazinyl]-propyl}-ester

1,4-Dihydro-2,6-dimethyl-4-(3-nitrophenyl)-pyridin-3,5-dicarbonsäure-3-(-)-menthyl-5-{2-[4-(2-ethoxyphenyl)-1-piperazinyl]-ethyl}-ester

1,4-Dihydro-2,6-dimethyl-4-(3-nitrophenyl)-pyridin-3,5-dicarbonsäure-3-(-)-menthyl-5-{3-[4-(3-trifluormethylphenyl)-1-piperazinyl]-propyl}-ester

1,4-Dihydro-2,6-dimethyl-4-(3-nitrophenyl)-pyridin-3,5-dicarbonsäure-3-(-)-menthyl-5-{2-[4-(3-trifluormethylphenyl)-1-piperazinyl]-ethyl}-ester

1,4-Dihydro-2,6-dimethyl-4-(3-nitrophenyl)-pyridin-3,5-dicarbonsäure-3-(-)-menthyl-5-{3-[4-(3-methoxyphenyl)-1-piperazinyl]-propyl}-ester

1,4-Dihydro-2,6-dimethyl-4-(3-nitrophenyl)-pyridin-3,5-dicarbonsäure-3-(-)-menthyl-5-{2-[4-(3-methoxyphenyl)-1-piperazinyl]-ethyl}-ester

1,4-Dihydro-2,6-dimethyl-4-(3-nitrophenyl)-pyridin-3,5-dicarbonsäure-3-(-)-menthyl-5-{3-[4-(3,4-methylendioxyphenyl)-1-piperazinyl]-propyl}-ester

1,4-Dihydro-2,6-dimethyl-4-(3-nitrophenyl)-pyridin-3,5-dicarbonsäure-3-(-)-menthyl-5-{2-[4-(3,4-methylendioxyphenyl)-1-piperazinyl]-ethyl}-ester

1,4-Dihydro-2,6-dimethyl-4-(3-nitrophenyl)-pyridin-3,5-dicarbonsäure-3-(-)-menthyl-5-{3-[4-(2-tolyl)-1-piperazinyl]-propyl}-ester

1,4-Dihydro-2,6-dimethyl-4-(3-nitrophenyl)-pyridin-3,5-dicarbonsäure-3-(-)-menthyl-5-{2-[4-(2-tolyl)-1-piperazinyl]-ethyl}-ester

1,4-Dihydro-2,6-dimethyl-4-(3-nitrophenyl)-pyridin-3,5-dicarbonsäure-3-(-)-menthyl-5-{3-[4-(2,4-dimethylphenyl)-1-piperazinyl]-propyl}-ester

1,4-Dihydro-2,6-dimethyl-4-(3-nitrophenyl)-pyridin-3,5-dicarbonsäure-3-(-)-menthyl-5-{2-[4-(2,4-dimethylphenyl)-1-piperazinyl]-ethyl}-ester

1,4-Dihydro-2,6-dimethyl-4-(3-nitrophenyl)-pyridin-3,5-dicarbonsäure-3-(-)-menthyl-5-{3-[4-(2,6-dimethylphenyl)-1-piperazinyl]-propyl}-ester

1,4-Dihydro-2,6-dimethyl-4-(3-nitrophenyl)-pyridin-3,5-dicarbonsäure-3-(-)-menthyl-5-{2-[4(2,6-dimethylphenyl)-1-piperazinyl]-ethyl}-ester

4-(2,3-Dichlorphenyl)-1,4-dihydro-2,6-dimethyl-pyridin-3,5-dicarbonsäure-3-(-)-menthyl-5-[3-(4-phenyl-1-piperazinyl)-propyl]-ester

4-(2,3-Dichlorphenyl)-1,4-dihydro-2,6-dimethyl-pyridin-3,5-dicarbonsäure-3(-)-menthyl-5-[2-(4-phenyl-1-piperazinyl)-ethyl]-ester

4-(2,3-Dichlorphenyl)-1,4-dihydro-2,6-dimethyl-pyridin-3,5-dicarbonsäure-3-(-)-menthyl-5-{3-[4-(2-methoxyphenyl)-1-piperazinyl]-propyl}-ester

4-(2,3-Dichlorphenyl)-1,4-dihydro-2,6-dimethyl-pyridin-3,5-dicarbonsäure-3-(-)-menthyl-5-{2-[4-(2-methoxyphenyl)-1-piperazinyl]-ethyl}-ester

4-(2,3-Dichlorphenyl)-1,4-dihydro-2,6-dimethyl-pyridin-3,5-dicarbonsäure-3-(-)-menthyl-5-{3-[4-(2-ethoxyphenyl)-1-piperazinyl]-propyl}-ester

4-(2,3-Dichlorphenyl-1,4-dihydro-2,6-dimethyl-pyridin-3,5-dicarbonsäure-3-(-)-menthyl-5-{2-[4-(2-ethoxyphenyl)-1-piperazinyl]-ethyl}-ester

1,4-Dihydro-2,6-dimethyl-4-(3-nitrophenyl)-pyridin-3,5-dicarbonsäure-3-(-)-menthyl-5-[2-(4,4-diphenylpiperidyl-1)-ethyl]-ester,

1,4-Dihydro-2,6-dimethyl-4-(3-nitrophenyl)-pyridin-3,5-dicarbonsäure-3-(-)-menthyl-5-[3-(4,4-diphenylpiperidyl-1)-propyl]-ester

1,4-Dihydro-2,6-dimethyl-4-(3-pyridyl)-pyridin-3,5-dicarbonsäure-3-(-)-menthyl-5-[2-(4,4-diphenylpiperidyl-1)-ethyl]-ester,

1,4-Dihydro-2,6-dimethyl-4-(3-pyridyl)-pyridin-3,5-dicarbonsäure-3-(-)-menthyl-5-[3-(4,4-diphenylpiperidyl-1)-propyl]-ester

1,4-Dihydro-2,6-dimethyl-4-(3-nitrophenyl)-pyridin-3,5-dicarbonsäure-3-(-)-menthyl-5-[2-(benzylmethylamino)-ethyl]-ester,

1,4-Dihydro-2,6-dimethyl-4-(3-nitrophenyl)-pyridin-3,5-dicarbonsäure3-(-)-menthyl-5-[3-(benzylmethylamino)-propyl]-ester

1,4-Dihydro-2,6-dimethyl-4-(3-nitrophenyl)-pyridin-3,5-dicarbonsäure-3-(-)-menthyl-5-[2-(4-morpholino)-ethyl]-ester,

1,4-Dihydro-2,6-dimethyl-4-(3-nitrophenyl)-pyridin-3,5-dicarbonsäure-3-(-)-menthyl-5-[2-(1-piperidino)-ethyl]-ester,

1,4-Dihydro-2,6-dimethyl-4-(3-nitrophenyl)-pyridin-3,5-dicarbonsäure-3-(-)-menthyl-5-[2-(1-pyrrolidino)-ethyl]-ester,
1,4-Dihydro-2,6-dimethyl-4-(3-nitrophenyl)-pyridin-3,5-dicarbonsäure-3-(-)-menthyl-5-[3-(4-morpholino)-propyl]-ester,
1,4-Dihydro-2,6-dimethyl-4-(3-nitrophenyl)-pyridin-3,5-dicarbonsäure-3-(-)-menthyl-5-[3-(1-piperidono)-propyl]-ester,
1,4-Dihydro-2,6-dimethyl-4-(3-nitrophenyl)-pyridin-3,5-dicarbonsäure-3-(-)-menthyl-5-[3-(1-pyrrolidino)-propyl]-ester
1,4-Dihydro-2,6-dimethyl-4-(3-nitrophenyl)-pyridin-3,5-dicarbonsäure-3-(-)-menthyl-5-[2-(4-phenylpiperidyl-1)-ethyl]-ester,
1,4-Dihydro-2,6-dimethyl-4-(3-nitrophenyl)-pyridin-3,5-dicarbonsäure-3-(-)-menthyl-5-[3-(4-phenylpiperidyl-1)-propyl]-ester,
1,4-Dihydro-2,6-dimethyl-4-(3-nitrophenyl)-pyridin-3,5-dicarbonsäure-3-(-)-menthyl-5-{2-[4-(4-fluorbenzoyl)-piperidyl-1)-ethyl]-ester,
1,4-Dihydro-2,6-dimethyl-4-(3-nitrophenyl)-pyridin-3,5-dicarbonsäure-3-(-)-menthyl-5-{3-[4-(4-fluorbenzoyl)-piperidyl-1)-propyl]-ester
und den Salzen dieser Verbindungen.

8. Verfahren zur Herstellung der Verbindungen der Formel I nach Anspruch 1, dadurch gekennzeichnet, daß man

a) Zimtsäurederivate der Formel II

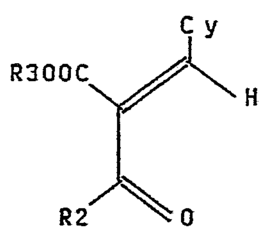

(II),

mit Enaminderivaten der Formel III

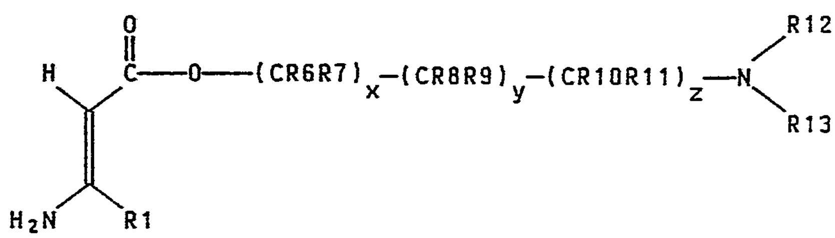

(III),

oder

b) Zimtsäurederivate der Formel II mit Ammoniak und $\beta$-Ketocarbonsäurederivaten der Formel IV

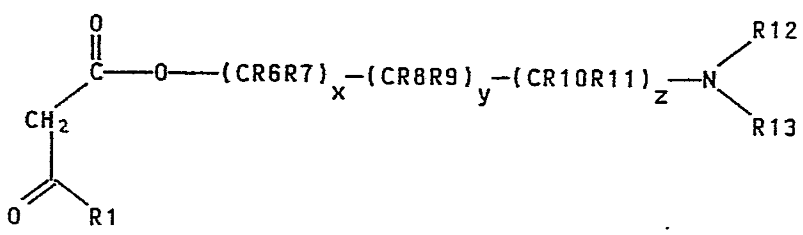

(IV),

oder

c) Enamine der Formel V

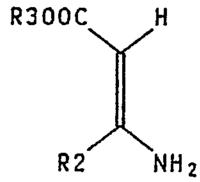

(V),

mit Benzylidencarbonsäurederivaten der Formel VI

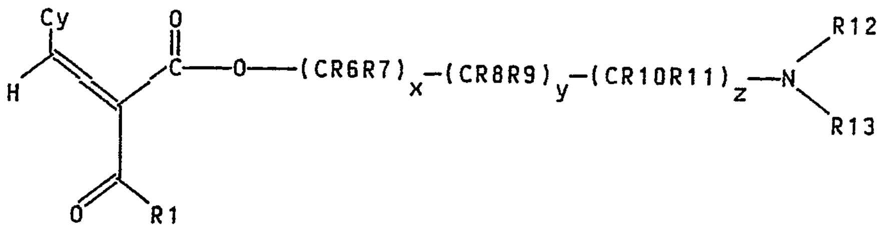

(VI),

oder

d) Ketoverbindungen der Formel VII

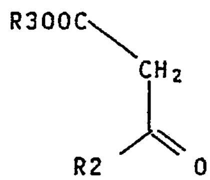

(VII),

mit Ammoniak und Benzylidencarbonsäurederivaten der Formel VI, oder

e) Aldehyde der Formel VIII

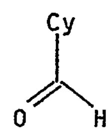

(VIII),

mit Enaminen der Formel V und β-Ketocarbonsäurederivaten der Formel IV, oder

f) Aldehyde der Formel VIII mit Enaminderivaten der Formel III und Ketoverbundungen der Formel VII, oder

g) 1,4-Dihydropyridine der Formel IX

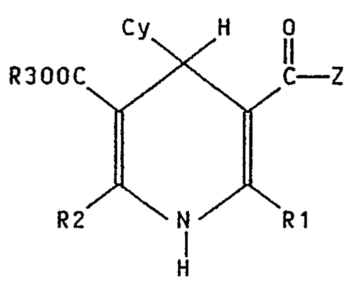

(IX),

mit Aminderivaten der Formel X

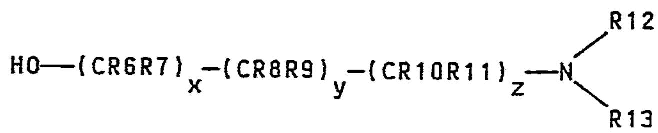

(X),

oder

h) 1,4-Dihydropyridinderivate der Formel XI

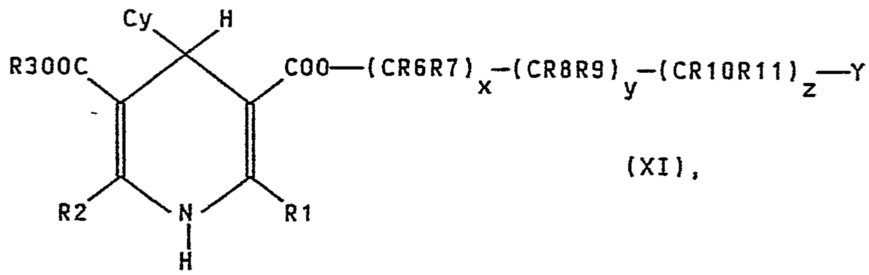

(XI),

mit Aminen der Formel XII

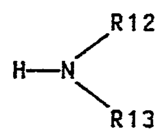

(XII),

als solche(n) oder in Form ihrer Salze umsetzt und gewünschtenfalls anschließend erhaltene Salze in die freien Basen oder erhaltene Base in die Salze überführt, wobei Cy, R1, R2, R3, R4, R5, R6, R7, R8, R9, R10, R11, R12, R13, x, y und z die in Anspruch 1 angegebenen Bedeutungen haben, Z gemeinsam mit der Carbonylgruppe, woran es gebunden ist, eine Carboxylgruppe oder ein reaktives Carbonsäurederivat und Y eine Fluchtgruppe darstellt.

9. Arzneimittel enthaltend eine oder mehrere Verbindungen nach einem oder mehreren der Ansprüche 1 bis 7 und/oder ihre pharmakologisch verträglichen Salze.

10. Verbindungen nach einem oder mehreren der Ansprüche 1 bis 7 und ihre pharmakologisch verträglichen Salze zur Anwendung bei der Behandlung und/oder Prophylaxe von Hypertonie, koronaren Herzkrankheiten, peripheren und cerebralen Zirkulationsstörungen und/oder Krankheiten, die auf einer erhöhten Wasser-oder Natriumretention beruhen.

(Patentansprüche für folgende Vertragsstaaten: AT, ES und GR )

1. Verfahren zur Herstellung von Verbindungen der Formel I

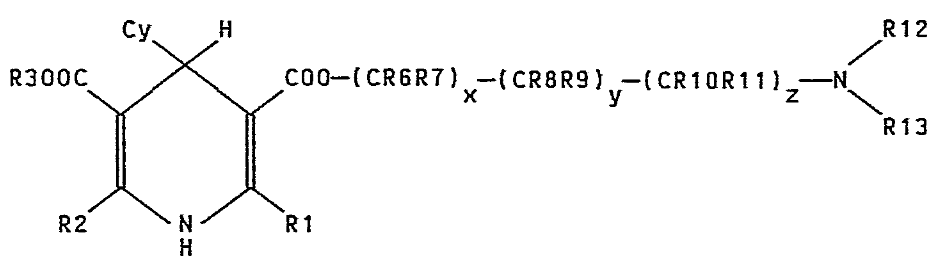

(I)

worin Cy einen Cyclus der Formel

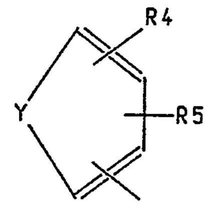

darstellt, in dem Y Sauerstoff (O), Schwefel (S), Vinylen (-CH = CH-), Azomethin (-CH = N-) oder eine Gruppe der Formel

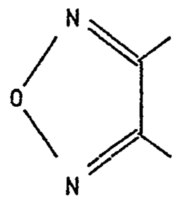

oder

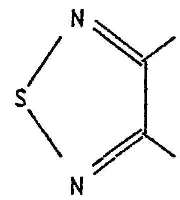

bedeutet,

R1 und R2 gleich oder verschieden sind und Wasserstoff, 1-6C-Alkyl oder 3-7C-Alkoxyalkyl bedeuten,

R3 einen (-)-Menthylrest darstellt,

R4 und R5 gleich oder verschieden sind und Wasserstoff, Hydroxy, Halogen, Nitro, Cyano, Trifluormethyl, 1-4C-Alkyl, 1-4C-Alkoxy, ganz oder teilweise durch Fluor substituiertes 1-4C-Alkoxy, 1-4C-Alkoxy-carbonyl, 2-5C-Acyl, Amino oder Mono-oder Di-1-4C-alkylamino bedeuten,

R6 Wasserstoff (H) oder 1-4C-Alkyl bedeutet,

R7 Wasserstoff (H), 1-4C-Alkyl oder gemeinsam mit R12 1-4C-Alkylen bedeutet, das gewünschtenfalls durch eine oder mehrere 1-4C-Alkylgruppen substituiert ist,

R8 Wasserstoff (H) oder 1-4C-Alkyl bedeutet,

R9 Wasserstoff (H), 1-4C-Alkyl, 1-4C-Alkoxy, Aryl, Heteroaryl oder gemeinsam mit R12 1-4C-Alkylen bedeutet, das gewünschtenfalls durch eine oder mehrere 1-4C-Alkylgruppen substituiert ist,

R10 Wasserstoff (H) oder 1-4C-Alkyl bedeutet,

R11 Wasserstoff (H) oder 1-4C-Alkyl bedeutet,

R12 Wasserstoff (H), 1-4C-Alkyl oder gemeinsam mit R7 oder R9 1-4C-Alkylen bedeutet, das gewünschtenfalls durch eine oder mehrere 1-4C-Alkylgruppen substituiert ist, und

R13 1-4C-Alkyl, Aryl, Aryl-1-4C-alkyl, Aryl-2-4C-alkenyl, Aryl-2-4C-alkinyl, 2-5C-Acyl, Arylcarbonyl oder Heteroarylcarbonyl bedeutet, oder

R12 und R13 gemeinsam und unter Einschluß des Stickstoffatoms, an das beide gebunden sind, einen Rest der Formel

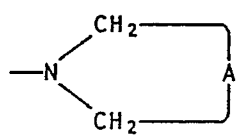

darstellen, worin

A $-CH_2-(CH_2)_m-$,

$-CH_2-C(R14)R15-CH_2-$,

$-CH = CR15-CH_2-$,

$-CH_2-O-CH_2-$,

$-CH_2-S-CH_2-$,

$-CH_2-NR16-CH_2-$oder

$-(CH_2)_2-NR16-CH_2-$bedeutet,

R14 Wasserstoff (H) oder Aryl und

R15 Wasserstoff (H), 1-4C-Alkyl, 1-4C-Alkoxy, Hydroxy, Aryl, Arylcarbonyl oder Heteroaryl bedeutet, oder

R14 und R15 gemeinsam Diarylmethylen bedeuten,

R16 1-4C-Alkyl, Aryl, Aryl-1-4C-alkyl, Aryl-2-4C-alkenyl, Aryl-2-4c-alkinyl, Diaryl-1-4C-alkyl, Heteroaryl, Heteroaryl-1-4C-alkyl, Heteroaryl-aryl-1-4C-alkyl, Di-heteroaryl-1-4C-alkyl, Arylcarbonyl, Heteroarylcarbonyl, Arylsulfonyl, Aryl-1-4C-alkylcarbonyl oder Aryl-2-4C-alkenylcarbonyl bedeutet,

x, y und z gleich oder verschieden sind und die Zahlen 0, 1, 2, 3 oder 4 bedeuten, wobei die Summe von x, y und z eine Zahl von 1 bis 5 ist, und

m die Zahl 1, 2 oder 3 bedeutet, wobei

Aryl für einen Ring der Formel

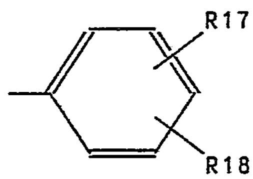

steht, worin R17 und R18 gleich oder verschieden sind und die Bedeutung Wasserstoff (H), 1-4C-Alkyl, 1-4C-Alkoxy, Halogen, Hydroxy, Trifluormethyl oder gemeinsam Methylendioxy haben, und

Heteroaryl für einen 5-oder 6-gliedrigen Heterocyclus mit einem oder zwei gleichen oder verschiedenen Heteroatomen aus der Gruppe Sauerstoff (O), Schwefel (S) oder Stickstoff (N) steht, der ungesättigt oder teilweise oder ganz gesättigt ist und der einen oder zwei Substituenten aus der Gruppe 1-4C-Alkyl, 1-4C-Alkoxy, Halogen, Trifluormethyl oder Cyan tragen kann,

und den Salzen dieser Verbindungen,

dadurch gekennzeichnet, daß man

a) Zimtsäurederivate der Formel II

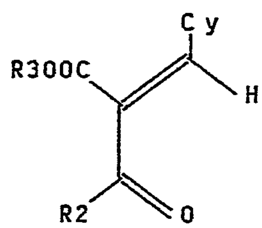

(II),

mit Enaminderivaten der Formel III

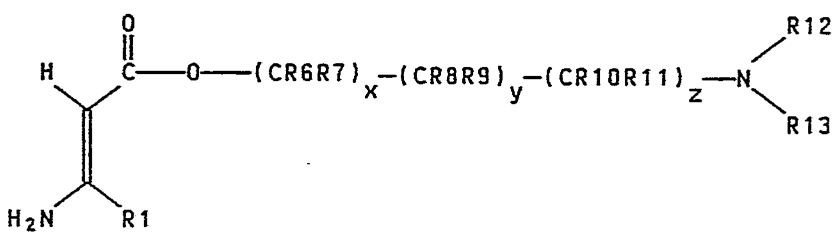

(III),

oder

b) Zimtsäurederivate der Formel II mit Ammoniak und $\beta$-Ketocarbonsäurederivaten der Formel IV

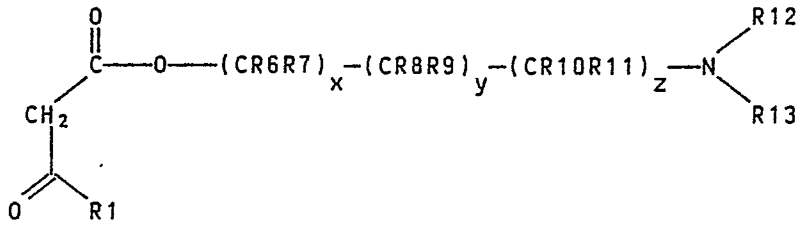

(IV),

oder

c) Enamine der Formel V

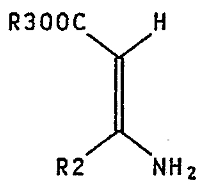

(V),

mit Benzylidencarbonsäurederivaten der Formel VI

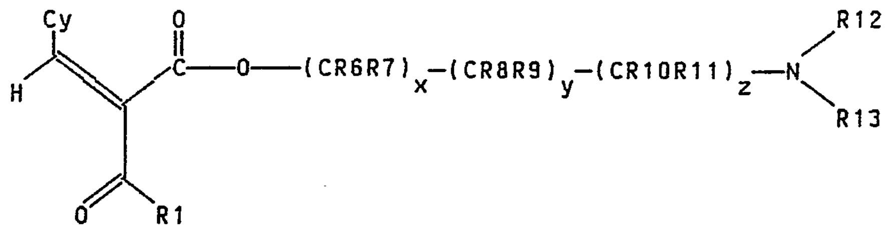

(VI),

oder

d) Ketoverbindungen der Formel VII

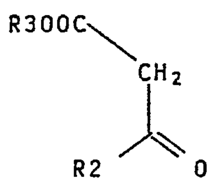

(VII),

mit Ammoniak und Benzylidencarbonsäurederivaten der Formel VI, oder

e) Aldehyde der Formel VIII

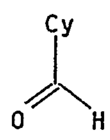

(VIII),

mit Enaminen der Formel V und $\beta$-Ketocarbonsäurederivaten der Formel IV, oder

f) Aldehyde der Formel VIII mit Enaminderivaten der Formel III und Ketoverbindungen der Formel VII, oder

g) 1,4-Dihydropyridine der Formel IX

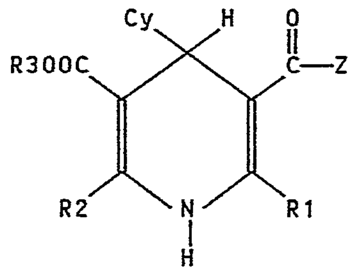

(IX),

mit Aminderivaten der Formel X

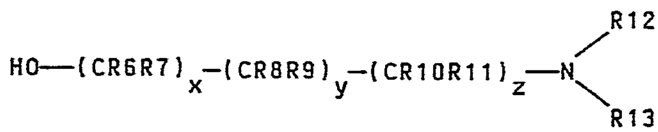

(X),

oder

h) 1,4-Dihydropyridinderivate der Formel XI

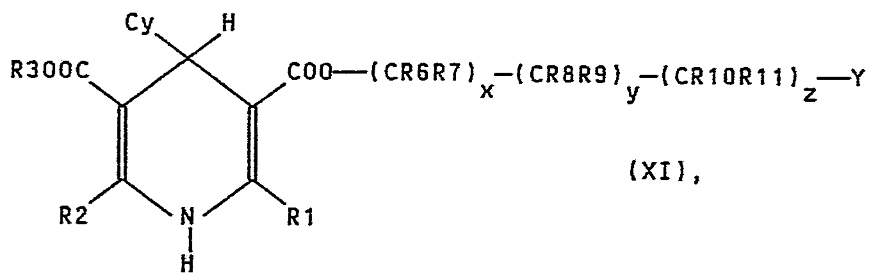

(XI),

mit Aminen der Formel XII

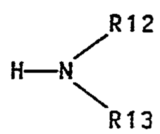

(XII),

als solche(n) oder in Form ihrer Salze umsetzt und gewünschtenfalls anschließend erhaltene Salze in die freien Basen oder erhaltene Basen in die Salze überführt, wobei Cy, R1, R2, R3, R4, R5, R6, R7, R8, R9, R10, R11, R12, R13, x, y und z die oben angegebenen Bedeutungen haben, Z gemeinsam mit der Carbonylgruppe, woran es gebunden ist, eine Carboxylgruppe oder ein reaktives Carbonsäurederivat und Y eine Fluchtgruppe darstellt.

2. Verfahren nach Anspruch 1 zur Herstellung von Verbindungen der Formel I nach Anspruch 1, worin
Cy 3-Nitrophenyl, 2,3-Dichlorphenyl oder 3-Pyridyl bedeutet,
R1 und R2 gleich oder verschieden sind und 1-4C-Alkyl bedeuten,
R3 einen (-)-Menthylrest darstellt,
die Gruppierung $(CR6R7)_x$-$(CR8R9)_y$-$(CR10R11)_z$ Ethylen oder Propylen bedeutet,
R12 1-4C-Alkyl bedeutet und
R13 Benzyl bedeutet, oder
R12 und R13 gemeinsam und unter Einschluß des Stickstoffatoms, an das beide gebunden sind, einen Rest der Formel

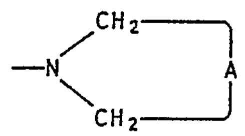

darstellen, worin
A $-CH_2-C(R14)R15-CH_2-$,
$-CH_2-O-CH_2-$oder
$-CH_2-NR16-CH_2-$bedeutet,
R14 Wasserstoff (H) oder Phenyl bedeutet,
R15 Wasserstoff (H), Phenyl oder 4-Fluorbenzoyl bedeutet,
R16 Aryl, 2-Pyridyl oder Benzhydryl bedeutet, wobei
Aryl für einen Ring der Formel

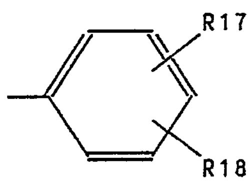

steht, worin R17 und R18 gleich oder verschieden sind und die Bedeutung Wasserstoff (H), 1-4C-Alkyl, 1-4C-Alkoxy, Trifluormethyl oder gemeinsam Methylendioxy haben,
und den Salzen dieser Verbindung.

3. Verfahren nach Anspruch 1 zur Herstellung von Verbindungen der Formel I nach Anspruch 1, charakterisiert durch die folgende Formel Ig

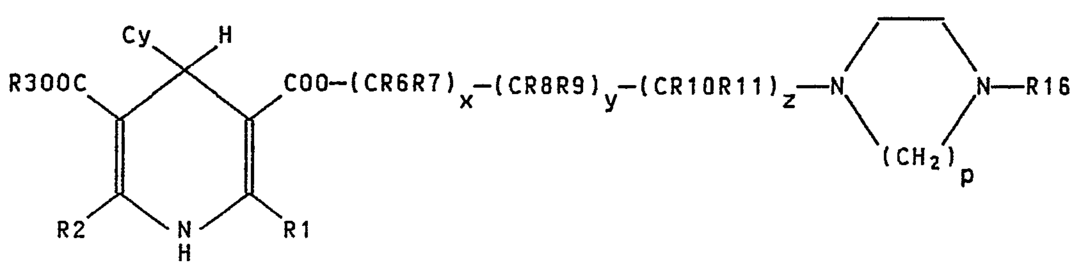

(Ig)

worin
Cy Phenyl, 2-Nitrophenyl, 3-Nitrophenyl, 2-Cyanophenyl, 3-Cyanophenyl, 2-(1,1,2,2-Tetrafluorethoxy)-phenyl, 3-(1,1,2,2-Tetrafluorethoxy)-phenyl, 2-Difluormethoxyphenyl, 3-Difluormethoxyphenyl, 2-Chlorphenyl, 3-Chlorphenyl, 2,3-Dichlorphenyl, 2-Fluorphenyl, 3-Fluorphenyl, 2-Trifluormethylphenyl, 3-Trifluormethylphenyl, 3-Pyridyl oder Benzoxdiazolyl bedeutet,
R1 Methyl bedeutet,
R2 Methyl bedeutet,
R3 einen (-)-Menthylrest darstellt,
die Gruppierung $(CR6R7)_x-(CR8R9)_y-(CR10R11)_z$ Ethylen oder Propylen bedeutet,
R16 Aryl, Diaryl-methyl, 2-Pyridyl oder 2-Pyrimidyl bedeutet, wobei
Aryl für einen Ring der Formel

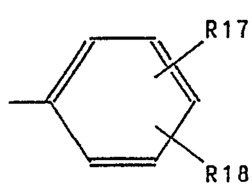

steht worin R17 und R18 gleich oder verschieden sind und die Bedeutung Wasserstoff (H), 1-4C-Alkyl, 1-4C-Alkoxy, Halogen, Hydroxy, Trifluormethyl oder gemeinsam Methylendioxy haben.
p die Zahl 2 darstellt,
und ihren Salzen.

4. Verfahren nach Anspruch 1 zur Herstellung von Verbindungen der Formel Ig nach Anspruch 3, worin
Cy 3-Nitrophenyl, 2-Chlorphenyl, 2,3-Dichlorphenyl, 2-Trifluormethylphenyl, 2-Difluormethoxyphenyl oder Benzoxdiazolyl bedeutet,
R1 Methyl bedeutet,
R2 Methyl bedeutet,
R3 einen (-)-Menthylrest darstellt,
die Gruppierung $(CR6R7)_x$-$(CR8R9)_y$-$(CR10R11)_z$ Ethylen oder Propylen bedeutet,
R16 Phenyl, 2-Methoxyphenyl, 2-Ethoxyphenyl, 3-Trifluormethylphenyl, 3-Methoxyphenyl, 3,4-Methylendioxyphenyl, 2-Methylphenyl, 2,4-Dimethylphenyl, 2,6-Dimethylphenyl, Benzhydryl oder 2-Pyridyl bedeutet,
p die Zahl 2 darstellt,
und ihren Salzen.

5. Verfahren nach Anspruch 1 zur Herstellung von Verbindungen der Formel Ig nach Anspruch 3, worin
Cy 3-Nitrophenyl oder 2,3-Dichlorphenyl bedeutet,
R1 Methyl bedeutet,
R2 Methyl bedeutet,
R3 einen (-)-Menthylrest darstellt,
die Gruppierung $(CR6R7)_x$-$(CR8R9)_y$-$(CR10R11)_z$ Ethylen oder Propylen bedeutet,
R16 Phenyl, 2-Methoxyphenyl, 2-Ethoxyphenyl, 3-Trifluormethylphenyl, 3-Methoxyphenyl, 3,4-Methylendioxyphenyl, 2-Methylphenyl, 2,4-Dimethylphenyl, 2,6-Dimethylphenyl, Benzhydryl oder 2-Pyridyl bedeutet, und
p die Zahl 2 darstellt,
und ihren Salzen.

6. Verfahren nach Anspruch 1 zur Herstellung von Verbindungen der Formel I nach Anspruch 1, worin
Cy 3-Nitrophenyl, 2-Chlorphenyl, 2,3-Dichlorphenyl, 2-Trifuormethylphenyl, 2-Difluormethoxyphenyl, 3-Pyridyl oder Benzoxdiazolyl bedeutet,
R1 Methyl bedeutet,
R2 Methyl bedeutet,
R3 einen (-)-Menthylrest darstellt,
die Gruppierung $(CR6R7)_x$-$(CR8R9)_y$-$(CR10R11)_z$ Ethylen oder Propylen bedeutet,
R12 und R13 gemeinsam und unter Einschluß des Stickstoffatoms, an das beide gebunden sind, einen Rest der Formel

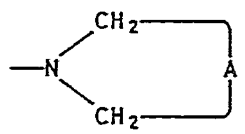

darstellen, worin
A $-CH_2-C(R14)R15-CH_2-$bedeutet,
R14 Phenyl oder 4-Methoxyphenyl bedeutet und
R15 Phenyl oder 4-Methoxyphenyl bedeutet,
und ihren Salzen.

7. Verfahren nach Anspruch 1 zur Herstellung von Verbindungen der Formel I nach Anspruch I, ausgewählt aus der Gruppe bestehend aus
1,4-Dihydro-2,6-dimethyl-4-(3-nitrophenyl)-pyridin-3,5-dicarbonsäure-3-(-)-menthyl-5-{2-[4-(2-methoxyphenyl)-1-piperazinyl]-ethyl}-ester,
1,4-Dihydro-2,6-dimethyl-4-(3-nitrophenyl)-pyridin-3,5-dicarbonsäure-3-(-)-menthyl-5{3-[4-(2-methoxyphenyl)-1-piperazinyl]-propyl}-ester,
1,4-Dihydro-2,6-dimethyl-4-(3-nitrophenyl)-pyridin-3,5-dicarbonsäure-3-(-)-menthyl-5-[2-(4-phenyl-1-piperazinyl)-ethyl]-ester,
1,4-Dihydro-2,6-dimethyl-4-(3-nitrophenyl)-pyridin-3,5-dicarbonsäure-3-(-)-menthyl-5-[3-(4-phenyl-1-piperazinyl)-propyl]-ester,
1,4-Dihydro-2,6-dimethyl-4-(3-nitrophenyl)-pyridin-3,5-dicarbonsäure-3-(-)-menthyl-5-{2-[4-(2-pyridyl)-1-piperazinyl]-ethyl}-ester,
1,4-Dihydro-2,6-dimethyl-4-(3-nitrophenyl)-pyridin-3,5-dicarbonsäure-3-(-)-menthyl-5-{3-[4-(2-pyridyl)-1-piperazinyl]-propyl}-ester,
1,4-Dihydro-2,6-dimethyl-4-(3-nitrophenyl)-pyridin-3,5-dicarbonsäure-3-(-)-menthyl-5-[2-(4-benzhydryl-1-piperazinyl)-ethyl]-ester,
1,4-Dihydro-2,6-dimethyl-4-(3-nitrophenyl)-pyridin-3,5-dicarbonsäure-3-(-)-menthyl-5-[3-(4-benzhydryl-1-piperazinyl)-propyl]-ester
1,4-Dihydro-2,6-dimethyl-4-(3-nitrophenyl)-pyridin-3,5-dicarbonsäure-3-(-)-menthyl-5-{3-[4-(2-ethoxyphenyl)-1-piperazinyl]-propyl}-ester
1,4-Dihydro-2,6-dimethyl-4-(3-nitrophenyl)-pyridin-3,5-dicarbonsäure-3-(-)-menthyl-5-{2-[4-(2-ethoxyphenyl)-1-piperazinyl]-ethyl}-ester
1,4-Dihydro-2,6-dimethyl-4-(3-nitrophenyl)-pyridin-3,5-dicarbonsäure-3-(-)-menthyl-5-{3-[4-(3-trifluormethylphenyl)-1-piperazinyl]-propyl}-ester
1,4-Dihydro-2,6-dimethyl-4-(3-nitrophenyl)-pyridin-3,5-dicarbonsäure-3-(-)-menthyl-5-{2-[4-(3-trifluormethylphenyl)-1-piperazinyl]-ethyl}-ester
1,4-Dihydro-2,6-dimethyl-4-(3-nitrophenyl)-pyridin-3,5-dicarbonsäure-3-(-)-menthyl-5-{3-[4-(3-methoxyphenyl)-1-piperazinyl]-propyl}-ester
1,4-Dihydro-2,6-dimethyl-4-(3-nitrophenyl)-pyridin-3,5-dicarbonsäure-3-(-)-menthyl-5-{2-[4-(3-methoxyphenyl)-1-piperazinyl]-ethyl}-ester
1,4-Dihydro-2,6-dimethyl-4-(3-nitrophenyl)-pyridin-3,5-dicarbonsäure-3-(-)-menthyl-5-{3-[4-(3,4-methylendioxyphenyl)-1-piperazinyl]-propyl}-ester
1,4-Dihydro-2,6-dimethyl-4-(3-nitrophenyl)-pyridin-3,5-dicarbonsäure-3-(-)-menthyl-5-{2-[4-(3,4-menthylendioxyphenyl)-1-piperazinyl]-ethyl}-ester
1,4-Dihydro-2,6-dimethyl-4-(3-nitrophenyl)-pyridin-3,5-dicarbonsäure-3-(-)-menthyl-5-{3-[4-(2-tolyl)-1-piperazinyl]-propyl}-ester
1,4-Dihydro-2,6-dimethyl-4-(3-nitrophenyl)-pyridin-3,5-dicarbonsäure-3-(-)-menthyl-5-{2-[4-(2-tolyl)-1-piperazinyl]-ethyl}-ester
1,4-Dihydro-2,6-dimethyl-4-(3-nitrophenyl)-pyridin-3,5-dicarbonsäure-3-(-)-menthyl-5-{3-[4-(2,4-dimethylphenyl)-1-piperazinyl]-propyl}-ester
1,4-Dihydro-2,6-dimethyl-4-(3-nitrophenyl)-pyridin-3,5-dicarbonsäure-3-(-)-menthyl-5-{2-[4-(2,4-dimethylphenyl)-1-piperazinyl]-ethyl}-ester
1,4-Dihydro-2,6-dimethyl-4-(3-nitrophenyl)-pyridin-3,5-dicarbonsäure-3-(-)-menthyl-5-{3-[4-(2,6-dimethylphenyl)-1-piperazinyl]-propyl}-ester
1,4-Dihydro-2,6-dimethyl-4-(3-nitrophenyl)-pyridin-3,5-dicarbonsäure-3-(-)-menthyl-5-{2-[4(2,6-dimethylphenyl)-1-piperazinyl]-ethyl}-ester
4-(2,3-Dichlorphenyl)-1,4-dihydro-2,6-dimethyl-pyridin-3,5-dicarbonsäure-3-(-)-menthyl-5-[3-(4-phenyl-1-piperazinyl)-propyl]-ester
4-(2,3-Dichlorphenyl)-1,4-dihydro-2,6-dimethyl-pyridin-3,5-dicarbonsäure-3(-)-menthyl-5-[2-(4-phenyl-1-piperazinyl)-ethyl]-ester
4-(2,3-Dichlorphenyl)-1,4-dihydro-2,6-dimethyl-pyridin-3,5-dicarbonsäure-3-(-)-menthyl-5-{3-[4-(2-methoxyphenyl)-1-piperazinyl]-propyl}-ester
4-(2,3-Dichlorphenyl)-1,4-dihydro-2,6-dimethyl-pyridin-3,5-dicarbonsäure-3-(-)-menthyl-5-{2-[4-(2-methoxyphenyl)-1-piperazinyl]-ethyl}-ester
4-(2,3-Dichlorphenyl)-1,4-dihydro-2,6-dimethyl-pyridin-3,5-dicarbonsäure-3-(-)-menthyl-5-{3-[4-(2-ethoxyphenyl)-1-piperazinyl]-propyl}-ester
4-(2,3-Dichlorphenyl-1,4-dihydro-2,6-dimethyl-pyridin-3,5-dicarbonsäure-3-(-)-menthyl-5-{2-[4-(2-ethoxyphenyl)-1-piperazinyl]-ethyl}-ester
1,4-Dihydro-2,6-dimethyl-4-(3-nitrophenyl)-pyridin-3,5-dicarbonsäure-3-(-)-menthyl-5-[2-(4,4-diphenylpiperidyl-1)-ethyl]-ester,
1,4-Dihydro-2,6-dimethyl-4-(3-nitrophenyl)-pyridin-3,5-dicarbonsäure-3-(-)-menthyl-5-[3-(4,4-diphenylpiperidyl-1)-propyl]-ester
1,4-Dihydro-2,6-dimethyl-4-(3-pyridyl)-pyridin-3,5-dicarbonsäure-3-(-)-menthyl-5-[2-(4,4-diphenylpiperidyl-1)-ethyl]-ester,
1,4-Dihydro-2,6-dimethyl-4-(3-pyridyl)-pyridin-3,5-dicarbonsäure-3-(-)-menthyl-5-[3-(4,4-diphenylpiperidyl-1)-propyl]-ester
1,4-Dihydro-2,6-dimethyl-4-(3-nitrophenyl)-pyridin-3,5-dicarbonsäure-3-(-)-menthyl-5-[2-(benzylmethylamino)-ethyl]-ester,
1,4-Dihydro-2,6-dimethyl-4-(3-nitrophenyl)-pyridin-3,5-dicarbonsäure3-(-)-menthyl-5-[3-(benzylmethylamino)-propyl]-ester
1,4-Dihydro-2,6-dimethyl-4-(3-nitrophenyl)-pyridin-3,5-dicarbonsäure-3-(-)-menthyl-5-[2-(4-morpholino)-ethyl]-ester,
1,4-Dihydro-2,6-dimethyl-4-(3-nitrophenyl-pyridin-3,5-dicarbonsäure-3-(-)-menthyl-5-[2-(1-piperidino)-ethyl]-ester,
1,4-Dihydro-2,6-dimethyl-4-(3-nitrophenyl)-pyridin-3,5-dicarbonsäure-3-(-)-menthyl-5-[2-(1-pyrrolidino)-ethyl]-ester,
1,4-Dihydro-2,6-dimethyl-4-(3-nitrophenyl)-pyridin-3,5-dicarbonsäure-3-(-)-menthyl-5-[3-(4-morpholino)-propyl]-ester,
1,4-Dihydro-2,6-dimethyl-4-(3-nitrophenyl)-pyridin-3,5-dicarbonsäure-3-(-)-menthyl-5-[3-(1-piperidino)-propyl]-ester,
1,4-Dihydro-2,6-dimethyl-4-(3-nitrophenyl)-pyridin-3,5-dicarbonsäure-3-(-)-menthyl-5-[3-(1-pyrrolidino)-propyl]-ester,
1,4-Dihydro-2,6-dimethyl-4-(3-nitrophenyl)-pyridin-3,5-dicarbonsäure-3-(-)-menthyl-5-[2-(4-phenylpiperidyl-1)-ethyl]-ester
1,4-Dihydro-2,6-dimethyl-4-(3-nitrophenyl)-pyridin-3,5-dicarbonsäure-3-(-)-menthyl-5-[3-(4-phenylpiperidyl-1)-propyl]-ester,
1,4-Dihydro-2,6-dimethyl-4-(3-nitrophenyl)-pyridin-3,5-dicarbonsäure-3-(-)-menthyl-5-{2-[4-(4-fluorbenzoyl)-piperidyl-1)-ethyl]-ester,
1,4-Dihydro-2,6-dimethyl-4-(3-nitrophenyl)-pyridin-3,5-dicarbonsäure-3-(-)-menthyl-5-{3-[4-(4-fluorbenzoyl)-piperidyl-1)-propyl]-ester
und den Salzen dieser Verbindungen.

9. Verfahren zur Herstellung von Arzneimitteln enthaltend eine oder mehrere Verbindungen nach einem oder mehreren der Ansprüche 1 bis 7 und/oder ihre pharmakologisch verträglichen Salze, dadurch gekennzeichnet, daß man eine oder mehrere Verbindungen nach einem oder mehreren der Ansprüche 1 bis 7 und/oder ihre pharmakologisch verträglichen Salze mit einem galenischen Hilfstroff mischt und in eine für Arzneimittel übliche Formulierung überführt.